# NEUE EUROPÄISCHE PATENTSCHRIFT

(11) **EP 3 011 037 B2**
(45) Veröffentlichungstag und Bekanntmachung des Hinweises auf die Entscheidung über den Einspruch: **26.06.2024**
(45) Hinweis auf die Patenterteilung: 10.10.2018
(21) Anmeldenummer: 14750139.9
(22) Anmeldetag: 06.06.2014
(51) Int. Cl.: C12N 15/82, C12N 15/113, C07K 14/415

(54) **RESISTENZGEN GEGEN RIZOMANIA**
RHIZOMANIA-RESISTANT GENE
GÈNE DE RÉSISTANCE À LA RHIZOMANIE

(30) Priorität: 17.06.2013 DE 102013010026
(43) Veröffentlichungstag der Anmeldung: 27.04.2016
(60) Teilanmeldung: 18198740.5
(73) Patentinhaber: KWS SAAT SE & Co. KGaA, 37574 Einbeck (DE)
(72) Erfinder: TÖRJÈK, Otto, 37574 Einbeck (DE); BORCHARDT, Dietrich, 37574 Einbeck (DE); MECHELKE, Wolfgang, 37574 Einbeck (DE); LEIN, Jens Christoph, 37083 Göttingen (DE)
(74) Vertreter: Eisenführ Speiser
(86) Internationale Anmeldenummer: PCT/DE2014/000310
(87) Internationale Veröffentlichungsnummer: WO 2014/202044

(56) Entgegenhaltungen:
- WO-A1-2020/053313
- DE-A1-102013 010 026
- GIDNER SARA ET AL: "QTL mapping of BNYVV resistance from the WB41 source in sugar beet", GENOME, Bd. 48cl, Nr. 2, April 2005 (2005-04), Seiten 279-285, XP009180707, ISSN: 0831-2796 in der Anmeldung erwähnt
- LEIN JENS CHRISTOPH ET AL: "Resistance gene analogues are clustered on chromosome 3 of sugar beet and cosegregate with QTL for rhizomania resistance", GENOME, Bd. 50, Nr. 1, Januar 2007 (2007-01), Seiten 61-71, XP001526559, ISSN: 0831-2796
- TIAN YANYAN ET AL: "The absence of TIR-type resistance gene analogues in the sugar beet (Beta vulgaris L.) genome.", JOURNAL OF MOLECULAR EVOLUTION, Bd. 58, Nr. 1, Januar 2004 (2004-01), Seiten 40-53, XP002731101, ISSN: 0022-2844
- GRIMMER M K ET AL: "An anchored linkage map for sugar beet based on AFLP, SNP and RAPD markers and QTL mapping of a new source of resistance to Beet necrotic yellow vein virus", THEORETICAL AND APPLIED GENETICS ; INTERNATIONAL JOURNAL OF PLANT BREEDING RESEARCH, SPRINGER, BERLIN, DE, Bd. 114, Nr. 7, 9. Februar 2007 (2007-02-09), Seiten 1151-1160, XP019510491, ISSN: 1432-2242, DOI: 10.1007/S00122-007-0507-3
- BUTORINA A K ET AL: "Molecular genetic investigation of sugar beet (L.)", RUSSIAN JOURNAL OF GENETICS, NAUKA/INTERPERIODICA, MO, Bd. 47, Nr. 10, 8. Oktober 2011 (2011-10-08), Seiten 1141-1150, XP019962364, ISSN: 1608-3369, DOI: 10.1134/S102279541110005X
- AMIRI R ET AL: "A new RAPD marker for beet necrotic yellow vein virus resistance gene in Beta vulgaris", BIOLOGIA PLANTARUM, KLUWER ACADEMIC PUBLISHERS, DO, Bd. 53, Nr. 1, 21. März 2009 (2009-03-21), Seiten 112-119, XP019670809, ISSN: 1573-8264
- PAVLI OURANIA I ET AL: "Achievements and prospects in breeding for rhizomania resistance in sugar beet", FIELD CROPS RESEARCH, Bd. 122, Nr. 3, Juni 2011 (2011-06), Seiten 165-172, XP002731102, ISSN: 0378-4290
- THIEL et al.: "Identification of Beet necrotic yellow vein virus P25 Pathogenicity Factor - Interacting Sugar Beet Proteins That Represent Putative Virus Targets or Components of Plant Resistance", Molecular Plant-Microbe Interactions,, vol. 22, no. 8, 2009, pages 999-1010,
- DATABASE Nucleotide 2006, TIAN et al.: "Beta vulgaris clone cZR-7(f) NBS-LRR type resistance protein mRNA, complete cds", retrieved from NCBI Database accession no. DQ907612.1
- Tandem Technology: "Magistral leaflet", Ses VanderHave Technical Bulletin, 8 August 2007 (2007-08-08),
- Anonymous: "Abstract brochure Part A:Papers", Fourth Meeting of the ECPGR Working Group on Beta and the World Beta Network, 20 June 2012 (2012-06-20), - 22 June 2012 (2012-06-22), page 9,
- MEULEMANS et al.: "Interaction between different genes and influence of the genetic background in the expression of Rhizomania resistance", 1st joint IIRB-ASSBT Congress,, 2003, pages 161-173,
- Wan-Song NI et al.: "Construction of a Plant Transformation-ready Expression cDNA Library for Thellungiella halophila Using Recombination Cloning", Journal of Integrative Plant Biology, vol. 49, no. 9, 2007, pages 1313-1319,
- KWS Saat SE: "KWS sees the Breeding Reservation weakened by a patent decision of the EPO", AgroPages, 13 December 2018 (2018-12-13), Retrieved from the Internet: URL:http://news.agropages.com/News/NewsDet ail---28699.htm
- Friedrich J. Kopisch-Obuch et al.,: "Maintaining High Levels of Rhizomania Resistance in Sugar Beet Cultivars", 76th IIRB Congress, 6 June 2018 (2018-06-06), pages 1-13,
- Gina G Capistrano-Gossmann; Et Al: "Crop wild relative populations of Beta vulgaris allow direct mapping of agronomically important genes", Nature Communications, vol. 8, 2017, pages 1-8, & SES VanderHave, "Sequence element from Capistrano et al around probe CAU38880", [cited 25.01.2019] & SES VanderHave. "Rz2 Capistrano vs Rz3 KWS". [cited 25.01.2019] & SES VanderHave. "SEQ ID NO:1 vs Capistrano", [cited 14,02.2019]
- SES VanderHave. ?Rz2/3 vs shotgun", [cited 28.01.2019] & Dohm et al, "GenBank AYZW01060923.1" 17.12.2013, [cited 14.02.2019]
- SES VanderHave, "SEQ ID NO:1 vs ARYA01032019.1? 13.05.2013, [cited 04.02.2019] & SES VanderHave. "GenBank ARYA01032019.1" 13.06.2013, [cited 14.02.2019]
- anonymous: "2013 Sugarbeet Research and Extension Reports", North Dakota State University, vol. 44, 1 January 2014 (2014-01-01), page 2pp, 249, 263, 272, 273,
- CUI, Y. et al.: "Fine Mapping of the Bsrl Barley Stripe Mosaic Virus Resistance Gene in the Model Grass Brachypodium distachyon", PLOS One, vol. 7, no. 6, June 2012 (2012-06), pages e38333-1-e38333-10,
- Declaration of Dr. Harlene Hatterman-Valenti & Declaration of Dr. Hendrik Tschoep & Scheme of the DNA extraction procedure performed at SESVANDERHAVE
- Data sequencing of the variety 36812 RR (cfr D15) from the external provider Eurofins Genomics and explanations on how the sequencing has been carried out
- Pferdmenges, Friederike et al., "Beet necrotic yellow vein virus (BNYW) P-type overcomes resistance in Rz1 x Rz2sugar beet hybrids after mechanical inoculation? 2006: poster
- Pferdmenges F.: "Occurrence, spread and pathogenicity of different Beet necrotic yellow vein virus (BNYVV) isolates", Dissertation Georg-August Universität Göttingen, 1 September 2007 (2007-09-01), pages 1-111,
- Crop Production Magazine, Vol. 13, No. 5, June 2011 Issue; particular relevance : pages 46-54, https://www.cpm-magazme.co.uk/wp-content/u ploads/2016/07/CPM_ June_ 2011.pdf
- Rush Charles et al: "The Continuing Saga of Rhizomania of Sugar Beets in the United States", Plant Disease, vol. 90, no. 1, 1 January 2006 (2006-01-01), pages 1-12,
- KWS, "What is RZ2.0 Double Resistance? "Web extract from KWS. Dated by Google in 2011, https://www.kws.com/corp/en/products/sugar beet/progress-made-by-innovation/r-d-andbr eedinq/rz-2-0/
- Abram, Mike, "Aggressive rhizomania resistance-beating strain found in Norfolk sugar beet" Web extract Farmers weekly dated, Feb. 15,2011, https://www.fwi.co.uk/arable/aggressive-rh izomania-resistance-beating-strain-found-i nnorfolk-sugar-beet
- Harju Val, Et Al: "Rhizomania P type - a new threat to growers?", British Sugar beet review, vol. 70, no. 3, 1 January 2002 (2002-01-01), pages 22-27, XP055889669,

## Beschreibung

### Gebiet der Erfindung

Die vorliegende Erfindung betrifft eine Nukleinsäuremolekül, welches ein Polypeptid kodiert, das in der Lage ist, eine Resistenz gegenüber einem Pathogen, insbesondere gegenüber "Beet Necrotic Yelllow Vein Virus" in einer Pflanze, insbesondere der Gattung Beta, in welcher das Polypeptid exprimiert wird, zu vermitteln. Weiterhin betrifft die Erfindung ein Polypeptid, welches in der Lage ist eine Resistenz gegenüber einem Pathogen in einer Pflanze, insbesondere eine Resistenz gegenüber BNYVV in einer Pflanze der Gattung Beta, in welcher das Polypeptid exprimiert wird, zu vermitteln und welches durch das erfindungsgemäße Nukleinsäuremolekül kodiert wird. Die Erfindung betrifft weiterhin eine transgene Pflanze, Pflanzenzelle, Pflanzenorgan, Pflanzengewebe, Pflanzenteil oder einen Samen einer Pflanze, welche das Nukleinsäuremolekül oder Teile davon umfassen sowie Verfahren zur Herstellung einer solchen transgenen Pflanze oder Pflanzenzelle. Ferner schließt die Erfindung auch Verfahren zum Nachweis des Resistenz-vermittelnden Nukleinsäuremoleküls sowie Verfahren zur Selektion von Pflanzen oder Pflanzenzellen, welche das Resistenz-vermittelnde Nukleinsäuremolekül aufweisen.

### Hintergrund der Erfindung

Die Rizomania ist die wirtschaftlich bedeutendste Zuckerrübenkrankheit weltweit, die Ertragsverluste von 50 % und mehr verursachen kann. Die Krankheit, die auch "Wurzelbärtigkeit" genannt wird, wird durch das "Beet Necrotic Yellow Vein Virus" (BNYVV) hervorgerufen und durch den bodenbürtigen Protozoen *Polymyxa betae* übertragen. Eine BNYVV-Infektion äußert sich in einer gesteigert Proliferation der dünnen Wurzeln und Seitenwurzeln und in der Ausbildung eines stark verkleinerten Wurzelkörpers mit reduzierten Zuckergehalt. Infizierte Pflanzen zeigen eine verminderte Wasseraufnahme und sind somit empfindlicher gegenüber Trockenstress. Wenn sich die Infektion auf die Gesamtpflanze ausweitet, kommt es zur Gelbfärbung der Blattvenen, zu nekrotischen Läsionen und gelben Flecken auf den Blättern. Da eine kurative Bekämpfung der Krankheit wie bei anderen viralen Krankheiten nicht möglich ist, kann ein Schaden nur über den Anbau von resistenten Sorten verhindert werden. Derzeit sind im Wesentlichen drei Majorgene gegen die Rizomania untersucht: RZ-1 (auch als "Holly" bezeichnet), RZ-2 und RZ-3. Darüber hinaus werden weitere, aber weniger bedeutende Rizomania-Resistenzgene in der Literatur beschrieben. Dabei ist das Resistenzgen RZ-1 bereits in den meisten Zuchtlinien (Saatelter oder/und Pollenspender Elternkomponenten) inkorporiert. Es zeigte sich jedoch, dass in stark infizierten Regionen oder in Regionen mit diversen BNYVV-Pathotypen (bspw. Sohi & Maleki, 2004) die Resistenz, welche durch RZ-1 vermittelt wird, nicht ausreicht. Aus diesem Grund wurde schon vor geraumer Zeit vorgeschlagen RZ-1 mit beispielsweise RZ-2 oder RZ-3 zu kombinieren. RZ-2 und RZ-3 stammen aus *Beta vulgaris* subsp. *maritima*-Quellen (WB42, WB41) und kartieren genetisch in der gleichen Region auf Chromosom 3 des Zuckerrübengenoms, während RZ-1 südlich von RZ-2 und RZ-3 aber ebenfalls auf Chromosom 3 kartiert. Scholten et al. (1999) bestimmte einen Abstand von 20-25 cM zwischen den RZ-Majorgenen RZ-1 und RZ-2. Gidner *et al.* (2005) findet einen kleineren Abstand von 5 cM zwischen RZ-1 und RZ-2 und schließt nicht aus, dass RZ-2 und RZ-3 auf dem gleichen Lokus kartieren. Schmidlin *et al.* (2008) hatte mittels Expressionsanalyse in infizierten Rüben unterschiedlich induzierte Gene identifiziert, welche aber nicht RZ-2 oder RZ-3 entsprachen. In der Studie von Larson *et al.* (2008) wurden mit der MALDI-TOF-MS Methode einige BNYVV induzierte Proteine in der Zuckerrübe detektiert, die Proteine, welche durch RZ-1, RZ-2 oder RZ-3 kodiert werden, konnten die Wissenschaftler jedoch nicht identifizieren. Zudem ist die Sequenzregion, insbesondere um diese Resistenzgene herum, repetitiv, was die Entwicklung von diagnostischen Marker besonders schwierig macht. So sind bis jetzt weder hochauflösende Markerkarten noch verifizierte Kandidatengene für die genannten Rizomania-Resistenzgene öffentlich verfügbar. Außerdem ist bislang der funktionelle Hintergrund dieser RZ-Resistenzgene, d. h. die genetische Struktur, völlig unbekannt.

Für eine nachhaltige Züchtung gegen Rizomania, die der Gefahr von Resistenz-brechenden BNYVV-Isolaten entgegen wirken soll, ist es notwendig, ständig neue Resistenzgene zu identifizieren und diese in die Genpools der Kulturpflanzen wie Zuckerrüben zu integrieren.

### Zusammenfassung der Erfindung

Die vorliegende Erfindung wurde vor dem Hintergrund des vorstehend beschriebenen Stands der Technik gemacht, wobei es Aufgabe der vorliegenden Erfindung ist, ein Nukleinsäuremolekül und/oder ein Polypeptid bereitzustellen, welches in der Lage ist, Resistenz gegenüber Rizomania in einer Pflanze zu vermitteln. Weiterhin ist es Aufgabe eine transgene Rizomania-resistente Pflanze sowie eine Methode zu deren Herstellung bereitzustellen. Weiterhin ist es Aufgabe der vorliegenden Erfindung Methoden zur Nutzung und Entwicklung molekulare Marker bereitzustellen, welche eine effiziente Züchtung gegen Rizomania und die Entwicklung neuer resistenter Pflanzenlinien ermöglichen.

Ausgestaltungen der vorliegenden Erfindung, welche die Aufgabe lösen, basieren auf der genetischen Feinkartierung, Identifizierung, Isolierung und Charakterisierung eines Gens, das aus dem Donor *Beta vulgaris* subsp. *maritima* stammt und für ein Polypeptid bzw. Protein kodiert, das in der Lage ist, Resistenz gegenüber einem Pathogen in einer Pflanze, in welcher das Polypeptid exprimiert wird, zu vermitteln.

Einige der in dieser Anmeldung verwendeten Begriffe werden nachfolgend zunächst näher erläutert:
Der Begriff "etwa" im Zusammenhang mit der Angabe einer Länge einer Nukleotidsequenz bedeutet eine Abweichung um +/- 200 Basenpaaren, bevorzugt um +/- 100 Basenpaaren und besonders bevorzugt um +/- 50 Basenpaaren.

Eine "Pflanze der Gattung Beta" gehört zu der Familie der Fuchsschwanzgewächse (Amaranthaceae). Zu diesen Pflanzen zählen Pflanzen der Spezies *Beta macrocarpa, Beta vulgaris, Beta lomatogona, Beta macrorhiza, Beta corolliflora, Beta trigyna* und *Beta nana.* Eine Pflanze der Spezies *Beta vulgaris* ist insbesondere eine Pflanze der Subspezies *Beta vulgaris* subsp. *maritima* (See-Mangold) oder *Beta vulgaris* subsp. *vulgaris.* Hierzu zählen beispielsweise *Beta vulgaris* subsp. *vulgaris* var. *altissima* (Zuckerrübe i.e.S.), *Beta vulgaris* ssp. *vulgaris* var. *vulgaris* (Mangold), *Beta vulgaris* ssp. *vulgaris* var. *conditiva* (Rote Rübe/Bete), *Beta vulgaris* ssp. *vulgaris* var. *crassa*/*alba* (Futterrübe).

Unter "Hybridisieren" oder "Hybridisierung" wird ein Vorgang verstanden, bei dem sich ein einzelsträngiges Nukleinsäuremolekül an einen weitestgehend komplementären Nukleinsäurestrang anlagert, d.h. mit diesem Basenpaarungen eingeht. Standardverfahren zur Hybridisierung sind beispielsweise in Sambrook et al. 2001 beschrieben. Vorzugsweise wird darunter verstanden, dass mindestens 60%, weiter bevorzugt mindestens 65%, 70%, 75%, 80% oder 85%, besonders bevorzugt 90%, 91%, 92%, 93%, 94%, 95%, 96%, 97%, 98% oder 99% der Basen des Nukleinsäuremoleküls eine Basenpaarung mit dem weitestgehend komplementären Nukleinsäurestrang eingehen. Die Möglichkeit einer solchen Anlagerung hängt von der Stringenz der Hybridisierungsbedingungen ab. Der Begriff "Stringenz" bezieht sich auf die Hybridisierungsbedingungen. Hohe Stringenz ist dann gegeben, wenn eine Basenpaarung erschwert ist, niedrige Stringenz, wenn eine Basenpaarung erleichtert ist. Die Stringenz der Hybridisierungsbedingungen hängt beispielsweise von der Salzkonzentration bzw. lonenstärke und der Temperatur ab. Generell kann die Stringenz durch eine Erhöhung der Temperatur und/oder eine Erniedrigung des Salzgehaltes erhöht werden. Unter "stringenten Hybridisierungsbedingungen" sind solche Bedingungen zu verstehen, bei denen eine Hybridisierung überwiegend nur zwischen homologen Nukleinsäuremolekülen stattfindet. Der Begriff "Hybridisierungsbedingungen" bezieht sich dabei nicht nur auf die bei der eigentlichen Anlagerung der Nukleinsäuren herrschenden Bedingungen, sondern auch auf die bei den anschließenden Waschschritten herrschenden Bedingungen. Stringente Hybridisierungsbedingungen sind beispielsweise Bedingungen, unter denen überwiegend nur solche Nukleinsäuremoleküle hybridisieren, die mindestens 70%, vorzugsweise mindestens 75%, mindestens 80%, mindestens 85%, mindestens 90% oder mindestens 95% Sequenzidentität aufweisen. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde. Der hier verwendete Begriff "stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 x SSC und 0,1 % SDS bei 68 °C. Bevorzugt findet eine Hybridisierung unter stringenten Bedingungen statt.

Unter einem "isolierten Nukleinsäuremolekül" wird ein aus seiner natürlichen bzw. ursprünglichen Umgebung herausgelöstes Nukleinsäuremolekül verstanden. Der Begriff umfasst auch ein synthetisch hergestelltes Nukleinsäuremolekül. Unter einem "isolierten Polypeptid" wird ein aus seiner natürlichen bzw. ursprünglichen Umgebung herausgelöstes Polypeptid verstanden. Der Begriff umfasst auch ein synthetisch hergestelltes Polypeptid.

Ein "molekularer Marker" ist eine Nukleinsäure, die polymorph ist in einer pflanzlichen Population. Dadurch ist ein solcher Marker in der Lage verschiedene allelische Zustände (Allele) zu detektieren und zu unterscheiden. Genutzt werden hierfür bekannte analytische Verfahren wie beispielsweise RFLP, AFLP, SNP, SSR oder KASP. Der Begriff "molekularer Marker" bezieht sich auch auf Nukleotidsequenzen, welche komplementär oder zumindest weitestgehend komplementär oder homolog sind zu genomischen Sequenzen, zum Beispiel Nukleinsäuren welche als Sonden oder Primer eingesetzt werden. Marker, die genetische Polymorphismen beschreiben sind, können unter Verwendung gut etablierter Methoden nachgewiesen werden. Dies sind z.B. eine PCR-basierte Sequenz-spezifische Amplifikation, eine Detektion von 'Restriction Fragment Length Polymorphisms' (RFLP), eine Detektion von Polynukleotid-Polymorphismen mittels 'Allele Specific Hybridization' (ASH), eine Detektion von amplifizierten Variable Sequences' des Pflanzengenoms, eine Detektion einer 'Self-Sustained Sequence Replication', eine Detektion von 'Simple Sequence Repeats' (SSRs), eine Detektion von 'Single Nucleotide Polymorphisms' (SNPs), oder eine Detektion von 'Amplified Fragment Length Polymorphisms' (AFLPs). Ferner sind auch die Methoden zur Detektion von 'Expressed Sequence Tags' (ESTs) und SSR-Marker abgeleitet von EST-Sequenzen und 'Randomly Amplified Polymorphic DNA' (RAPD) bekannt.

Ein "Promotor" meint eine nicht-translatierte regulatorische DNA-Sequenz, typischerweise stromaufwärts einer kodierenden Region, welche die Bindestelle für die RNA-Polymerase beinhaltet und die Transkription der DNA initiiert.

Ein "Pathogen" meint einen Organismus, der in Interaktionen mit einer Pflanze zu Krankheitssymptomen an einem oder mehreren Organen bei der Pflanze führt. Zu diesen Pathogenen zählen beispielsweise tierische, pilzliche, bakterielle oder virale Organismen oder Oomyceten.

Unter einer "Pathogeninfektion" ist der früheste Zeitpunkt zu verstehen, zu dem ein Pathogen mit einem pflanzlichen Wirtsgewebe interagiert. Beispielsweise im Fall des viralen Pathogens BNYW wird dieser durch den Protozoen *Polymyxa betae* übertragen. *Polymyxa* bildet Sporen, die in der Erde über viele Jahrzehnte überdauern können. In diesen Sporen überdauert auch der Virus. Wenn diese Dauersporen zu mobilen Zoosporen auskeimen, kann der Virus über diese in Zellen des pflanzlichen Wirtsgewebes gelangen und dort mit dem Wirt interagieren (Esser 2000).

Pflanzliche "Organe" meinen beispielsweise Blätter, Sprossachse, Stamm, Wurzeln, Hypocotyl, vegetative Knospen, Meristeme, Embryos, Antheren, Ovula oder Früchte. Pflanzliche "Teile" meinen einen Zusammenschluss mehrerer Organe, z.B. eine Blüte oder ein Samen, oder einen Teil eines Organs, z.B. eine Querschnitt durch den Spross. Pflanzliche "Gewebe" sind zum Beispiel Kallusgewebe, Speichergewebe, meristematische Gewebe, Blattgewebe, Sprossgewebe, Wurzelgewebe, Pflanzentumorgewebe oder reproduktives Gewebe. Unter pflanzlichen "Zellen" sind beispielsweise isolierte Zellen mit einer Zellwand oder Aggregate davon oder Protoplasten zu verstehen.

Der Begriff "Resistenz" ist breit zu verstehen und deckt den Bereich des Schutzes von einer Verzögerung bis zur kompletten Hemmung der Entwicklung der Krankheit ab. Ein Beispiel für einen Pathogen von Bedeutung ist der Beet Necrotic Yellow Vein Virus (BNYW). Vorzugsweise erreicht eine resistente Pflanzenzelle der Erfindung oder resistente Pflanze der Erfindung eine Resistenz gegen BNYW. Eine Resistenz gegenüber einem Pathogen ist gleichzusetzen mit einer Resistenz gegenüber der Krankheit, welche dieser Pathogen verursacht, beispielsweise ist eine Resistenz gegenüber BNYW auch eine Resistenz gegenüber Rizomania.

"Transgene Pflanze" bezieht sich auf einen Pflanze, in dessen Genom mindestens eine Nukleinsäure integriert wurde. Dabei kann es sich um eine heterologe Nukleinsäure handeln. Vorzugsweise ist die Nukleinsäure stabil integriert, was bedeutet, dass die integrierte Nukleinsäure in der Pflanze stabil erhalten bleibt, exprimiert werden kann und auch stabil an die Nachkommen vererbt werden kann.

Die vorliegende Anmeldung offenbart ein Nukleinsäuremolekül, welches ein Polypeptid kodiert, das in der Lage ist, eine Resistenz gegenüber einem Pathogen in einer Pflanze, in welcher das Polypeptid exprimiert wird, zu vermitteln. Das Nukleinsäuremolekül umfasst eine Nukleotidsequenz, welche ausgewählt ist aus
a) einer Nukleotidsequenz, die ein Polypeptid mit einer Aminosäuresequenz gemäß SEQ ID NO: 2 oder SEQ ID NO: 3 kodiert,
b) einer Nukleotidsequenz, die die kodierende Sequenz der DNA-Sequenz gemäß SEQ ID NO: 1 umfasst,
c) einer Nukleotidsequenz, die mit der komplementären Sequenz zu der Nukleotidsequenz nach a) oder b) unter stringenten Bedingungen hybridisiert,
d) einer Nukleotidsequenz, die ein Polypeptid kodiert, welches sich durch Substitution, Deletion und/oder Addition von einer oder mehrerer Aminosäuren der Aminosäuresequenz, die durch die Nukleotidsequenz nach a) oder b) kodiert wird, von einem Polypeptid, das durch die Nukleotidsequenz nach a) oder b) kodiert wird, ableitet,
e) einer Nukleotidsequenz, die ein Polypeptid kodiert, welches eine Aminosäuresequenz aufweist, die zu mindestens 60% identisch ist zu einer Aminosäuresequenz, welche durch die Nukleotidsequenz nach a) oder b) kodiert wird, oder
f) einer Nukleotidsequenz, welche mindestens eine Nukleotid-bindende Domäne (NBS bzw. NB-ARC) entsprechend den Aminosäurepositionen 168-227 der SEQ ID NO: 2 oder entsprechend den Aminosäurepositionen 182-241 der SEQ ID NO: 3, mindestens eine Leuzin-reiche Domäne (LRR) entsprechend den Aminosäurepositionen 591-613 der SEQ ID NO: 2 oder entsprechend den Aminosäurepositionen 605-627 der SEQ ID NO: 3 und/oder mindestens eine interne repetitive Domäne (IR) entsprechend den Aminosäurepositionen 1013-1072 der SEQ ID NO: 2 oder entsprechend den Aminosäurepositionen 1027-1086 der SEQ ID NO: 3 kodiert.

Bei dem Nukleinsäuremolekül kann es sich um ein isoliertes Nukleinsäuremolekül handeln. Bevorzugt handelt es sich um DNA, und besonders bevorzugt um cDNA (kodierende DNA). Vorzugsweise vermittelt das Polypeptid, welches durch das erfindungsgemäße Nukleinsäuremolekül kodiert wird, eine Resistenz gegenüber dem viralen Pathogen "Beet Necrotic Yellow Vein Virus" (BNYVV), welcher die Pflanzenkrankheit Rizomania verursacht. Weiterhin vermittelt das Polypeptid, welches durch das erfindungsgemäße Nukleinsäuremolekül kodiert wird, insbesondere in einer Pflanze der Gattung Beta eine Resistenz gegenüber einem Pathogen. Bevorzugt handelt es sich bei der Pflanze um eine Pflanze der Spezies *Beta vulgaris,* besonders bevorzugt um eine Pflanze der Subspezies *Beta vulgaris* subsp. *maritima oder Beta vulgaris* subsp. *vulgaris*; hierzu zählen beispielsweise die Kulturarten Zuckerrübe, Rote Bete, Futterrübe, Blattmangold und Stielmangold.

In einer Ausführungsform des erfindungsgemäßen Nukleinsäuremoleküls umfasst das Nukleinsäuremolekül die Nukleotidsequenz nach a). Die Aminosäuresequenz gemäß SEQ ID NO: 2 des kodierten Polypeptids und/oder gemäß SEQ ID NO: 3 des kodierten Polypeptids stellt das Resistenzprotein des RZ-3-Gens dar. Es handelt sich hierbei um ein Resistenzgen/- protein des Typs NBS-LRR, das durch bestimmte Strukturmotive charakterisiert ist. Die allgemeine Struktur von solchen Resistenzproteinen in Pflanzen ist bereits gut untersucht (Martin *et al.* 2003). Jedoch ist das Prinzip der strukturellen Ausgestaltung insbesondere der sog. LRR-Domäne, welche als potentielle Erkennungsdomäne für zumeist unbekannte pathogene Effektoren gilt, nicht vorhersagbar. Demzufolge ist die Identifizierung eines BNYW-Resistenz-vermittelnden Gens bzw. Proteins allein auf Grundlage der bekannten Strukturmotive unmöglich. Die Identifizierung des RZ-3-Resistenzgens fand im Zuge eines Map-based-Cloning-Prozesses statt, der eine intensive genetische Kartierung und Feinkartierung der Zielregion, in welcher das RZ-3-Resistenzgen zunächst vermutet wurde, erforderte. Die Entwicklungsarbeiten werden weiter unten genauer beschreiben.

Das identifizierte Resistenzprotein gehört zum Typ NBS-LRR und weist eine Nukleotid-bindende Domäne (NBS, auch bekannt unter NB-ARC (nucleotide-binding adaptor shared by APAF-1, R proteins, and CED-4)) entsprechend den Aminosäurepositionen 168-227 der SEQ ID NO: 2 oder entsprechend den Aminosäurepositionen 182-241 der SEQ ID NO: 3, eine Leuzin-reiche Domäne (LRR) entsprechend den Aminosäurepositionen 591-613 der SEQ ID NO: 2 oder entsprechend den Aminosäurepositionen 605-627 der SEQ ID NO: 3 und mindestens eine interne repetitive Domäne (IR; Internal Repeat-Domäne) entsprechend den Aminosäurepositionen 1013-1072 der SEQ ID NO: 2 oder entsprechend den Aminosäurepositionen 1027-1086 der SEQ ID NO: 3 auf. Die NBS-Domäne wird durch die Nukleotide 2019-2882 der SEQ ID NO: 1, die LRR-Domäne durch die Nukleotide 3288-3356 der SEQ ID NO: 1 und die IR-Domäne durch die Nukleotide 4554-4871 der SEQ ID NO: 1 kodiert. Die NB-ARC-Domäne ist eine zentrale Nukleotid-bindende Domäne. Sie ist wahrscheinlich eine funktionelle ATPase-Domäne, welche voraussichtlich die Aktivität eines Resistenzproteins reguliert. Die NB-ARC-Domäne besteht aus drei Subdomänen: NB, ARC1 und ARC2. Charakteristische Motive der NB-ARC-Domäne sind APAF-1 (apoptotic proteaseactivating factor-1), welche mutmaßlich für die hypersensitive Reaktion verantwortlich ist, hhGRExE, Walker-A- oder P-loop, Walker-B, GxP, RNBS-A bis D und MHD (Ooijen et al., 2008). Einige der genannten Motive konnten bereits identifiziert werden. In einer weiteren Ausführungsform des erfindungsgemäßen Nukleinsäuremoleküls umfasst das Nukleinsäuremolekül die Nukleotidsequenz nach b). Die Nukleotidsequenz umfasst die kodierenden Sequenzen der DNA-Sequenz gemäß SEQ ID NO: 1, welche für die Aminosäuresequenzen gemäß SEQ ID NO: 2 und 3 kodieren.

In einer weiteren Ausführungsform des erfindungsgemäßen Nukleinsäuremoleküls umfasst das Nukleinsäuremolekül die Nukleotidsequenz nach d). Diese Nukleotidsequenz kodiert ein Polypeptid, das ein Derivat des Polypeptids darstellt, welches von der Nukleotidsequenz nach a) oder b) kodiert wird. Ein Derivat des Polypeptids stellt eine abgeleitete Aminosäuresequenz dar, welche mindestens eine Substitution, Deletion oder Addition von einer oder mehrerer Aminosäuren aufweist, wobei die Funktionalität des kodierten Polypeptids/Proteins erhalten bleibt. Bei der Substitution einer Aminosäure durch eine andere Aminosäure mit gleichen oder äquivalenten oder ähnlichen chemisch-physikalischen Eigenschaften spricht man von einem "konservativen Austausch" oder "semikonservativen Austausch". Beispiele für physikalischchemische Eigenschaften einer Aminosäure sind beispielsweise die Hydrophobie oder die Ladung. Dem Fachmann ist bekannt, welche Aminosäuresubstitution einen konservativen oder semikonservativen Austausch darstellt. Das allgemeine Fachwissen erlaubt es darüber hinaus, dass der Fachmann erkennen, identifizieren und nachweisen kann, welche Aminosäuredeletionen und -additionen für die Funktionalität des Resistenzproteins RZ-3 unschädlich sind und an welchen Positionen diese möglich sind. Dem Fachmann ist bewusst, dass im Falle des vorliegenden NBS-LRR-Proteins für Modifikationen der Aminosäuresequenz (Substitutionen, Deletionen oder Additionen von einer oder mehrerer Aminosäuren) insbesondere die Funktionalität der oben definierten konservierten Domänen erhalten bleiben muss und dass daher in diesen Domänen nur begrenzt vorstehende Modifikationen möglich sind. Die Nukleotidsequenz dieser Ausführungsform kodiert dann für ein Derivat bzw. für eine abgeleitete Aminosäuresequenz, wenn die Nukleotidsequenz zu mindestens 80%, 85%, 90%, 92%, 94%, 96%, 97%, 98%, 99% homolog oder identisch ist zu der Nukleotidsequenz nach a) oder b) ist. Vorzugsweise können solche Nukleotidsequenzen, welche für ein Derivat bzw. für eine abgeleitete Aminosäuresequenz kodieren, entweder direkt oder indirekt (beispielsweise via Amplifikations- oder Replikationsschritte) aus einer Ausgangsnukleotidsequenz, welche über die Gesamtlänge oder zumindest teilweise der SEQ ID NO: 1 oder einer anderen hier offenbarten Sequenz entspricht, erzeugt werden.

In einer weiteren Ausführungsform des erfindungsgemäßen Nukleinsäuremoleküls umfasst das Nukleinsäuremolekül die Nukleotidsequenz nach e). Diese Nukleotidsequenz kodiert für ein Polypeptid, welches eine Aminosäuresequenz aufweist, die zu mindestens 80%, 85%, 90%, 92%, 94%, 96%, 97%, 98%, 99% identisch ist zu einer Aminosäuresequenz, welche durch die Nukleotidsequenz nach a) oder b) kodiert wird.

In einer weiteren Ausführungsform des erfindungsgemäßen Nukleinsäuremoleküls umfasst das Nukleinsäuremolekül die Nukleotidsequenz nach f). Die Nukleotidsequenz kodiert für ein Polypeptid, das mindestens eine Nukleotid-bindende Domäne (NBS) entsprechend den Aminosäurepositionen 168-227 der SEQ ID NO: 2 oder entsprechend den Aminosäurepositionen 182-241 der SEQ ID NO: 3, mindestens eine Leuzin-reiche Domäne (LRR) entsprechend den Aminosäurepositionen 591-613 der SEQ ID NO: 2 oder entsprechend den Aminosäurepositionen 605-627 der SEQ ID NO: 3 und mindestens eine interne repetitive Domäne (IR) entsprechend den Aminosäurepositionen 1013-1072 der SEQ ID NO: 2 oder entsprechend den Aminosäurepositionen 1027-1086 der SEQ ID NO: 3 umfasst. Besonders bevorzugt liegen diese Domänen in dem Polypeptid sequentiell vom N- zum C-Terminus in der Reihenfolge NBS - LRR - IR vor, wobei zwischen Domänen jeweils ein oder mehrere weitere Aminosäuren anwesend sein können.

Die vorliegende Erfindung betrifft weiterhin auch ein Polypeptid, welches in der Lage ist eine Resistenz gegenüber einem Pathogen in einer Pflanze, in welcher das Polypeptid exprimiert wird, zu vermitteln und welches durch das erfindungsgemäße Nukleinsäuremolekül kodiert wird, wobei der Pathogen vorzugsweise BNYVV ist und/oder die Pflanze vorzugsweise eine Pflanze der Gattung Beta, insbesondere eine Pflanze der Spezies *Beta vulgaris*, ist. Besonders bevorzugt weist das Polypeptid eine Aminosäuresequenz gemäß SEQ ID NO: 2 oder gemäß SEQ ID NO: 3 auf. Bei dem Polypeptid kann es sich um ein isoliertes Polypeptid handeln.

In einem weiteren Aspekt betrifft die vorliegende Erfindung einen Vektor, welcher das erfindungsgemäße Nukleinsäuremolekül umfasst. Bei dem Vektor kann es sich um ein Plasmid, ein Cosmid, eine Phage oder einen Expressionsvektor, einen Transformationsvektor, Shuttle-Vektor oder Klonierungsvektor handelt, er kann doppel- oder einzelsträngig, linear oder zirkulär sein oder kann einen prokaryotischen oder eukaryotischen Wirt entweder durch Integration in dessen Genom oder extrachromosomal transformieren. Bevorzugt ist das erfindungsgemäße Nukleinsäuremolekül in einem Expressionsvektor mit einer oder mehreren regulatorischen Sequenzen, welche die Transkription und optional die Expression in einer prokaryotischen oder eukaryotischen Wirtszelle erlauben, operativ verknüpft. Beispielsweise steht das Nukleinsäuremolekül unter der Kontrolle eines geeigneten Promotors oder eines Terminators. Geeignete Promotoren können Promotoren sein, welche konstitutiv induziert werden (Bsp.: 35S-Promoter aus dem "Cauliflower mosaic virus" (Odell et al. 1985), besonders geeignet sind solche Promotoren, welche pathogen-induzierbar sind (Bsp.: PR1-Promotor aus Petersilie (Rushton et al., 1996). Besonders geeignete Pathogen-induzierbare Promotoren sind synthetische bzw. chimäre Promotoren, welche in der Natur nicht vorkommen, aus mehreren Elementen zusammengesetzt sind und einen Minimalpromotor beinhalten sowie stromaufwärts des Minimalpromotors mindestens ein cis-regulatorisches Element aufweisen, welches als Bindungsstelle für spezielle Transkriptionsfaktoren dient. Chimäre Promotoren werden den gewünschten Anforderungen nach konzipiert und werden durch unterschiedliche Faktoren induziert oder reprimiert. Beispiele für solche Promotoren finden sich in WO 00/29592, WO 2007/147395 und WO 2013/091612. Ein geeigneter Terminator ist beispielsweise der nos-Terminator (Depicker et al., 1982).

Zusätzlich zu den oben beschriebenen Vektoren, stellt die vorliegende Erfindung auch ein Verfahren bereit, das die Einbringung eines beschriebenen Vektors in eine Wirtszelle umfasst. Der Vektor kann beispielsweise durch Konjugation, Mobilisation, biolistische Transformation, Agrobakterium-vermittelte Transformation, Transfektion, Transduktion, Vakuuminfiltration oder Elektroporation eingebracht werden. Solche Verfahren ebenso wie Verfahren zur Präparation von beschriebenen Vektoren sind dem Fachmann geläufig (Sambrook et al. 2001).

In einem weiteren Aspekt betrifft die vorliegende Erfindung eine Wirtszelle, welche das erfindungsgemäße Nukleinsäuremolekül oder den Vektor der vorliegenden Erfindung umfasst. Eine Wirtszelle im Sinne der Erfindung kann eine prokaryotische (z.B. bakteriell) Zelle oder eine Hefezelle sein. Vorzugsweise ist die Wirtszelle ein Agrobakterium wie *Agrobacterium tumefaciens* oder *Agrobacterium rhizogenes*, welche das erfindungsgemäße Nukleinsäuremolekül oder den Vektor der vorliegenden Erfindung umfasst. Dem Fachmann sind sowohl zahlreiche Methoden wie Konjugation oder Elektroporation bekannt, mit denen er das erfindungsgemäße Nukleinsäuremolekül oder den Vektor der vorliegenden Erfindung in ein Agrobakterium einbringen kann, als auch Methoden wie diverse Transformationsverfahren (biolistische Transformation, Agrobakterium-vermittelte Transformation), mit denen er das erfindungsgemäße Nukleinsäuremolekül oder den Vektor der vorliegenden Erfindung in eine Pflanzenzelle einbringen kann (Sambrook et al. 2001).

In einem weiteren Aspekt betrifft die vorliegende Erfindung eine transgene Pflanzenzelle, welche das erfindungsgemäße Nukleinsäuremolekül als Transgen oder den Vektor der vorliegenden Erfindung umfasst. Eine solche transgene Pflanzenzelle ist beispielsweise eine Pflanzenzelle, welche mit dem erfindungsgemäßen Nukleinsäuremolekül oder mit dem Vektor der vorliegenden Erfindung, vorzugsweise stabil, transformiert ist. In einer bevorzugten Ausgestaltung der transgenen Pflanzenzelle ist, das Nukleinsäuremolekül mit einer oder mehreren regulatorischen Sequenzen, welche die Transkription und optional die Expression in der Pflanzenzelle erlauben, operativ verknüpft. Das Gesamtkonstrukt aus dem erfindungsgemäßen Nukleinsäuremolekül und der/den regulatorischen Sequenzen stellt dann das Transgen dar. Solche regulatorische Sequenzen sind beispielsweise ein Promoter oder ein Terminator. Dem Fachmann sind zahlreiche in Pflanzen anwendbare, funktionelle Promotoren und Terminatoren bekannt. Bevorzugt zeigt eine transgene Pflanzenzelle der vorliegenden Erfindung, insbesondere eine Zelle einer Pflanze der Gattung Beta, gegenüber einem Pathogen, insbesondere BNYVV, eine höhere Resistenz als eine korrespondierende nicht-transformierte Pflanzenzelle (die Pflanzenzelle ohne das Transgen). Das Level der Resistenz beispielsweise gegenüber BNYVV kann in Pflanzen der Gattung Beta qualitativ durch Bestimmung von Boniturnoten festgelegt werden (Boniturnotenschemata für Pflanze der Gattung Beta sind aus dem Stand der Technik bekannt, beispielsweise für Zuckerrüben Mechelke (1997)). Eine höhere Resistenz zeigt sich in einer Verbesserung der Resistenz um mindestens eine Boniturnote, um mindestens zwei Boniturnoten, um mindestens drei oder mehr Boniturnoten. Weiterhin betrifft die vorliegende Erfindung auch ein Verfahren zur Herstellung einer transgenen Pflanzenzelle der vorliegenden Erfindung, welches einen Schritt des Einbringens des erfindungsgemäßen Nukleinsäuremoleküls oder des Vektors der vorliegenden Erfindung in eine pflanzliche Zelle umfasst. Beispielsweise kann das Einbringen durch Transformieren stattfinden, vorzugsweise durch stabiles Transformieren. Die geeigneten Techniken zur Einbringung wie biolistische Transformation, Agrobakterium-vermittelte Transformation oder Elektroporation sind dem Fachmann bekannt (Sambrook et al. 2001).

In einem weiteren Aspekt betrifft die vorliegende Erfindung eine transgene Pflanze oder einen Teil davon, umfassend eine vorstehend beschriebene transgene Pflanzenzelle. Ein Teil kann dabei eine Zelle, ein Gewebe, ein Organ oder ein Zusammenschluss von mehreren Zellen, Geweben, oder Organen sein. Ein Zusammenschluss von mehreren Organen ist z.B. eine Blüte oder ein Samen. In einer besonderen Ausgestaltung betrifft die Erfindung einen Samen von der transgenen Pflanze, wobei der Samen das erfindungsgemäße Nukleinsäuremolekül als Transgen umfasst. Bevorzugt zeigt eine transgene Pflanze der vorliegenden Erfindung, insbesondere eine Pflanze der Gattung Beta, gegenüber einem Pathogen, insbesondere BNYW, eine höhere Resistenz als eine korrespondierende nicht-transformierte Pflanze (Pflanze ohne das Transgen). Das Level der Resistenz beispielsweise gegenüber BNYVV kann in Pflanzen der Gattung Beta qualitativ durch Bestimmung von Boniturnoten festgelegt werden (Boniturnotenschemata für Pflanze der Gattung Beta sind aus dem Stand der Technik bekannt, beispielsweise für Zuckerrüben Mechelke (1997)). Eine höhere Resistenz zeigt sich in einer Verbesserung der Resistenz um mindestens eine Boniturnote, um mindestens zwei Boniturnoten, um mindestens drei oder mehr Boniturnoten. Ferner stellt die Erfindung ein Verfahren zur Herstellung einer transgenen Pflanze bereit, welches einen Schritt des Einbringens des erfindungsgemäßen Nukleinsäuremoleküls oder des Vektors der vorliegenden Erfindung in eine pflanzliche Zelle und optional einen Schritt der Selektion einer transgenen Pflanzenzelle umfasst. Weiterhin ist ein solches Verfahren zur Herstellung einer transgenen Pflanze durch einen anschließenden Schritt gekennzeichnet, der die Regenerierung der transgenen Pflanze aus der im ersten Schritt erzeugten transgenen Pflanzenzelle einschließt. Methoden zur Regeneration sind dem Fachmann aus dem Stand der Technik bekannt.

In einem weiteren Aspekt betrifft die vorliegende Erfindung auch ein Verfahren zur Vermittlung oder Erhöhung einer Resistenz gegenüber einem Pathogen, insbesondere BNYVV, in einer Pflanze, bevorzugt einer Pflanze der Gattung Beta, welche einen Schritt des Transformierens einer Pflanzenzellen mit dem erfindungsgemäßen Nukleinsäuremolekül oder dem Vektor der vorliegenden Erfindung umfasst. Bevorzugt führt dieses Verfahren zu einer Verbesserung der Resistenz um mindestens eine Boniturnote, besonders bevorzugt zu einer Verbesserung der Resistenz um mindestens zwei, drei oder mehr Boniturnoten. Boniturnotenschemata für Pflanze der Gattung Beta sind aus dem Stand der Technik bekannt, beispielsweise für Zuckerrüben Mechelke (1997).

In einem weiteren Aspekt betrifft die vorliegende Erfindung eine regulatorische Sequenz eines Promotors, welche die Expression eines Gens, das das erfindungsgemäße Nukleinsäuremolekül umfasst, kontrolliert, dadurch gekennzeichnet, dass die regulatorische Sequenz in der Lage ist, die Expression einer heterologen DNA-Sequenz infolge einer Pathogeninfektion zu vermitteln oder zu modulieren, und die regulatorische Sequenz ein Nukleinsäuremolekül mit einer Nukleotidsequenz gemäß SEQ ID NO: 1 von den Nukleotiden 1-1403 umfasst. Vorzugsweise ist die heterologe DNA-Sequenz eine Nukleotidsequenz, welche für eine Komponente der pflanzlichen Pathogenabwehr kodiert (Bsp.: Resistenzgene (R-Gene) oder Gene, welche für am Signaltransfer beteiligte Enzyme wie Kinasen oder Phosphatasen sowie für G-Protein kodieren) oder welche für einen pathogenen Effektor kodiert (sog. Avirulenzgene (avr)). Weiterhin erfasst die vorliegende Erfindung ein rekombinantes DNA-Molekül, welches die vorstehend beschriebene regulatorische Sequenz umfasst. Vorzugsweise ist das rekombinante DNA-Molekül mit einer heterologen DNA-Sequenz operativ verknüpft.

In einem weiteren Aspekt bezieht sich die vorliegende Erfindung auf eine Wirtszelle, welche mit der vorstehend beschriebenen regulatorischen Sequenz oder mit dem genannten rekombinanten DNA-Molekül transformiert ist sowie auf eine transgene Pflanze, Pflanzengewebe oder Pflanzenzelle, welche die regulatorische Sequenz oder das rekombinante DNA-Molekül als Transgen umfasst. Weiterhin stellt die Erfindung ein Verfahren zur Herstellung einer transgenen Pflanzenzelle, welche einen Schritt des Einbringens der regulatorischen Sequenz der Erfindung oder des rekombinanten DNA-Molekül und optional einen Schritt der Selektion einer transgenen Pflanzenzelle umfasst. Ferner stellt die Erfindung ein Verfahren zur Herstellung einer transgenen Pflanze bereit, welche einen Schritt des Einbringens der regulatorischen Sequenz der Erfindung oder des rekombinanten DNA-Molekül in eine pflanzliche Zelle und optional einen Schritt der Selektion einer transgenen Pflanzenzelle umfasst. Weiterhin ist ein solches Verfahren zur Herstellung einer transgenen Pflanze durch einen anschließenden Schritt gekennzeichnet, der die Regenerierung der transgenen Pflanze aus der im ersten Schritt erzeugten transgenen Pflanzenzelle einschließt.

Wie bereits oben ausgeführt, fand die Identifizierung des RZ-3-Resistenzgens im Zuge eines Map-based-Cloning-Prozesses statt. Der durchgeführte Prozess umfasste beispielsweise die Schritte: Genetische Feinkartierung, physikalische Kartierung, Aufbau einer sehr großen spaltenden Population von über 8000 F2-spaltenden Nachkommen, Rekombinanten-Screen, Markerentwicklung in der Zielregion, vergleichende BAC Sequenzierung in resistenten und sensitiven Genotypen, bioinformatische Analysen, Proteinvorhersagen und Vergleich der Proteine. Solche langwierigen Entwicklungsarbeiten sind immer überaus kostspielig und es ist ungewiss, ob es tatsächlich gelingt das Gen zu identifizieren. Nach Integration des RZ-3-Lokus aus *Beta vulgaris* subsp. *maritima* in einer Pflanze der Gattung Beta, nämlich in Zuckerrübe (*Beta vulgaris* subsp. *vulgaris* var. *altissima*) wurden für die Verfolgung des RZ-3-Genomsegments in der Feinkartierung Marker mit guten diagnostischem Wert entwickelt, was sich als besonders schwierig herausstellte, da die Zielregion über weite Bereiche hin repetitiv ist. Überraschenderweise gelang dennoch die Entwicklung von einigen wenigen diagnostischen Markern, welche teilweise auch nur mit einer bestimmten Markertechnik wie Pyrosequenzen also als PSQ-Marker funktionierten oder nullallelisch waren.

Trotz dieser technischen Schwierigkeiten konnte durch umfangreiche Analyse unter Verwendung dieser Marker eine Eingrenzung des RZ-3-Lokus auf eine genomische Region von 0,67 cM erreicht werden. Dies entspricht einer physikalischen Länge von etwa 340.000 bp. Trotz intensiver Weiterentwicklungen war es nur noch beschränkt möglich, die *Beta vulgaris* subsp. *maritima*-Introgression um das Gen herum Marker-gestützt weiter zu reduzieren und Kandidatengene für das RZ-3-Gen zu identifizieren. Eine weitere Verkürzung der Introgression ist jedoch aus züchterischer Sicht in jedem Fall wünschenswert, um potentiell vorhandenen "Linkage Drag", eng gekoppelt zu dem RZ-3-Gen, zu eliminieren. In mehreren Schritten mittels Feinkartierung und unter Einbindung von Sequenzinformation aus physikalischen Karten konnte schließlich eine Zielregion auf nur noch etwa 0,07 cM eingeengt werden. Dies wurde jedoch nur dadurch möglich, dass insgesamt 8004 Pflanzen untersucht wurden, darunter informative rekombinante BC2S1 oder BC2S2 Pflanzen, welche intensiv mit jeweils 90-180 Nachkommen analysiert wurden. Dieses war notwendig, da die Resistenzausprägung aus unbekannten Gründen nicht immer eindeutig war. Diese Nachkommen wurden einzelpflanzenweise genotypisiert und parallel phänotypisiert. Mittels statistischer Verfahren (t-Test, Poweranalyse) wurden die Phänotypen der informativen Rekombinanten (homozygot resistent - RR; heterozygot resistent - Rs; homozygot anfällig - ss) nachgewiesen und damit Rückschlüsse auf den Genotyp der informativen Rekombinanten gezogen.

In der relativ kleinen Zielregion von etwa 38.000 bp konnten in dem anfälligen Genotyp zehn Gene annotiert werden. Für diese Zielregion wurden aus einer resistenten BAC Bibliothek überlappende Klone mit Hilfe von neuen Markern, welche spezifisch den Zielbereich beschreiben, identifiziert und schließlich sequenziert. Wegen der Repetivität der Zielregion zeigte die Sequenz des anfälligen Genotyps zahlreiche kleine Abschnitte mit unbekanntem Sequenzinhalt. Aus diesem Grund war die Assemblierung der RR- und ss-Sequenzen besonders anspruchsvoll. Es konnte dennoch ein putatives Resistenzgen identifiziert werden. Dieses enthielt in nahezu allen ss-Genotypen ein Retrotransposon mit einer Länge von ca. 8000 bp zwischen der LRR-Domäne und der IR-Domäne, was in RR-Genotypen nicht detektiert werden konnte. Eine Aminosäuresequenz, vorhergesagt aus der putativen Resistenzgensequenz, zeigt, dass das Gen mutmaßlich für ein NB-ARC-LRR-Protein kodiert. Es kann vermutet werden, dass diese Insertion des Retrotransposons die Funktion des Gens in anfälligen ss-Genotypen zerstört, denn sie trennt die Internal Repeat-Domäne (IR) von den beiden anderen Domänen (NB-ARC und LRR).

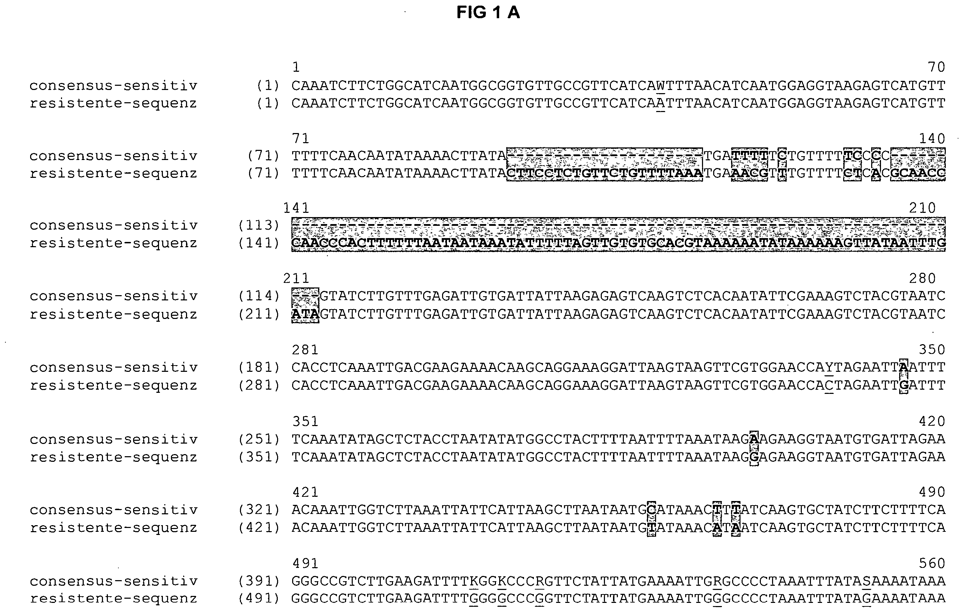

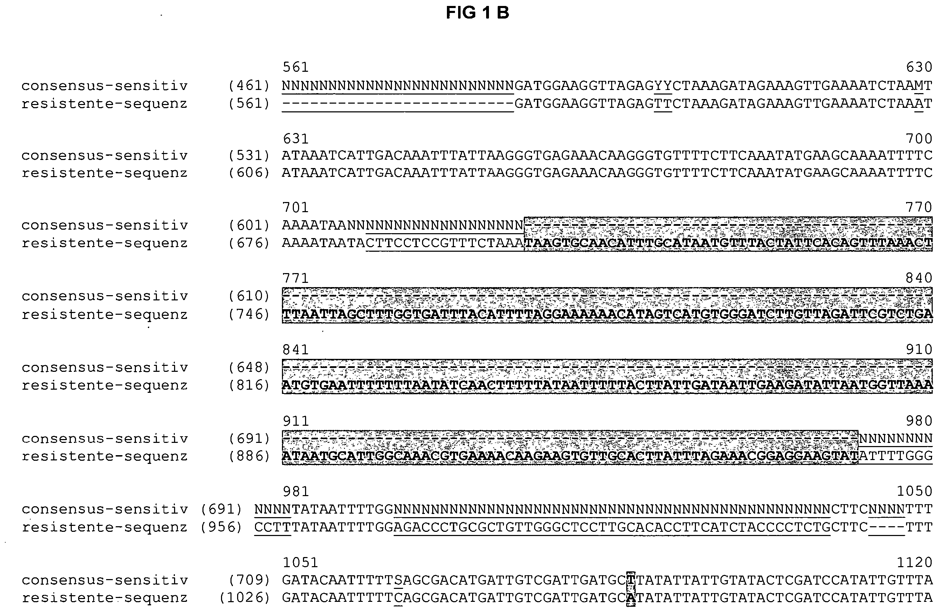

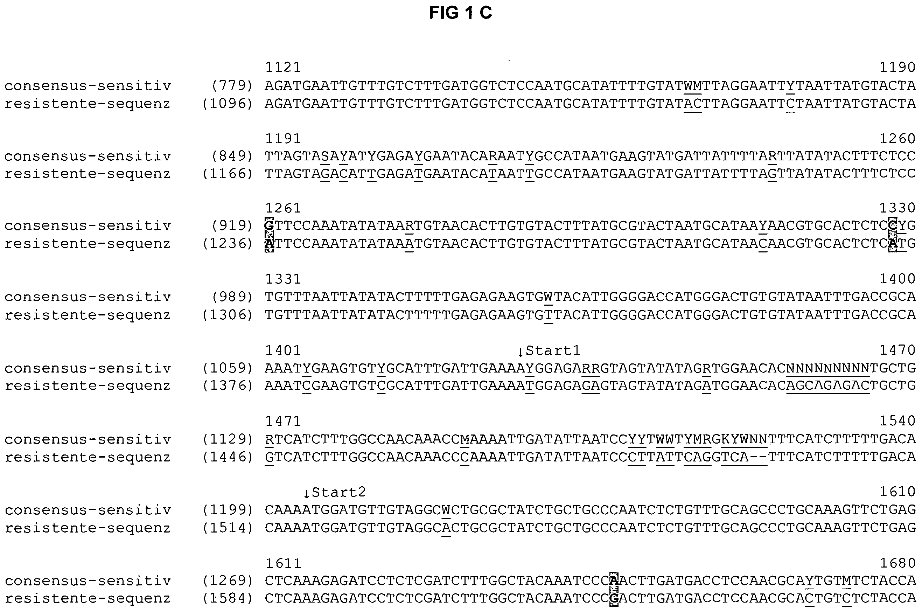

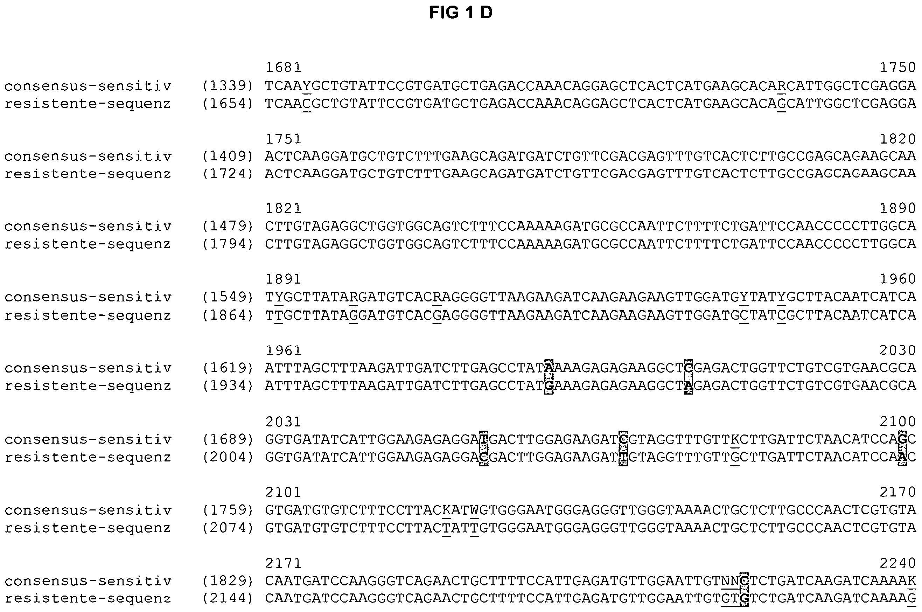

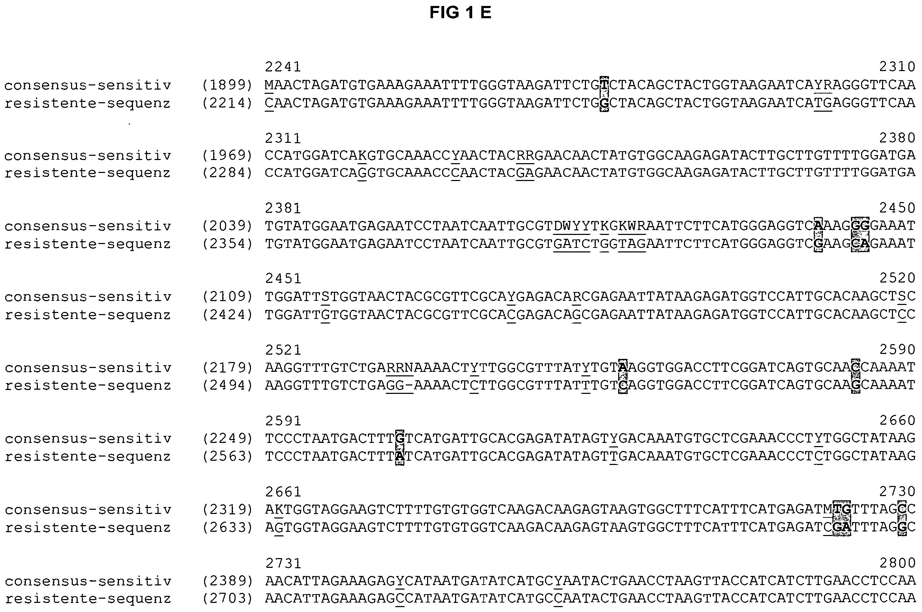

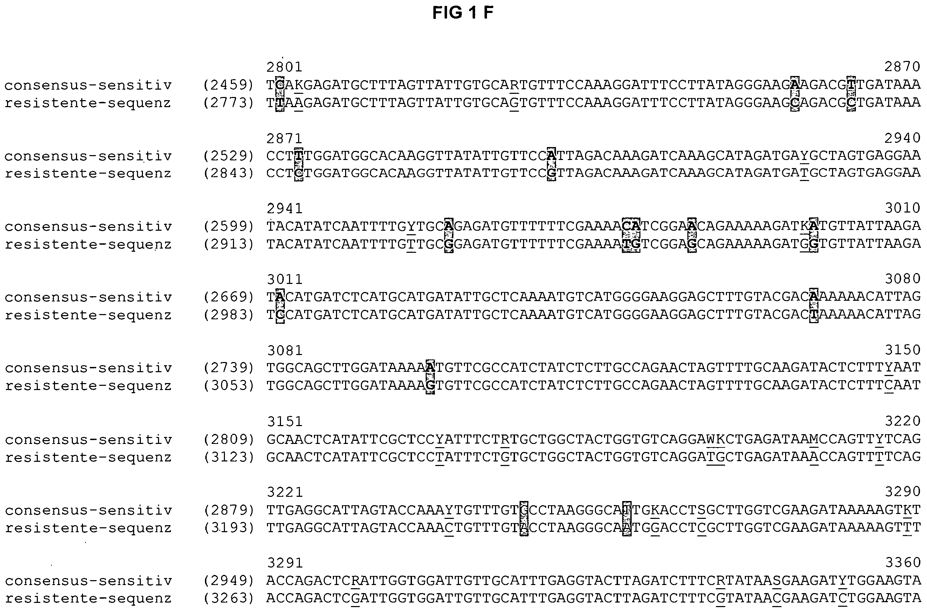

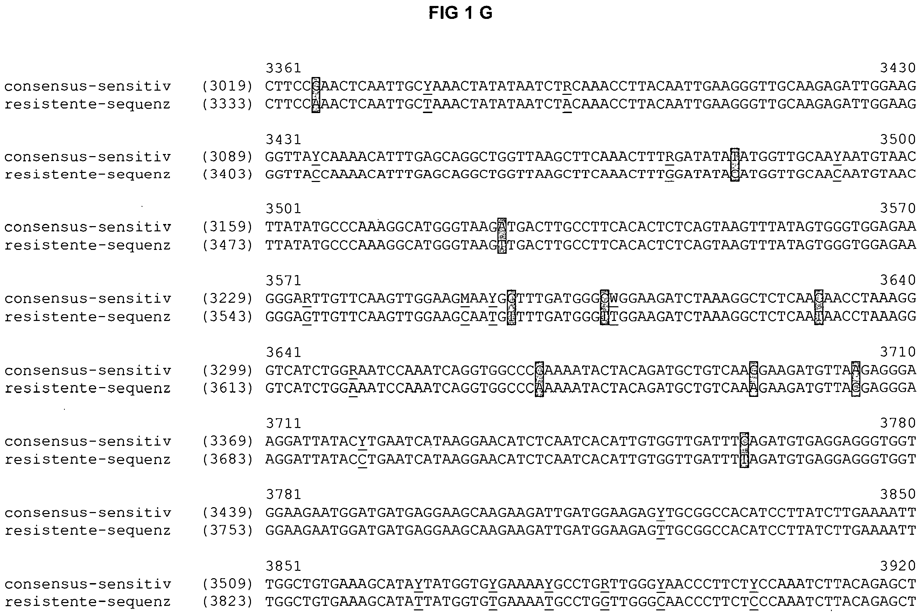

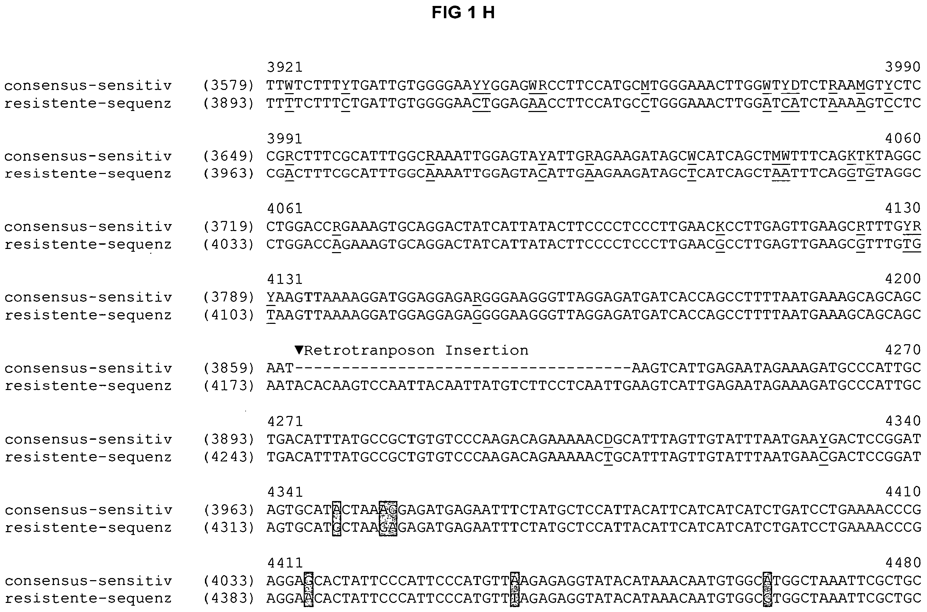

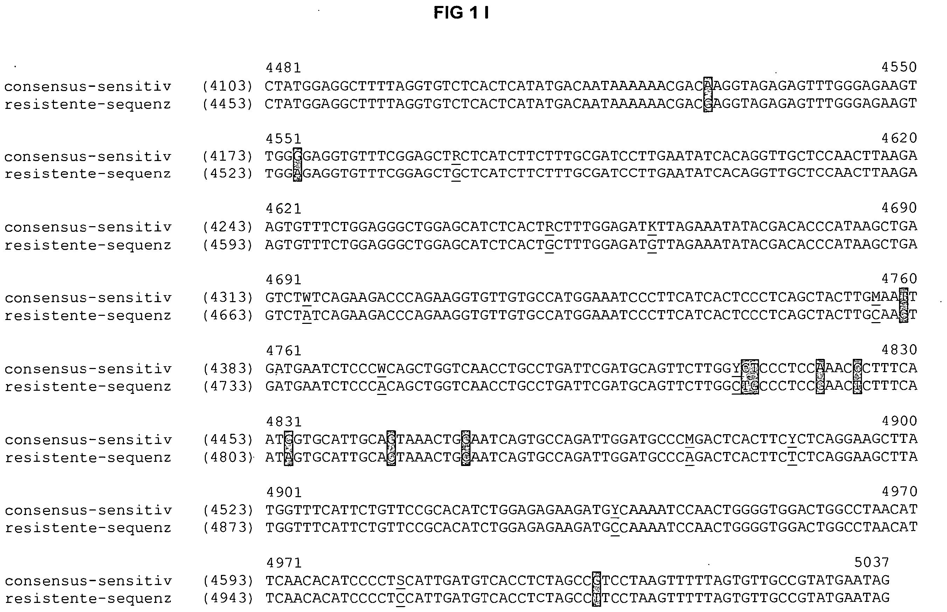

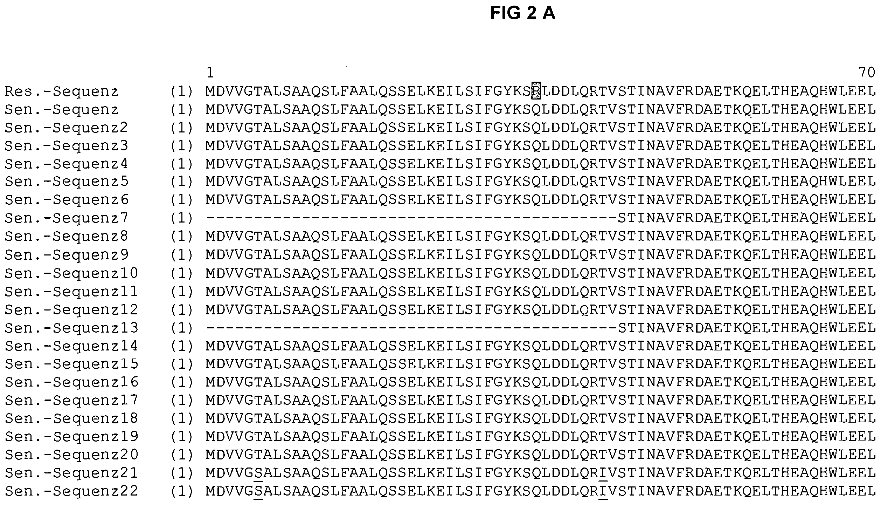

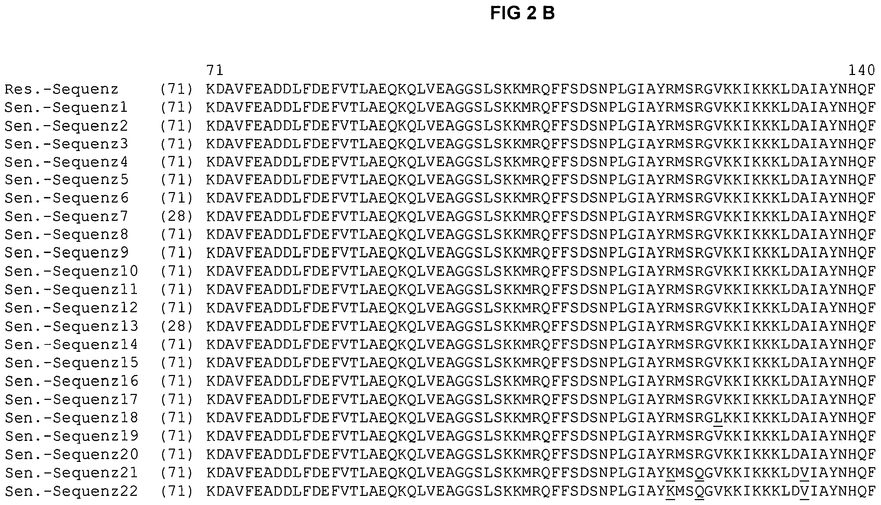

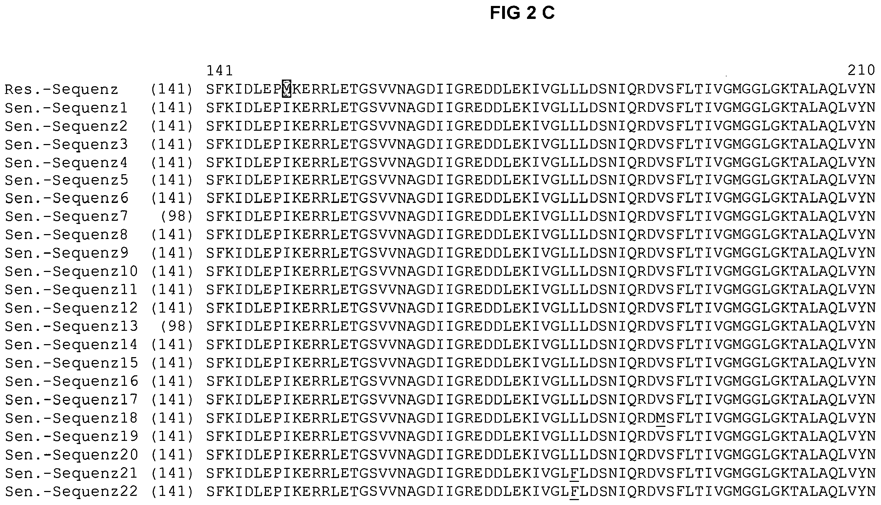

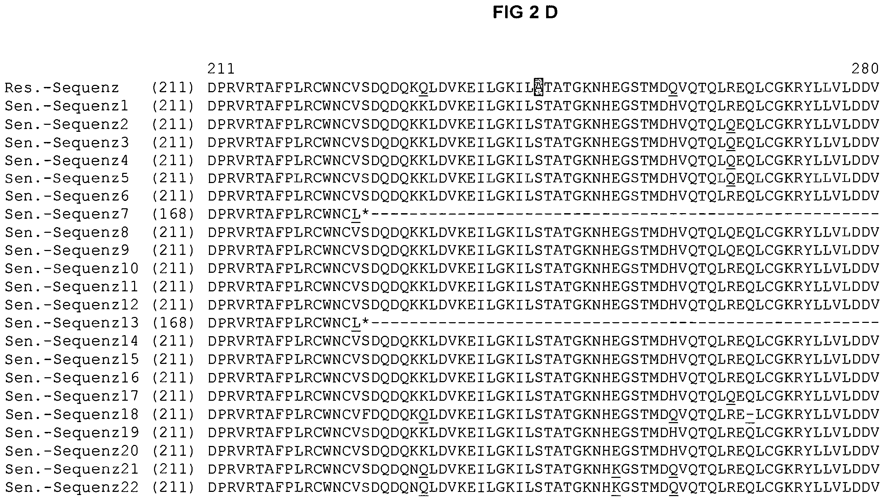

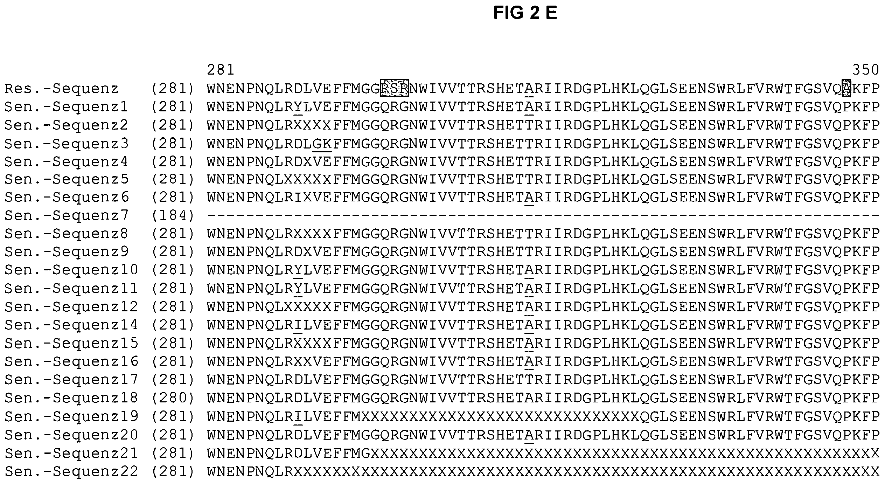

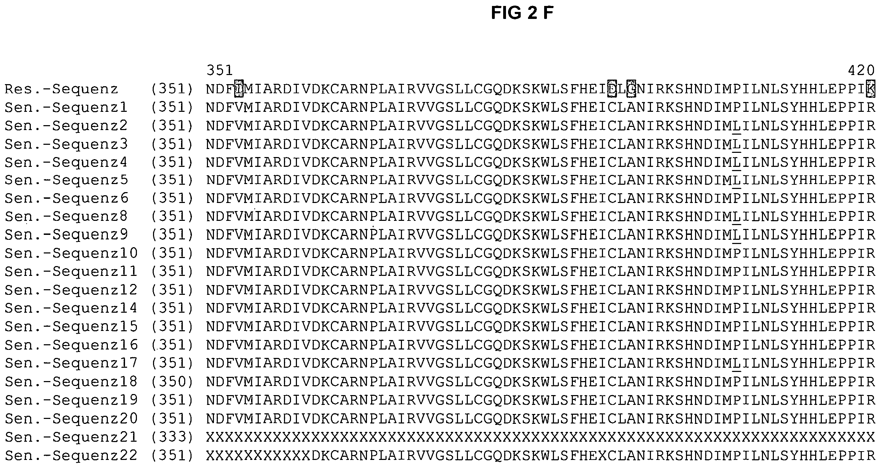

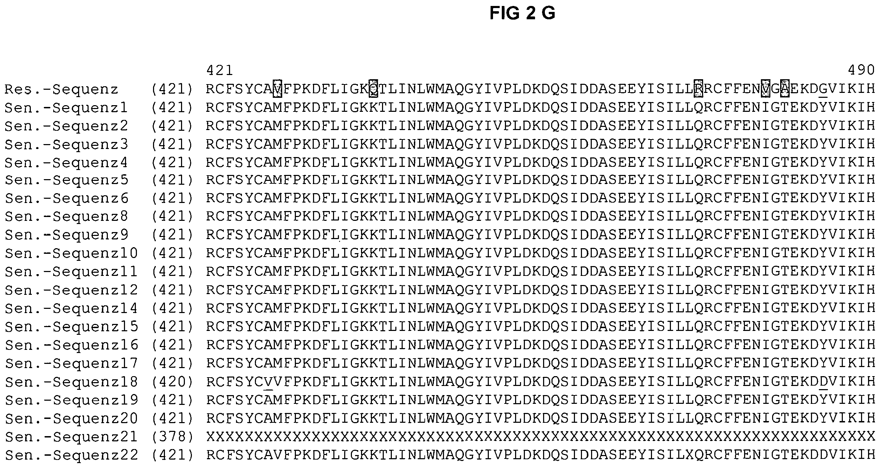

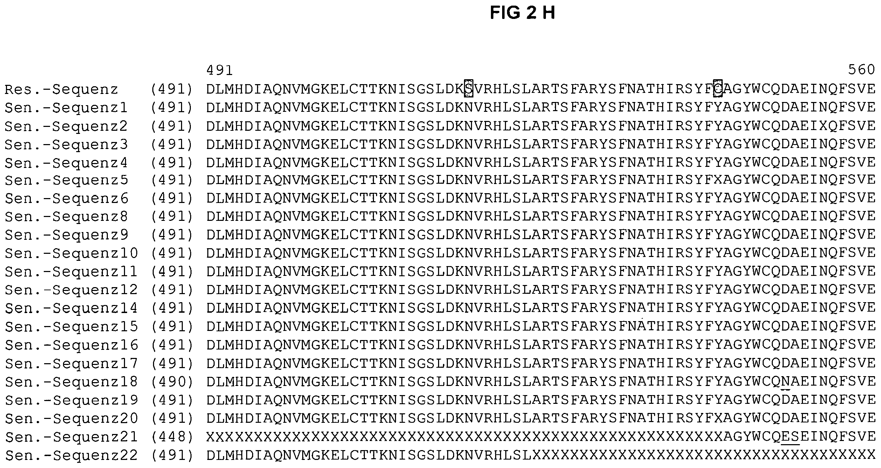

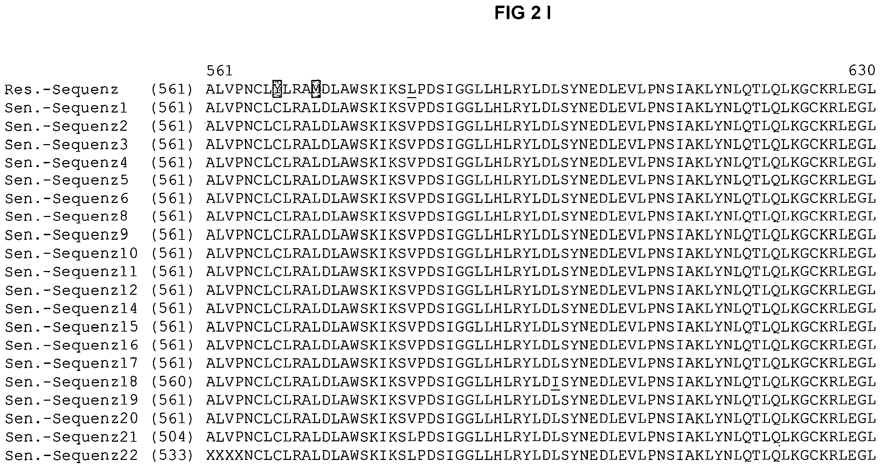

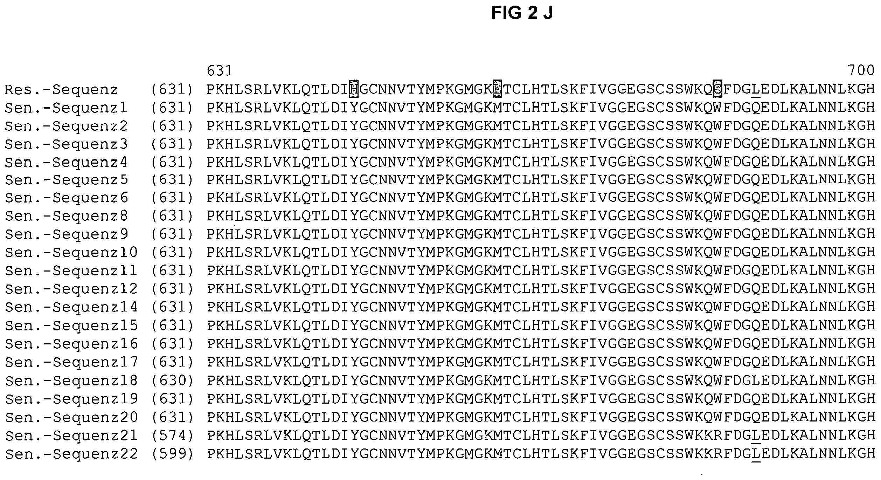

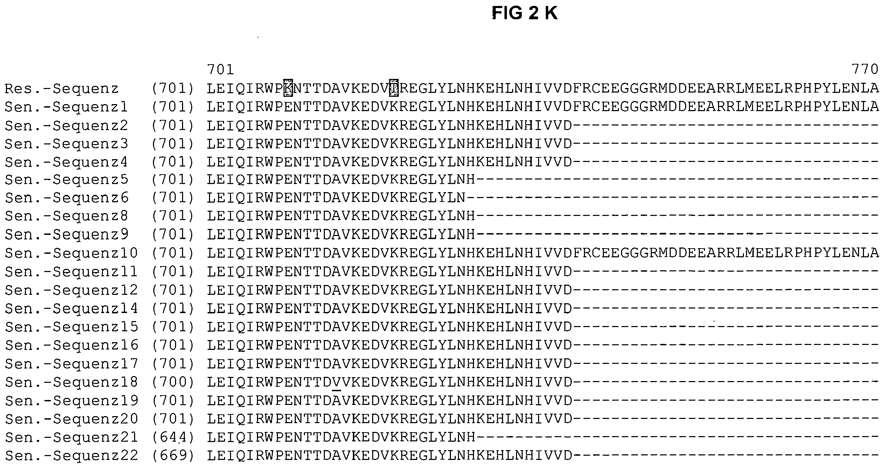

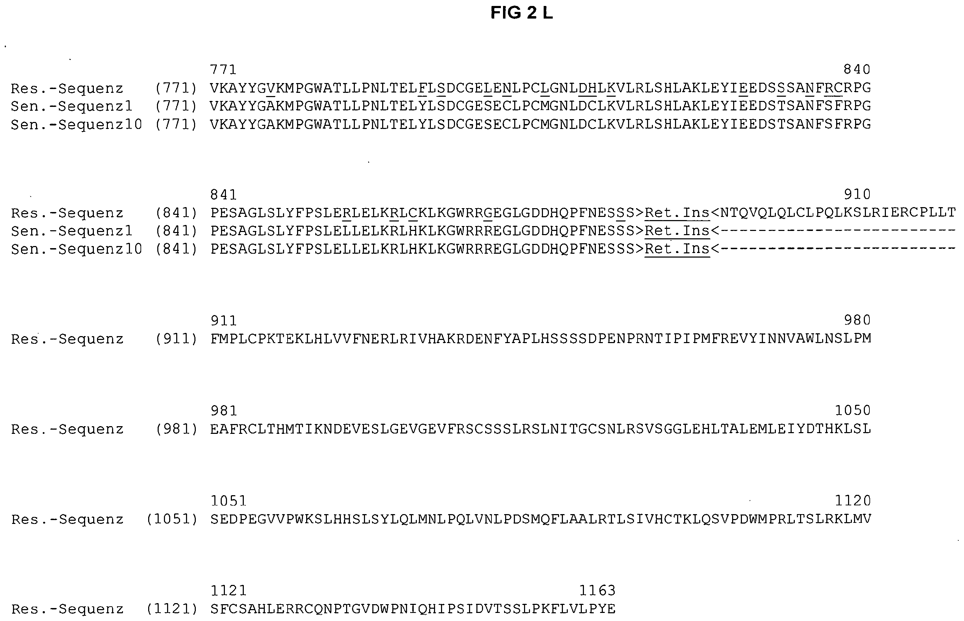

Der Vergleich des NBS-LRR-Gens in ss-Genotypen mit demjenigen in RR-Genotypen zeigte ferner diagnostische Polymorphismen, welche den Figuren 1, 2 und 3 entnommen werden können. Basierend auf diesen Polymorphismen im NBS-LRR Gen wurden Marker entwickelt und in einem breiten Set von nahezu 100 ss-und RR-Genotypen getestet. Die Markermuster aber auch die vergleichende Sequenzierung im Zielgen haben bestätigt, dass die Insertion nahezu immer mit der Sensitivität gekoppelt ist. Es fanden sich jedoch wenige ss-Genotypen, die die Retrotransposon-Insertion nicht aufwiesen und dennoch anfällig waren. Diese ss-Genotypen konnten jedoch eindeutig mittels Marker, welche die diagnostischen Polymorphismen gemäß Figuren 1, 2 und/oder 3 beschreiben, von den RR-Genotypen unterschieden werden.

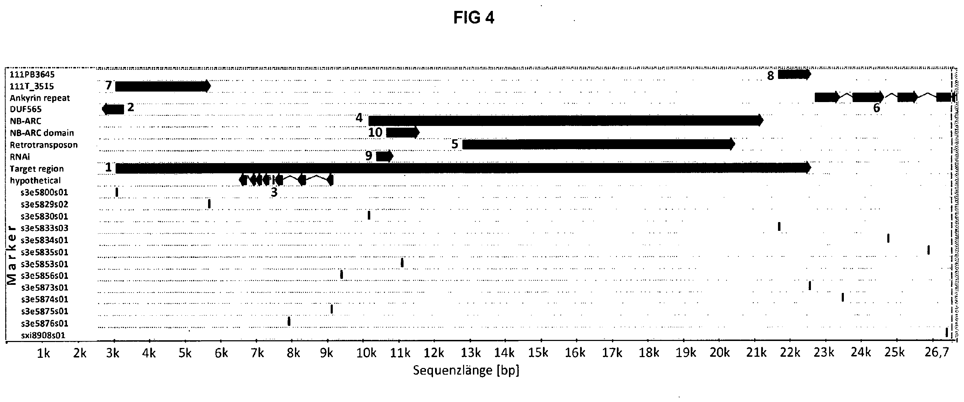

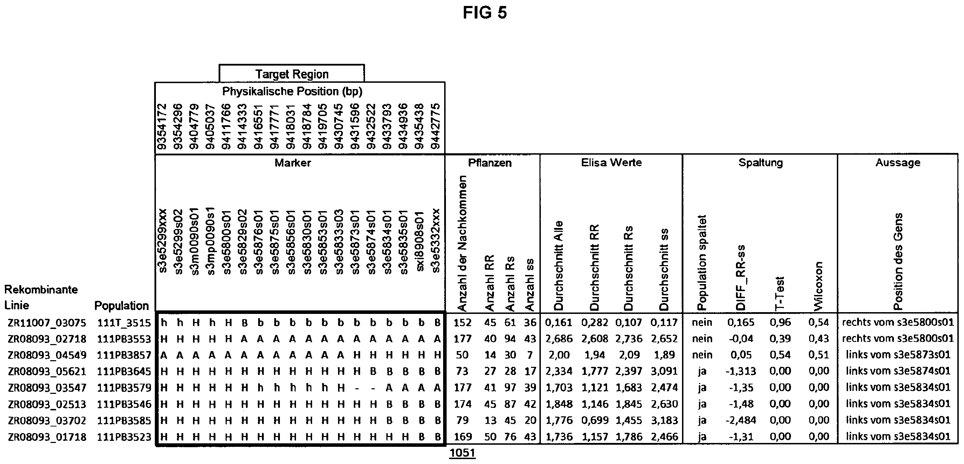

In der analysierten Population wurden Rekombinaten in der Zielregion identifiziert, die eine Rekombination zwischen dem NBS-LRR-Gen und dem stromabwärts gelegenen, benachbarten annotierten putativen Gen, das für ein Ankyrin repeat Protein kodieren könnte, zeigen. Im Falle von zwei Pflanzen sind die Rekombinationen zwischen dem NBS-LRR-Gen und dem stromaufwärts gelegenen, benachbarten annotierten putativen Gen, das für ein DUF565 Protein (Protein mit unbekannter Funktion) kodieren könnte, zu finden. Durch die Resistenzanalyse der Nachkommenschaft von allen diesen rekombinanten Pflanzen (Ein-Gen-Entfernung stromaufwärts und stromabwärts des NBS-LRR-Gens) konnte ganz eindeutig bewiesen werden, dass das Gen zwischen dem Ankyrin repeat Gen und dem DUF565-Gen, nämlich das hier charakterisierte NBS-LRR-Gen für die Resistenz im RR-Genotypen verantwortlich ist. Figur 4 zeigt die physikalische Karte der RZ-3-Zielregion mit den entwickelten Markern. Die Genotypdaten von acht engen rekombinanten Linien, sowie die statistische Analyse deren Nachkommenschaft ist in der Figur 5 dargestellt.

In einem weiteren Aspekt betrifft die vorliegende Erfindung ein Verfahren zur Identifikation eines Nukleinsäuremoleküls, welches ein Protein kodiert, das in der Lage ist, eine Resistenz gegenüber dem Pathogen BNYVV in einer Pflanze der Gattung Beta, in welcher das Protein exprimiert wird, zu vermitteln. Das Verfahren umfasst das Nachweisen der Abwesenheit einer Insertion in der kodierenden Nukleotidsequenz des Nukleinsäuremoleküls. Bevorzugt umfasst das Verfahren das Nachweisen der Abwesenheit einer Insertion, insbesondere eines Retrotransposons, in der kodierenden Nukleotidsequenz des Nukleinsäuremoleküls. Das Retrotransposon kann beispielsweise etwa 500 bp, etwa 1000 bp, etwa 2000 bp, etwa 4000 bp, etwa 8000 bp oder mehr als etwa 8000 bp lang sein. In einer besonderen Ausgestaltung des Verfahrens ist das Nukleinsäuremolekül das erfindungsgemäße Nukleinsäuremolekül wie oben beschrieben und kodiert das Resistenz-vermittelnde RZ-3-Gen oder ein funktionelles Homolog von RZ-3. Vorzugsweise handelt es sich bei der Pflanze der Gattung Beta um *Beta vulgaris* subsp. *maritima* oder *Beta vulgaris* subsp. *vulgaris* var. *altissima* (Zuckerrübe). Dem Fachmann ist bekannt, welche Methoden geeignet sind, die Abwesenheit der Insertion nachzuweisen. Beispielsweise kann der Fachmann in Kenntnis der hier offenbarten erfindungsgemäßen Nukleinsäuremoleküle molekulare Marker entwickeln, welche die Anwesenheit oder die Abwesenheit einer Insertion in oben beschriebener Region im NBS-LRR-Gen nachweisen (exemplarisches Vorgehen siehe Beispiele). Die vorliegende Erfindung schließt solche Marker sowie deren Verwendung zum Nachweis der An- oder Abwesenheit der Insertion zur Selektion resistenter, inbesondere BNYVV-resistenter, Pflanzen, insbesondere *Beta vulgaris* subsp. *maritima* oder *Beta vulgaris* subsp. *vulgaris* var. *altissima* (Zuckerrübe) mit ein. Vorzugsweise beschreiben solche Marker Loki an den Insertionsstellen des Retrotransposons. Insertionsstellen meinen Übergangstellen zwischen genomischer DNA und Retrotransposon auf 5'- und/oder 3'-Seite der Insertion. Übergangsstellen sind breit zu definieren und Markerloki können weniger als 1000 Nukleotide, bevorzugt weniger als 800 oder 600 Nukleotide, besonders bevorzugt weniger als 400, 200, 150, 100, 50, 40, 30, 20 oder 10 Nukleotide stromaufwärts oder stromabwärts von einer Insertionsstelle auf der DNA entfernt liegen. Alternativ oder ergänzend zu dem Schritt des Nachweises der An- oder Abwesenheit einer Insertion in der kodierenden Nukleotidsequenz des Nukleinsäuremoleküls, kann das Verfahren auch das Nachweisen von mindestens einem Polymorphismus gemäß Figur 1, 2 und/oder 3, bevorzugt von mindestens zwei oder drei Polymorphismen gemäß gemäß Figur 1, 2 und/oder 3, besonders bevorzugt von mindestens vier, fünf oder mehr Polymorphismen gemäß gemäß Figur 1, 2 und/oder 3 in der kodierenden Nukleotidsequenz des erfindungsgemäßen Nukleinsäuremoleküls unter Verwendung von molekularen Markern, welche die Polymorphismen, insbesondere diagnostische Polymorphismen, erkennen, umfassen. Bevorzugt findet dieser Nachweis unter Verwendung von mindestens einem molekularen Marker pro Polymorphismus, insbesondere pro diagnosistischen Polymorphismus, statt. Dem Fachmann ist bekannt, welche Markertechniken zum Nachweis eines entsprechenden Polymorphismus anzuwenden sind, und wie man molekulare Marker dafür konstruiert (Literatur). Weiterhin erfasst die vorliegende Erfindung molekulare Marker, welche einen Polymorphismus gemäß Figur 1, 2 und/oder 3 beschreiben bzw. detektieren, wie auch die Verwendung eines molekularen Markers zur Detektion eines Polymorphismus gemäß Figur 1, 2 und/oder 3. Weiterhin stellen die vorstehenden Identifikationsverfahren auch Verfahren zur Selektion einer Pflanze, welche eine Resistenz gegenüber BNYVV aufweist, dar. Das Verfahren zur Selektion umfasst einen abschließenden Schritt des Selektierens einer resistenten Pflanze.

Ferner konnte auch gezeigt werden, dass in der untersuchte RR Genotypen stromaufwärts zu RZ-3 (SEQ ID NO: 1) angrenzende genomische DNA-Sequenzabschnitt gemäß SEQ ID NO: 4 und stromabwärts zu RZ-3 (SEQ ID NO: 1) angrenzende genomische DNA-Sequenzabschnitt gemäß SEQ ID NO: 5 aufwies, welche eng-gekoppelt mit dem RZ-3-Gen sind und sich deshalb herausragend eignen als DNA-Regionen zur Entwicklung von diagnostischen Markern für RZ-3. Daher betrifft die vorliegende Erfindung ein Verfahren zur Selektion einer Pflanze, welche eine Resistenz gegenüber BNYVV aufweist. Das Verfahren zur Selektion umfasst die Verwendung eines molekularen Markers auf einer DNA-Sequenz gemäß SEQ ID NO: 4 und/oder auf einer DNA-Sequenz gemäß SEQ ID NO: 5 und einen abschließenden Schritt des Selektierens einer resistenten Pflanze. Dem Fachmann ist bekannt, wie er auf Grundlage der offenbarten Sequenzinformationen Marker entwickelt und einsetzt.

Durch die vorliegende Erfindung können ferner folgende Vorteile für die Züchtung und die Entwicklung neuer resistenter Pflanzenlinien der Gattung Beta erzielt werden: Sequenzinformationen sowie die identifizierten Polymorphismen, welche eine Unterscheidung zwischen resistenten RR- und anfälligen ss-Allelen des offenbarten Gens erlauben, machen die Marker-Entwicklung direkt im Gen möglich, was insbesondere im Hinblick auf die Entwicklung von optimierten Elitelinien ohne "Linkage Drag" eine bedeutende Erleichterung für den Pflanzenzüchter darstellt. Außerdem kann die Kenntnis über die sequentielle Struktur zur Identifizierung von weiteren Resistenzgenen, insbesondere gegen Rizomania, welche beispielsweise teilweise homolog sind, verwendet werden.

Die hier offenbarte Nutzung des resistenten Genallels in cis- oder trans-genetische Ansätzen eröffnet die Möglichkeit neue resistente Sorten der Gattung Beta zu entwickeln, welche eine anhand des Dosiseffekts eine höhere Resistenz aufweisen oder in welchen durch das Stacking des offenbarten Gens mit anderen Resistenzgenen ein Resistenzbruch vermieden werden und die Resistenzausprägung optimiert werden kann. Ferner sind Modifikationen des Gens mittels Tilling oder gezieltem Engineering zur Entwicklung neuer Resistenzallele denkbar.

Ferner betrifft die vorliegende Erfindung die Verwendung des identifizierten resistenten RZ3-Genallels in einem genetischen oder molekularen Stack mit anderen genetischen Elementen, welche agronomisch vorteilhafte Eigenschaften vermitteln können, in einer Pflanze. Hierdurch kann der ökonomische Wert von Kulturpflanzen deutlich gesteigert werden, indem beispielsweise die Ertragsleistung gesteigert wird oder neue Anbauflächen für eine Pflanze erschlossen werden, die zuvor unter Anderem aufgrund biotischen Faktoren wie starkem Pathogendruck oder abiotischen Faktoren wie Trockenheit dem Anbau dieser Pflanze nicht zugänglich waren. Eine agronomisch vorteilhafte Eigenschaft ist beispielsweise eine Toleranz gegenüber einem Herbizid wie Glyphosat, Glufosinat oder ALS-Inhibitoren. Dem Fachmann sind aus dem Stand der Technik zahlreiche weitere Herbizide und deren Anwendbarkeit bekannt. Er kann auf den Stand der Technik zurückgreifen, um Kenntnis zu erlangen, welche genetischen Elemente in welcher Art und Weise zu verwenden sind, um eine entsprechende Toleranz in Pflanzen zu implementieren. Ein weiteres Beispiel für eine agronomisch vorteilhafte Eigenschaft ist eine zusätzliche Pathogenresistenz, wobei Pathogene beispielsweise Insekten, Viren, Nematoden, Bakterien oder Fungi sein können. Durch Kombination unterschiedlicher Pathogenresistenzen/-toleranzen kann beispielsweise eine breite Pathogenabwehr für eine Pflanze erreicht werden, da genetische Elemente untereinander ergänzende Wirkungen aufweisen können. Hierfür sind dem Fachmann als genetische Elemente beispielsweise zahlreiche Resistenzgene bekannt. Ein weiteres Beispiel für eine agronomisch vorteilhafte Eigenschaft ist eine Kühle- oder Frosttoleranz. Pflanzen, welche diese Eigenschaft aufweisen, könnten früher im Jahr bereits ausgesät werden oder könnten länger zum Beispiel auch über Frostperioden im Feld verbleiben, was beispielsweise zu gesteigerten Erträgen führen kann. Auch hier kann der Fachmann auf den Stand der Technik zurückgreifen, um geeignete genetische Elemente zu finden. Weitere Beispiele für agronomisch vorteilhafte Eigenschaften sind Wassernutzungseffizienz, Stickstoffnutzungseffizienz sowie Ertrag. Genetische Elemente, welche eingesetzt werden können, um solche Eigenschaften zu vermitteln, könnten im Stand der Technik gefunden werden.

Dem Fachmann sind weiterhin zahlreichen Modifikationen zur Pathogenabwehr bekannt. Neben den häufig beschriebenen Familien der R-Gene könnten der Avr/R-Ansatz, die Avr-Gen-Komplementierung (WO 2013/127379), die Autoaktivierung eines R-Gens (WO 2006/128444), der HIGS (host induced gene silencing)-Ansatz (z.B. WO2013/050024) oder der VIGS (virus induced gene silencing)-Ansatz vorteilhaft eingesetzt werden. Insbesondere die Autoaktivierung eines R-Gens könnte für die vorliegende Erfindung von Bedeutung sein. Hierfür ist eine Nukleinsäure zu erstellen, die für ein autoaktiviertes Resistenzprotein zur Erzeugung einer Resistenz gegenüber Pathogenen bei Pflanzen kodiert. Diese Nukleinsäure weist dann nur einen begrenzten Teil eines NBS-LRR-Resistenzgens wie das RZ3-Gen aufweist, der sich vom 5'-Ende des kodierenden Bereichs des NBS-LRR-Resistenzgens stromabwärts bis zum Beginn der NBS-Domäne des NBS-LRR-Resistenzgens erstreckt, wobei das NBS-LRR-Resistenzgen kein TIR-NBS-LRR-Resistenzgen ist.

Ferner schließt die Erfindung auch die Verwendung des resistenten RZ3-Genallels, identifiziert mit einem oben beschriebenen Verfahren, zur Kombination mit einer vorstehenden Modifikationen oder mit einem vorstehend beschriebenen genetischen Element, welches eine oder mehrere agronomisch vorteilhafte Eigenschaften in einer Pflanze vermitteln kann.

Ausgestaltungen und Ausführungsformen der vorliegenden Erfindung werden in exemplarischer Weise mit Bezug auf die angehängten Figuren und Sequenzen beschrieben:
Sequenzen:
   SEQ ID NO: 1 genomische DNA-Sequenz des Resistenzgens RZ-3. Die Sequenz umfasst von Nukleotid 1 bis 1403 die regulatorische Region des Promotors
   SEQ ID NO: 2 vorhergesagte Proteinsequenz des Resistenzproteins RZ-3_1
   SEQ ID NO: 3 vorhergesagte Proteinsequenz des Resistenzproteins RZ-3_2
   SEQ ID NO: 4 stromaufwärts zu RZ-3 (SEQ ID NO: 1) angrenzende chromosomale Region
   SEQ ID NO: 5 stromabwärts zu RZ-3 (SEQ ID NO: 1) angrenzende chromosomale Region
   SEQ ID NO: 6 Consensus-Sequenz der genomischen Sequenz des RZ-3-Gens in ss Genotypen
   SEQ ID NO: 7 Zielsequenz im RZ3-Gen des RNAi-Konstrukts im Vektor pZFN-C48-RNAi.
Figuren:
   Fig. 1 A-I: Nukleotidsequenzvergleich zwischen Consensus-Sequenz der genomischen Sequenz des RZ-3-Gens in ss Genotypen (SEQ ID NO: 6) und dem RZ-3-Gen der RR-Genotypen (SEQ ID NO: 1). Diagnostische Polymorphismen sind grau hinterlegt und fett gedruckt hervorgehoben. Unterstrichen sind solche Polymorphismen, welche nicht diagnostisch sind. Die potentiellen Transkriptionsstartpunkte in dem gen sind mit Pfeilen gekennzeichnet. Sie führen zu zwei Polypeptid-Varianten RZ-3_1 und RZ-3_2. Die Stelle des Retrotransposons ist mit einem schwarzen Dreieck auf der Spitze gekennzeichnet.
   Fig. 2 A-L: Aminosäuresequenzvergleich des vorhergesagten Polypeptids aus den RR-Genotypen (RZ-3_1; SEQ ID NO: 2) und Polypeptiden aus 22 unterschiedlichen ss-Genotypen. Diagnostische Polymorphismen sind grau hinterlegt und fett gedruckt hervorgehoben. Unterstrichen sind solche Polymorphismen, welche nicht diagnostisch sind.

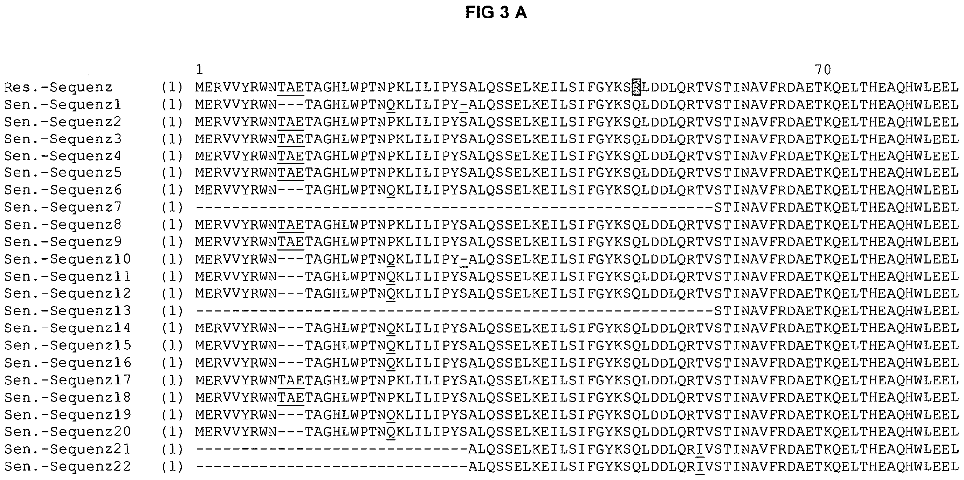

   Fig. 3 A-L: Aminosäuresequenzvergleich des vorhergesagten Polypeptids aus den RR-Genotypen (RZ-3_2; SEQ ID NO: 3) und Polypeptiden aus 22 unterschiedlichen ss-Genotypen. Diagnostische Polymorphismen sind grau hinterlegt und fett gedruckt hervorgehoben. Unterstrichen sind solche Polymorphismen, welche nicht diagnostisch sind.
   Fig. 4: Physikalische Karte der RZ-3-Zielregion. In der dargestellten Zielregion des sensitiven Referenzgenotyps wurden fünf Gene annotiert ("2" (DUF565), "3" (hypothetisches Protein), "4" (NBS-LRR Kandidatengen), "5" (Retrotransposon) und "6" (Ankyrin-Repeat)). Das NBS-LRR Kandidatengen ("4") beinhaltet in der sensitiven Referenz-Sequenz eine Retrotransposon-Insertion ("5"). Dieses Retrotransposon fehlt vollständig in der resistenten Sequenz, so dass in dem resistenten Genotyp nur noch vier Gene annotiert werden können ("2", "3", "4" und "6"). Die Positionen der engsten Rekombinationen (Rekombinanten: 111T_3515/ ZR11007_03075 mit Ziffer "7" und 111PB3645/ZR08093_05621 mit Ziffer "8") sind oberhalb dargestellt. Mit deren Hilfe konnte die kürzere Zielregion "1" eingrenzt werden. Die hierfür entwickelten Marker aus der Rekombinanten-Analyse sind als schwarze Striche im unteren Teil der Figur wiedergegeben. Zur Validierung des Gens im RNAi-Ansatz wurde für das *Gene silencing* des resistenten RZ-3-Genallels ein Gensegment ("9") teilweise aus dem Domainbereich "10" als Zielsequenz ausgewählt.
   Fig. 5: Markeranalyse der engsten Rekombinanten in der RZ-3 Zielregion (kleine Buchstaben im fett-gerahmten Bereich sind *in silico* generierte Markerdaten). Die acht rekombinanten Linien wurden mit insgesamt 1051 Nachkommen phänotypisiert und genotypisiert. Die Nachkommen wurden anhand der Markerdaten im NBS-LRR Kandidatengen bzw. der spaltenden flankierenden Region im Falle, dass das NBS-LRR Kandidatengen homozygot RR bzw. ss ist, in 3 Gruppen eingeteilt (RR-resistent homozygot, Rs - hetrozygot, ss-sensitiv homozygot). Daneben sind die entsprechenden ELISA-Werte wiedergegeben. Eine Spaltung oder Nicht-Spaltung der Nachkommen wurde mit T-test und Wilcoxon Statistik nach Signifikanz überprüft. Anhand der Ergebnisse konnte das Kandidatengen ganz eindeutig zwischen den Markern s3e5800s01 und s3e5873s01 eingegrenzt werden.

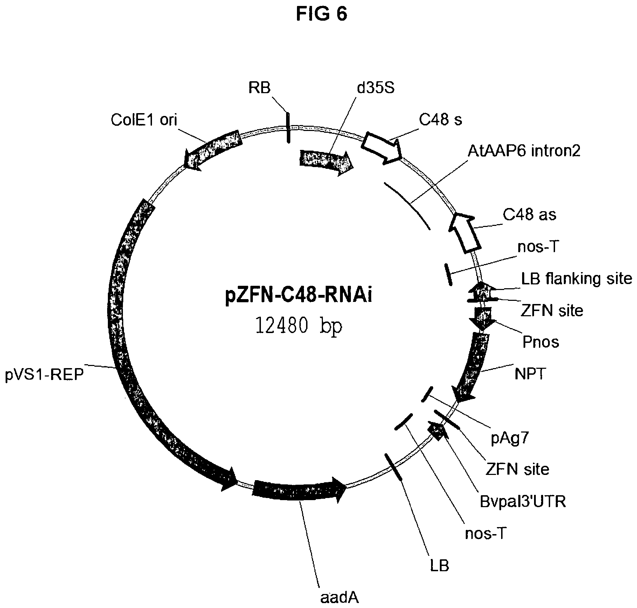

   Fig. 6: Transformationsvektor pZFN-C48-RNAi: d35S-promoter; C48 s: C48 sequence sense orientation; AtAAP6 intron2: Arabidopsis thaliana amino acid permease 6 intron; C48 as: C48 sequence antisense orientation; Nos-T: nos Terminator; LB flanking site: Left border flanking site; ZFN site: Zinc-finger nuclease recognition site (complementary); Pnos: Nos promoter; NPT: coding sequence; neomycin phosphotransferase (npt) gene; pAG7: pAG7 terminator; Bvpal3'UTR: 3' untranslated region of the Beta vulgaris Pal gene; LB: Left border; aadA: coding sequence; aminoglycoside-3"-adenylyltransferases (AAD); pVS1-REP: pVS1 replication origin; ColE1 ori: ColE1 replication origin; RB: right border.

### Beispiele

Kartierung und Feinkartierung des RZ-3-Gens / Genetische-Physikalische Karte Die RZ-3-Resistenz (auch als C48-Resistenz oder C48 bezeichnet) wurde in mehreren Schritten mittels Kartierung und Feinkartierung auf dem Chromosom 3 zwischen 57,1 und 57,8 cM (interne Referenzkarte) kartiert, also auf einer genetische Distanz zwischen zwei flankierenden Markern von 0,0714 cM in der genetischen Karte. Für die Kartierung wurden insgesamt 8004 Pflanzen aus der Kreuzung S504 (sensitiver Genotyp) x T74 (resistenter Genotyp) untersucht. Parallel zu der C48 QTL Kartierung wurden nach jedem Kartierungsschritt zielorientiert neue informative Marker entwickelt und für die Einschränkung der C48 Zielregion eingesetzt.

Die Feinkartierungskoordinaten wurden zusätzlich mit der Analyse der Nachkommen der informativen Rekombinanten bestätigt. Informative rekombinante BC2S1 oder BC2S2 Pflanzen wurden dazu intensiv mit jeweils 90-180 Nachkommen analysiert. Diese Nachkommen wurden einzelpflanzenweise genotypisiert und parallel phänotypisiert. Mittels statistischer Verfahren (t-Test, Poweranalyse) wurden die Phänotypen der informativen Rekombinanten (homozygot resistent-RR / heterozygot-Rs / homozygot anfällig-ss) nachgewiesen und damit Rückschlüsse auf den Genotyp der informativen Rekombinanten gezogen. Sofern sich die Homozygotenklassen der Nachkommen (RR versus ss) in der Resistenz unterscheiden, liegt das Gen im heterozygoten Bereich (Rs) der Elternpflanze; andernfalls im homozygoten Bereich (RR oder ss) der Elternpflanze.

Eine physikalische Karte wurde generiert für einen Rizomania-sensitiven Genotyp, indem Marker und deren genetische Positionen auf die Chromosomsequenzen projiziert worden sind. Mit der Einschränkung der C48 QTL-Region wurden anhand der Referenz-Sequenz und zusätzlichen vergleichenden Sequenzierungen in resistenten Genotypen (Next Generation Sequenzierung und Sanger Sequenzierung) neue informative Marker entwickelt.

Die durch die Feinkartierung identifizierte Region umfasst eine Sequenzlänge von 37996 Basenpaaren (Positionen flankierender SNP-Marker) in der sensitiven Referenz-Sequenz. Die Kollinearität zwischen den genetischen und der Physikalischen Karte in dem Zielbereich ist widerspruchsfrei (Reihenfolge von 12 Markern im Zielbereich).

### Identifizierung und Sequenzierung von resistenten BAC Klonen

Eine BAC Bibliothek ist entwickelt worden für einen ausgewählten RZ-3 (C48) resistenten Genotyp. Diese BAC Bank wurde mit den angewandten Markern in C48 QTL Bereich durchgemustert. Mehrere BAC Klone wurden für die oben identifizierte Zielregion gefunden. Davon wurden drei unterschiedlich lange BAC-Klone für Sequenzierung ausgewählt, welche vollständig die Zielregion erfassten. Die BAC Klone wurden sequenziert und anhand der entstandenen sequence-reads wurde eine "de novo" Assemblierung durchgeführt. Unter den entstandenen resistenten Sequenzcontigs hatte die grösste Sequenz eine Länge von 110909 bp (34537 reads) und umfasste komplett die Zielregion.

### Vergleich der sensitiven und resistenten Sequenzen - Sequenzauswertung

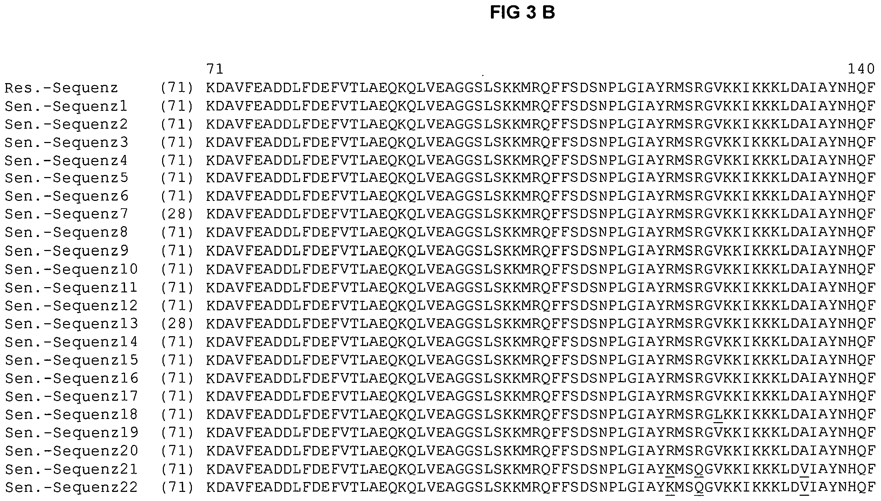

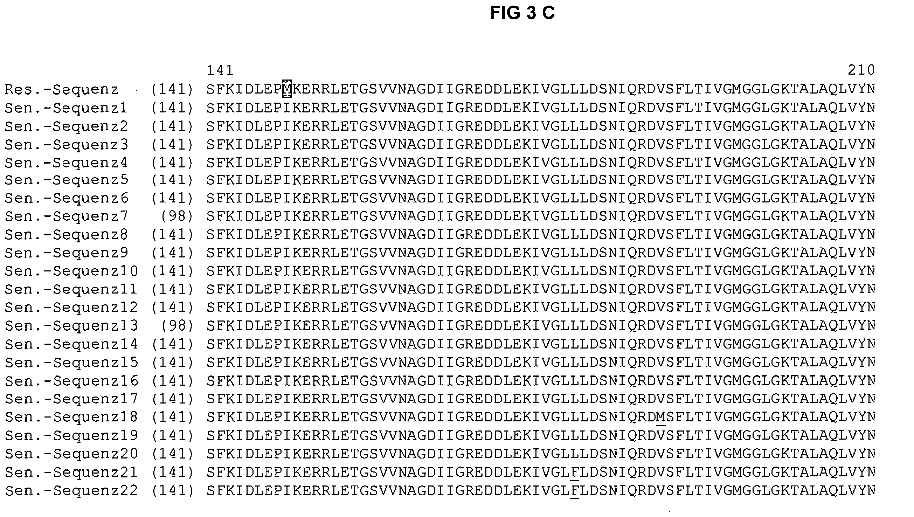

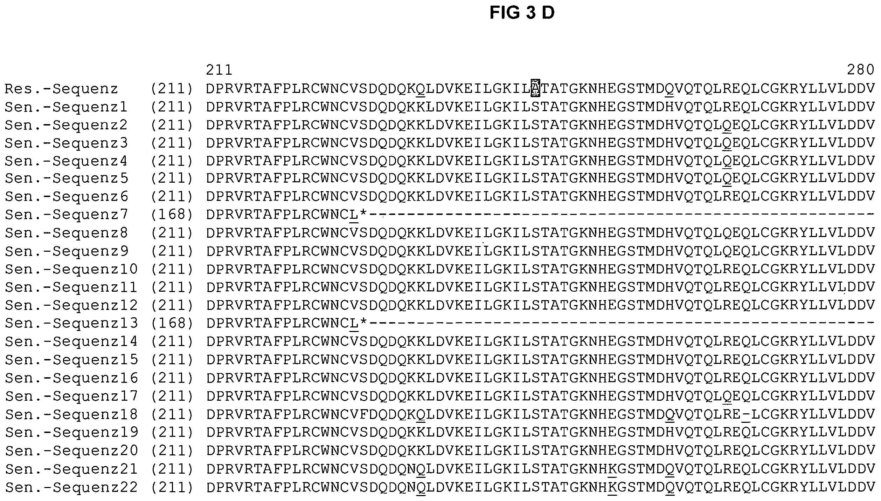

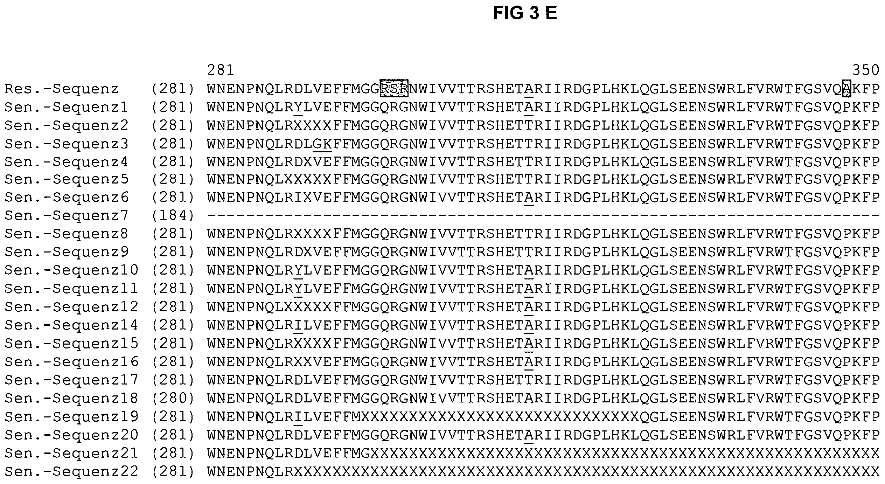

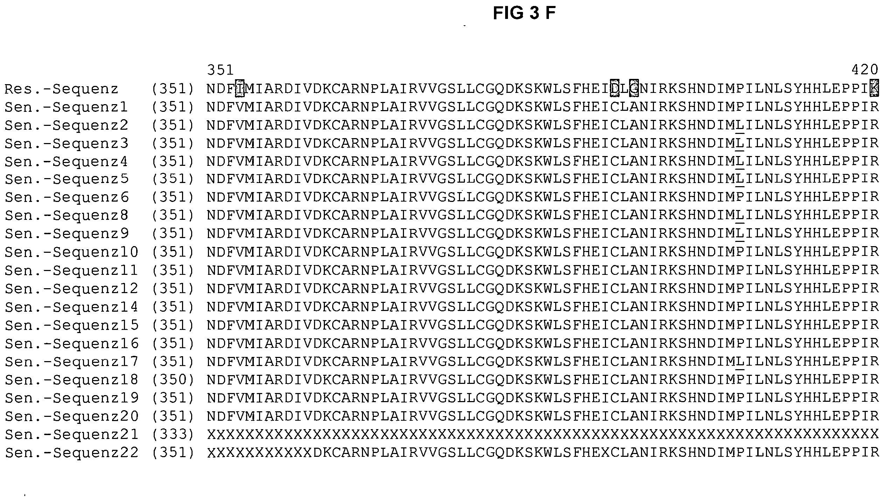

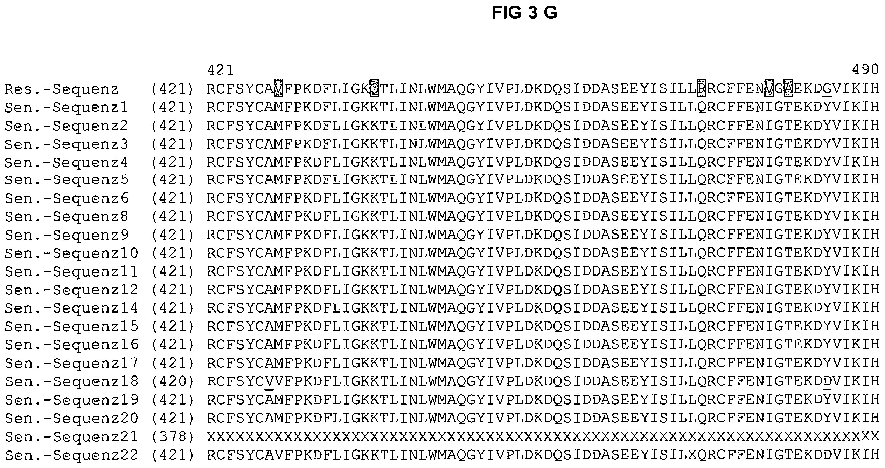

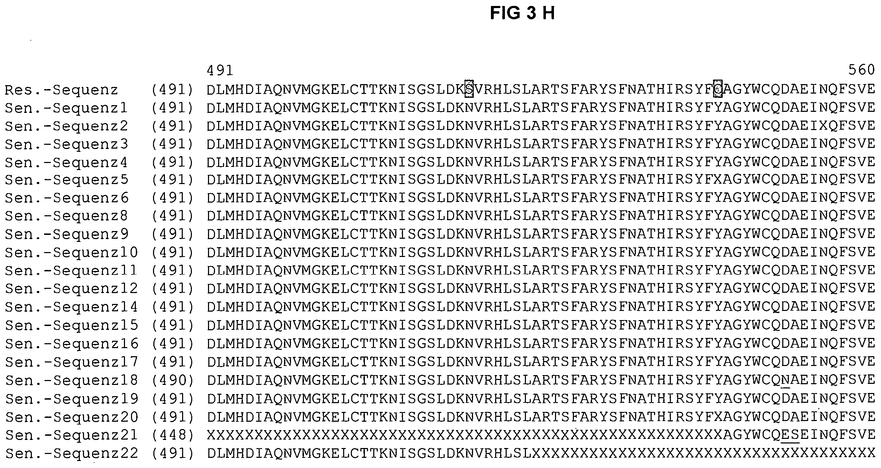

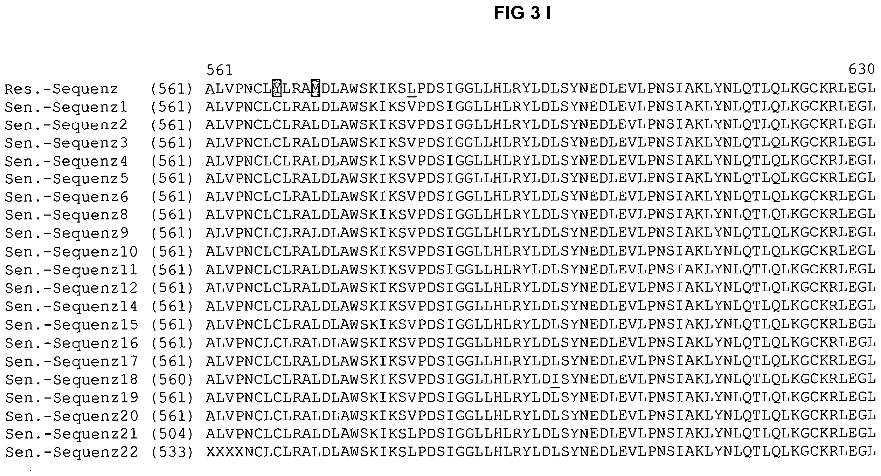

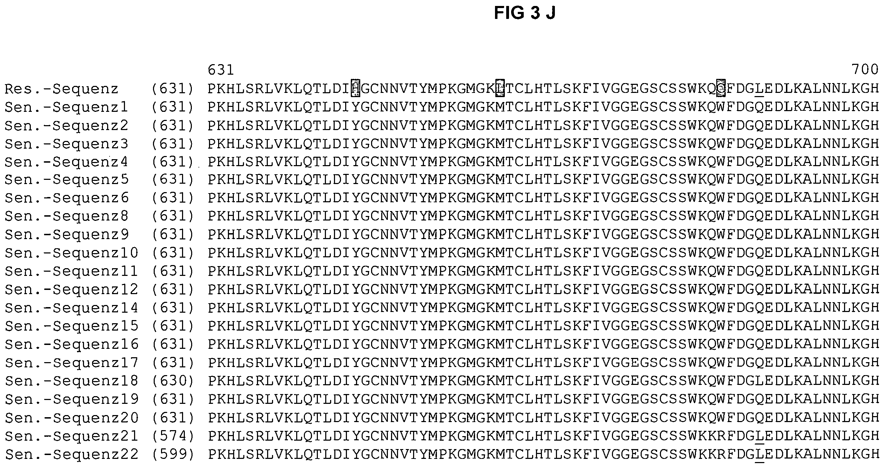

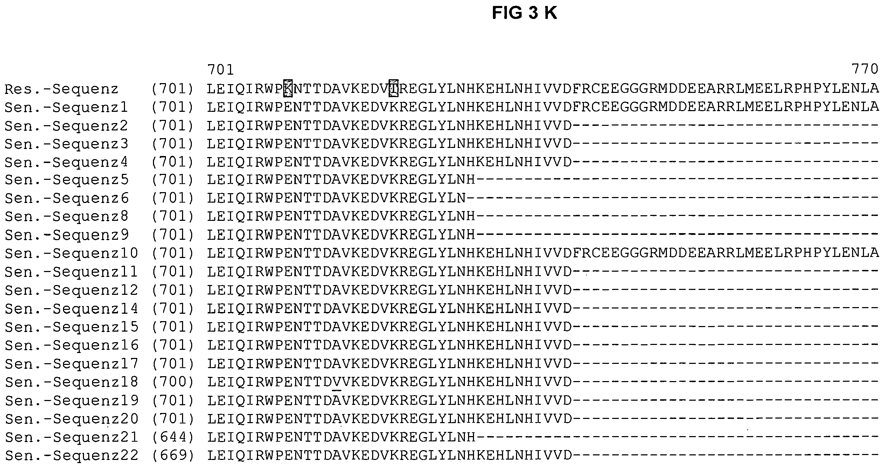

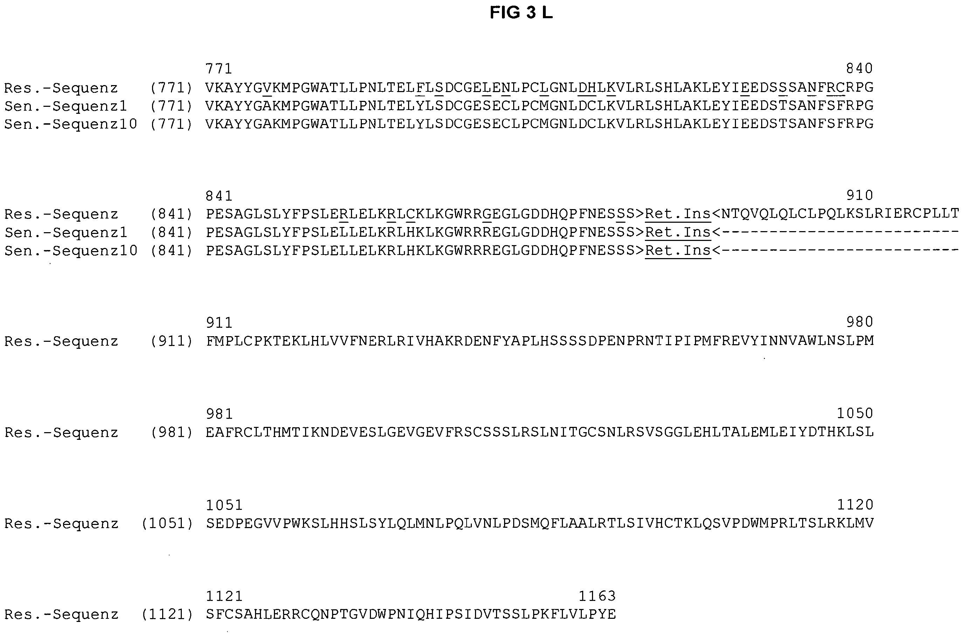

Die Kollinearität der beiden ss- und RR-Sequenzen wurde mit der Anwendung von unterschiedlichen Software-tools verglichen. Für die beiden resistenten und sensitiven Sequenzen wurde eine Genannotation mit den Softwares Maker und Pedant durchgeführt. Die Genannotation auf beiden Sequenzen zeigte die gleiche Abfolge von putativen Genen. Überraschenderweise konnte jedoch ein markanter Unterschied in einem dieser Gene festgestellt werden, nämlich in dem Gen der vorliegenden Erfindung (RZ-3). In dem sensitiven Genotyp konnte in diesem identifizierten NBS-LRR-Gen ein Retrotransposon annotiert werden. Die Insertion des Retrotransposon passierte im Gen zwischen den beiden Domänen der LRR-Domäne und der IR-Domäne. Der resistente Genotyp weist diese Insertion nicht auf und ist in SEQ ID NO: 1 wiedergegeben. Weiterhin wurden anschließend die vorhergesagten Polypeptidsequenzen verglichen und ausgewertet (teilweise dargstellt in siehe Fig. 2 und 3).

### Vergleichende Sequenzierung des NB-ARC-LRR-Kandidatengens

Das NB-ARC-LRR-Kandidatengen wurde in zwei Schritten vergleichend sequenziert. Die Retrotransposon-Insertionsstelle wurde in einem Genotypset mit insgesamt 92 resistenten und sensitiven Genotypen verifiziert. Diese Analyse zeigte, dass alle resistenten Genotypen keine Retrotransposoninsertion hatten. Von den sensitiven Genotypen konnte die Insertion in mehr als 90% der Fälle nachgewiesen werden. Damit scheint der Nachweis der Insertion gekoppelt zu sein mit dem anfälligen Genotyp. Wegen der gefundenen Wiedersprüche (etwa 10% der verbleibenden anfälligen Genotypen ohne Insertion) wurde die Sequenzierung jedoch in dem zweiten Schritt für das gesamte Gen vor der Insertionsstelle mit Promotorbereich ausgeweitet (SEQ ID NO: 1). Insgesamt wurden 31 ausgewählten resistente und anfällige Genotypen inklusive der widersprüchlichen Genotypen sequenziert und verglichen. Im Ergebnis waren alle resistenten Genotypen, die auf sieben unterschiedliche Resistenzquellen zurückzuführen sind, 100 % identisch für die verglichenen ca. 4100 Basenpaare. Außerdem wurden vollständig diagnostische Polymorphismen in der Nukleotidsequenz gefunden, von denen mehrere zu Aminosäuresubstitutionen in der Protein-Sequenz führen (siehe Figur 1, 2 und3). Einige diese Substitutionen besonders in den Domänenbereichen könnten den Funktionsverlust des identifizierten Resistenzproteins in den ss-Genotypen verursachen. Ferner wurden ebenfalls im Promotorbereich drei mit der Resistenz vollständig gekoppelte (Linkage diseqilibrium = 1) INDELs gefunden (Fig 1). Diese INDELs sind auch als potenzielle Kandidaten für den Funktionsverlust zu betrachten.

### Verifizierung des Gens mittels engen Rekombinanten

In der analysierten Population mit 8004 Pflanzen wurden 16 Rekombinaten in der Zielregion identifiziert (feinkartierter Bereich mit 37996 Basenpaaren). Von diesen 16 Genotypen enthielten 9 Pflanzen die Rekombination zwischen dem NB-ARC-LRR-Protein und dem benachbarten Ankyrin repeat-Protein auf der rechte Seite. Im Falle von zwei Pflanzen sind die Rekombinationen zwischen dem NB-ARC-LRR- Protein und dem links benachbarten DUF565 Protein (Protein mit unbekannter Funktion). Durch die Analyse der Nachkommenschaft von allen diesen rekombinanten Pflanzen (ein Gen Entfernung links und rechts) konnte ganz eindeutig bewiesen werden, dass das Gen zwischen DUF565 und das Ankyrin repeat Protein liegt, nämlich dass nur das NB-ARC Protein für die Resistenz verantwortlich ist.

### Exemplarischer Nachweis der Abwesenheit der Transposoninsertion

Für den Nachweis der Retrotransposon-Insertion wurden 3 spezielle dominante Primerkombinationen entwickelt. Die erste und die zweite Primerkombinationen sind in der Lage die Insertion zu detektieren, da jeweils ein Primer von den beiden Primerpaaren in dem Retrotransposon sitzen (linke oder rechte Flanke des Retrotransposons) und der zweite Primer bindet direkt vor oder nach dem Retrotransposon. Ein drittes Primerpaar detektiert das Fehlen des Retrotransposons, dadurch, dass die Primer vor und nach dem Retrotransposon eine Bindungsstelle finden. Ein PCR-Produkt unter Standardbedingungen kann nur dann entstehen, wenn das Retrotransposon fehlt, ansonsten mit dem Retrotransposon wäre das PCR Produkt zu groß und so würde auf diese Weise kein Amplikon entstehen.

### Verifizierung des Gens mittels RNAi-Ansatz

Neben der oben beschriebenen_Verifizierung des Gens mittels engen Rekombinanten erfolgte auch ein weiterer Nachweis der Resistenzwirkung des Gens mittels RNA-Interferenz. Hierfür wurde ein resistenter Standard-Zuckerrübengenotyp mit einem DNA-Konstrukt transformiert, welches eine doppelsträngige hairpin-RNA kodiert. Diese dsRNA war in der Lage posttransskriptional ein Gene Silencing zu bewirken, welches das resistente RZ-3-Genallel in seiner Wirkung vermindern oder ausschalten würde, wodurch der zuvor resistente Zuckerrübengenotyp sensitiv gegenüber Rizomania werden sollte.

Zur Bereitstellung eines geeigneten DNA-Konstrukts wurde ein definierter Zielsequenzbereich des resistenten RZ3-Genallels von 434 Basenpaaren Länge (SEQ ID NO: 7; Figur 4) ausgewählt, durch PCR amplifiziert und sowohl in sense- als auch in antisense-Richtung in den Vektor pZFN kloniert, der zur Synthese von hairpin-Strukturen geeignet ist (Fig. 6). Dieser Vektor weist einen doppelten CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen nos-Terminator auf. Die Transformation der Zuckerrüben mit dem bereitgestellten Vektor erfolgte nach dem Protokoll von Lindsey & Gallois (1990) unter Verwendung des Antibiotikums Kanamycin als Selektionsmarker. Nach mehreren Selektionsschritten wurde die erfolgreiche Transformation an transgenen Sprosse über PCR mittels Nachweis der Anwesenheit des nptll-Gen, des AAP6-Intron und der beiden t-DNA-Bordersequenzen (LB/RB) und der Abwesenheit von *vir* überprüft. Positive Sprosse wurden *in-vitro* auf jeweils 30 Sprosse klonal vermehrt, bewurzelt und im Gewächshaus in Erde überführt. Etwa 2 Wochen später wurden die transgenen Zuckerrübenpflanzen in Rizomaniakontaminierte Erde pikiert, in welcher sie für 8 bis 10 Wochen kultiviert wurden. Als Kontrolle wurden unter denselben Bedingungen nicht transformierte Pflanzen desselben resistenten genetischen Standard-Transformationshintergrunds herangezogen. Zum Nachweis der Ausprägung der Rizomania wurden die Wurzeln der Zuckerrübenpflanzen geerntet und mittels ELISA-Test der BNYVV-Befall quantifiziert, wobei ein niedriger ELISA-Wert eine Resistenz und ein hoher Wert eine Sensitivität anzeigt (Mechelke 1997, Clark & Adams 1977). Der ELISA-Wert der transformierten Zuckerrüben war mit einem Mittelwert von 3,55 signifikant höher als der ELISA-Wert der weiterhin resistenten Kontrolle mit einem Mittelwert von 1,27 und vergleichbar mit dem sensitiven Standard D108_ss (Tabelle 1). Danach zeigten die Ergebnisse des ELISA-Tests, dass durch das spezifische Gene-Silencing des resistenten RZ-3-Allels im Transformationshintergrund eine zuvor resistente Pflanze sensitiv gegenüber BNYW wurde. Folglich konnte das Gen der vorliegenden Erfindung eindeutig als das Resistenzgen RZ3 verifiziert werden.

**Tabelle 1: Ergebnisse des ELISA-Tests nach statistischer Analysen (D108_ss = sensitiver Standard; 6921_RR = resistenter Transformationshintergrund; 6921_RNAi = resistenter Transformationshintergrund mit dsRNA gerichtet gegen RZ3-Gen).**

| | D108_ss | 6921_RR | 6921_RNAi |
|---|---|---|---|
| n | 6 | 25 | 64 |
| Mittelwert | 3,98 | 1,27 | 3,55 |
| Standardfehler | 0,02 | 0,25 | 0,11 |
| Standardabweichung | 0,06 | 1,24 | 0,87 |
| *T-Test (Signifikanzlevel): p <0,0001* | | | |

### Referenzen

Clark, M.F.; Adams, A.N. (1977): Characteristics of the microplate method of enzyme-linked immunosorbent assay for the detection of plant viruses. J. Gen. Virol. 34, 475-483
Depicker A, Stachel S, Dhaese P, Zambryski P, Goodman HM (1982) Nopaline synthase: transcript mapping and DNA sequence. J Mol Appl Genet. 1(6): 561-73.
Esser K (2000) Kryptogamen 1: Cyanobakterien Algen Pilze Flechten Praktikum und Lehrbuch. Springer-Verlag, Berlin, Heidelberg, 3. Aufl. 2000.
Gidner S, Lennefors BL, Nilsson NO, Bensefelt J, Johansson E, Gyllenspetz U, Kraft T (2005) QTL mapping of BNYVV resistance from the WB41 source in sugar beet. Genome 48: 279-285.
Larson RL, Wintermantel WM, Hill A, Fortis L, Nunez A (2008) Proteome changes in sugar beet in response to Beet necrotic yellow vein virus. Physiological and Mol. Pl. Pathol. 72: 62-72.
Lindsey, K., and P. Gallois (1990) "Transformation of sugarbeet (Beta vulgaris) by Agrobacterium tumefaciens." Journal of experimental botany 41.5: 529-536.
Martin GB, Bogdanove AJ; Sessa G (2003) Understanding the functions of plant disease resistance proteins. Annual Review of Plant Biology 54: 23-61.
Mechelke W (1997) Probleme in der Rizomaniaresistenzzüchtung, Vorträge für Pflanzenzüchtung, Resistenzzüchtung bei Zuckerrüben, Gesellschaft für Pflanzenzüchtung e.V., 113-123.
Odell JT, Nagy F, Chua N-H (1985) Identification of DNA sequences required for activity of the cauliflower mosaic virus 35S promoter. Nature 313, 810-812
Rushton PJ, Torres JT, Parniske M, Wernert P, Hahlbrock K, and Somssich IE (1996) Interaction of elicitor-induced DNA-binding proteins with elicitor response elements in the promoters of parsley PR1 genes. EMBO J. 15(20): 5690-5700.
Sambrook J, Russell DW (2001) Molecular Cloning. A laboratory manual, Cold Spring Harbor Laboratory Press, 3. Aufl. 2001.
Schmidlin LEDEB, Weyens G, Lefebvre M, Gilmer D (2008) Identification of differentially expressed root genes upon rhizomania disease. Mol. Plant Pathol. 9(6):741-51.
Scholten OE, Bock TSMD, Klein-Lankhorst RM, Lange W (1999) Inheritance of resistance to Beet necrotic yellow vein virus in Beta vulgaris conferred by a second gene for resistance. Theor. Appl. Genet. 99:740-746.
Sohi HH, Maleki M(2004) Evidence for presence of types A and B of beet necrotic yellow vein virus (BNYVV) in Iran. Virus Genes 29(3): 353-8.
Van Ooijen G, Mayr G, Kasiem MMA, Albrecht M, Cornelissen BJC, Takken FLW (2008) Structure-function analysis of the NB-ARC domain of plant disease resistance proteins. Journal of Experimental Botany, 59(6): 1383-1397
WO/2000/29592 (Max-Planck-Gesellschaft zur Förderung der Wissenschaften e.V.). Chimeric promoters capable of mediating gene expression in plants upon pathogen infection and uses thereof.
WO/2006/128444 (KWS SAAT AG). AUTOACTIVATED RESISTANCE PROTEIN.
WO/2007/147395 (KWS SAAT AG). Pathogen induzierbarer synthetischer Promotor.
WO/2013/127379 (KWS SAAT AG). PATHOGEN-RESISTANT TRANSGENIC PLANT.
WO/2013/050024 (KWS SAAT AG). TRANSGENIC PLANT OF THE SPECIES BETA VULGARIS HAVING EN-HANCED RESISTANCE TO CERCOSPORA.
WO/2013/091612 (KWS SAAT AG). NOVEL PLANT-DERIVED CIS-REGULATORY ELEMENTS FOR THE DEVE-LOPMENT OF PATHOGEN-RESPONSIVE CHIMERIC PROMOTORS.

## Patentansprüche

1. Nukleinsäuremolekül, welches ein Polypeptid kodiert, das in der Lage ist, eine Resistenz gegenüber einem Pathogen in einer Pflanze, in welcher das Polypeptid exprimiert wird, zu vermitteln, **dadurch gekennzeichnet, dass** das Nukleinsäuremolekül eine Nukleotidsequenz umfasst, welche ausgewählt ist aus
a) einer Nukleotidsequenz, die ein Polypeptid mit einer Aminosäuresequenz gemäß SEQ ID NO: 2 oder SEQ ID NO: 3 kodiert,
b) einer Nukleotidsequenz, die die kodierende Sequenz der DNA-Sequenz gemäß SEQ ID NO: 1 umfasst,
c) einer Nukleotidsequenz, die ein Polypeptid kodiert, welches sich durch Substitution, Deletion und/oder Addition von einer Aminosäure der Aminosäuresequenz, die durch die Nukleotidsequenz nach a) oder b) kodiert wird, von einem Polypeptid, das durch die Nukleotidsequenz nach a) oder b) kodiert wird, ableitet,
d) einer Nukleotidsequenz, die ein Polypeptid kodiert, welches eine Aminosäuresequenz aufweist, die zu mindestens 80% identisch ist zu einer Aminosäuresequenz, welche durch die Nukleotidsequenz nach a) oder b) kodiert wird, oder
e) einer Nukleotidsequenz, welche mindestens die Aminosäurepositionen 168-227 der SEQ ID NO: 2 und mindestens die Aminosäurepositionen 591-613 der SEQ ID NO: 2 und mindestens die Aminosäurepositionen 1013-1072 der SEQ ID NO: 2 oder welche mindestens die Aminosäurepositionen 182-241 der SEQ ID NO: 3, mindestens die Aminosäurepositionen 605-627 der SEQ ID NO: 3 und mindestens die Aminosäurepositionen 1027-1086 der SEQ ID NO: 3 kodiert.

2. Vektor, welcher das Nukleinsäuremolekül nach Anspruch 1 umfasst.

3. Prokaryotische Zelle oder Hefezelle, welche das Nukleinsäuremolekül nach Anspruch 1 oder den Vektor nach Anspruch 2 umfasst.

4. Polypeptid, welches in der Lage ist eine Resistenz gegenüber einem Pathogen in einer Pflanze, in welcher das Polypeptid exprimiert wird, zu vermitteln und welches durch das Nukleinsäuremolekül nach Anspruch 1 kodiert wird.

5. Transgene Pflanzenzelle, welche das Nukleinsäuremolekül nach Anspruch 1 als Transgen oder den Vektor nach Anspruch 2 umfasst.

6. Transgene Pflanze oder ein Teil davon, umfassend eine Pflanzenzelle nach Anspruch 5.

7. Samen von der Pflanze nach Anspruch 6, wobei der Samen das Nukleinsäuremolekül nach Anspruch 1 als Transgen umfasst.

8. Verfahren zur Herstellung einer transgenen Pflanzenzelle, **dadurch gekennzeichnet, dass** das Verfahren einen Schritt des Einbringens des Nukleinsäuremoleküls nach Anspruch 1 oder des Vektors nach Anspruch 2 in die Pflanzenzelle umfasst.

9. Verfahren zur Herstellung einer transgenen Pflanze, **dadurch gekennzeichnet, dass** das Verfahren die nachfolgenden Schritte umfasst
a) Einbringen des Nukleinsäuremoleküls nach Anspruch 1 oder des Vektors nach Anspruch 2 in eine pflanzliche Zelle und
b) Regenerierung der transgenen Pflanze aus der transgenen Pflanzenzelle aus Schritt a).

10. Verfahren zur Identifikation eines Nukleinsäuremoleküls, welches ein Protein kodiert, das in der Lage ist, eine Resistenz gegenüber dem Pathogen BNYVV in einer Pflanze der Gattung Beta, in welcher das Protein exprimiert wird, zu vermitteln, **dadurch gekennzeichnet, dass** das Verfahren den nachfolgenden Schritt umfasst
i. Nachweisen der Abwesenheit einer Insertion in der kodierenden Nukleotidsequenz des Nukleinsäuremoleküls nach Anspruch 1 oder
ii. Nachweisen von mindestens einem Polymorphismus gemäß Figur 1, 2 und/oder 3 in der kodierenden Nukleotidsequenz des Nukleinsäuremoleküls nach Anspruch 1 unter Verwendung von molekularen Markern, welche den Polymorphismus erkennen.

11. Molekularer Marker zur Selektion BNYVV-resistenter Pflanzen, welcher einen Polymorphismus gemäß Figur 1, 2 oder 3 detektiert.

12. Verwendung eines molekularen Markers nach Anspruch 11 zur Selektion BNYW resistenter Pflanzen umfassend
i) die Detektion eines Polymorphismus gemäß Figur 1, 2 oder 3
oder
ii) umfassend den Nachweis der An- oder Abwesenheit einer Insertion in der kodierenden Nukleotidsequenz eines Nukleinsäuremoleküls gemäß Anspruch 1.

13. Verwendung eines mit dem Nukleinsäuremolekül nach Anspruch 1 eng-gekoppelten molekularen Markers auf einer DNA-Sequenz gemäß SEQ ID NO: 4 oder auf einer DNA-Sequenz gemäß SEQ ID NO: 5 in einem Verfahren zur Selektion einer Pflanze, welche eine Resistenz gegenüber BNYVV aufweist.

## Claims

1. A nucleic acid molecule, which encodes a polypeptide capable of imparting resistance to a pathogen in a plant in which the polypeptide is expressed, **characterized in that** the nucleic acid molecule comprises a nucleotide sequence selected from
a) a nucleotide sequence that encodes a polypeptide with an amino acid sequence according to SEQ ID NO. 2 or SEQ ID NO. 3,
b) a nucleotide sequence comprising the coding sequence of the DNA sequence according to SEQ ID NO. 1,
c) a nucleotide sequence that encodes a polypeptide derived by substitution, deletion and/or addition of an amino acid of the amino acid sequence encoded by the nucleotide sequence according to a) or b), derived from a polypeptide encoded by the nucleotide sequence according to a) or b),
d) a nucleotide sequence that encodes a polypeptide having an amino acid sequence, which is at least 80% identical to an amino acid sequence encoded by the nucleotide sequence according to a) or b), or
e) a nucleotide sequence which encodes at least the amino acid positions 168-227 of SEQ ID NO. 2 and at least the amino acid positions 591-613 of SEQ ID NO. 2 and at least the amino acid positions 1013-1072 of SEQ ID NO. 2 or which encodes at least the amino acid positions 182-241 of SEQ ID NO. 3, at least the amino acid positions 605-627 of SEQ ID NO. 3 and at least the amino acid positions 1027-1086 of SEQ ID NO. 3.

2. A vector comprising the nucleic acid molecule according to claim 1.

3. A prokaryotic cell or yeast cell comprising the nucleic acid molecule according to claim 1 or the vector according to claim 2.

4. A polypeptide, capable of imparting a resistance to a pathogen in a plant in which the polypeptide is expressed and which polypeptide is encoded by the nucleic acid molecule according to claim 1.

5. A transgenic plant cell comprising the nucleic acid molecule according to claim 1 as a transgene, or the vector according to claim 2.

6. A transgenic plant or a part thereof comprising a plant cell according to claim 5.

7. A seed of the plant according to claim 6, wherein the seed comprises the nucleic acid molecule according to claim 1 as a transgene.

8. A method for producing a transgenic plant cell, **characterized in that** the method comprises a step of introducing the nucleic acid molecule according to claim 1 or the vector according to claim 2 into the plant cell.

9. A method for producing a transgenic plant, **characterized in that** the method comprises the following steps:
a) introducing the nucleic acid molecule according to claim 1 or the vector according to claim 2 into a plant cell, and
b) regenerating the transgenic plant from the transgenic plant cell from step a).

10. A method for identification of a nucleic acid molecule, which encodes a protein capable of imparting resistance to the pathogen BNYVV in a plant of the Beta genus, in which the protein is expressed, **characterized in that** the method comprises the following steps:
i. detecting the absence of an insertion in the coding nucleotide sequence of the nucleic acid molecule according to claim 1 or
ii. detecting at least one polymorphism according to Figures 1, 2 and/or 3 in the coding nucleotide sequence of the nucleic acid molecule according to claim 1 using molecular markers, which detect the polymorphism.

11. A molecular marker for selection of BNYVV-resistant plants, suitable for detecting a polymorphism according to Figures 1, 2 or 3.

12. Use of a molecular marker according to claim 11 for selection of BNYVV-resistant plants, comprising
i) detection of a polymorphism according to Figures 1, 2 or 3
or
ii) comprising detection of the presence or absence of an insertion in the coding nucleotide sequence of a nucleic acid molecule according to claim 1.

13. Use of a molecular marker which is closely coupled with the nucleic acid molecule according to claim 1 on a DNA sequence according to SEQ ID NO. 4 or on a DNA sequence according to SEQ ID NO. 5 in a method for selecting a plant having a resistance to BNYVV.

## Revendications

1. Molécule d'acide nucléique codant pour un polypeptide capable d'occasionner, chez une plante dans laquelle ledit polypeptide est exprimé, une résistance à un pathogène, **caractérisée en ce que** ladite molécule d'acide nucléique comprend une séquence de nucléotides choisie parmi
a) une séquence de nucléotides codant pour polypeptide ayant une séquence d'acides aminés selon la SEQ ID N° : 2 ou la SEQ ID N° : 3,
b) une séquence de nucléotides comprenant la séquence codante de la séquence d'ADN selon la SEQ ID N° : 1.
c) une séquence de nucléotides codant pour un polypeptide dérivé d'un polypeptide codé par la séquence de nucléotides selon a) ou b) en effectuant, au sein de la séquence d'acides aminés codée par la séquence d'acides aminés selon a) ou b), une substitution, délétion et/ou addition d'un acide aminé,
d) une séquence de nucléotides codant pour un polypeptide présentant une séquences d'acides aminés qui est à au moins 80 % identique à une séquence d'acides aminés codée par la séquence d'acides aminés selon a) ou b), ou
e) une séquence de nucléotides codant au moins pour les positions d'acide aminé 168-227 de la SEQ ID N° : 2 et au moins pour les positions d'acide aminé 591-613 de la SEQ ID N° : 2 et au moins pour les positions d'acide aminé 1013-1072 de la SEQ ID N° : 2 ou bien au moins pour les positions d'acide aminé 182-241 de la SEQ ID N° : 3, au moins pour les positions d'acide aminé 605-627 de la SEQ ID N° : 3 et au moins pour les positions d'acide aminé 1027-1086 de la SEQ ID N° : 3.

2. Vecteur comprenant la molécule d'acide nucléique selon la revendication 1.

3. Cellule procaryote ou cellule de levure comprenant la molécule d'acide nucléique selon la revendication 1 ou le vecteur selon la revendication 2.

4. Polypeptide qui est capable d'occasionner, chez une plante dans laquelle ledit polypeptide est exprimé, une résistance à un pathogène et qui est codé par la molécule d'acide nucléique selon la revendication 1.

5. Cellule végétale transgénique comprenant la molécule d'acide nucléique selon la revendication 1 en tant que transgène ou le vecteur selon la revendication 2.

6. Plante transgénique ou partie de celle-ci, comprenant une cellule végétale selon la revendication 5.

7. Graine de la plante selon la revendication 6, ladite graine comprenant la molécule d'acide nucléique selon la revendication 1 en tant que transgène.

8. Procédé de fabrication d'une cellule végétale transgénique, **caractérisé en ce que** ledit procédé comprend une étape consistant à introduire dans ladite cellule végétale la molécule d'acide nucléique selon la revendication 1 ou le vecteur selon la revendication 2.

9. Procédé de fabrication d'une plante transgénique, **caractérisé en ce que** ledit procédé comprend les étapes suivantes
a) introduire dans une cellule végétale la molécule d'acide nucléique selon la revendication 1 ou le vecteur selon la revendication 2 et
b) régénérer la plante transgénique à partir de la cellule transgénique issue de l'étape a).

10. Procédé d'identification d'une molécule d'acide nucléique codant pour une protéine capable d'occasionner, chez une plante du genre Beta dans laquelle ladite protéine est exprimée, une résistance au pathogène BNYVV, **caractérisé en ce que** ledit procédé comprend l'étape suivante
i. détecter l'absence d'une insertion dans la séquence de nucléotides codante de la molécule d'acide nucléique selon la revendication 1 ou
ii. détecter au moins un polymorphisme selon les figures 1, 2 et/ou 3 dans la séquence de nucléotides codante de la molécule d'acide nucléique selon la revendication 1 en utilisant des marqueurs moléculaires qui reconnaissent ledit polymorphisme.

11. Marqueur moléculaire qui est destiné à la sélection de plantes résistant au BNYVV et qui détecte un polymorphisme selon les figures 1, 2 ou 3.

12. Utilisation d'un marqueur moléculaire selon la revendication 11 afin de sélectionner des plantes résistant au BNYVV, comprenant
i) la détection d'un polymorphisme selon les figures 1, 2 ou 3
ou
ii) comprenant la détection de la présence ou absence d'une insertion dans la séquence de nucléotides codante d'une molécule d'acide nucléique selon la revendication 1.

13. Utilisation d'un marqueur moléculaire, qui est étroitement couplé à la molécule d'acide nucléique selon la revendication 1 et qui est situé sur une séquence d'ADN selon la SEQ ID N° : 4 ou sur une séquence d'ADN selon la SEQ ID N° : 5, dans un procédé de sélection d'une plante présentant une résistance au BNYVV.


### SEQUENCE LISTING

<110> KWS SAAT AG
<120> Resistenzgen gegen Rizomania
<130> KWS 0209 PCT
<150> DE102013010026.7
   <151> 2013-06-17
<160> 7
<170> Patentin version 3.5
<210> 1
   <211> 5009
   <212> DNA
   <213> Beta vulgaris
<400> 1
   caaatcttct ggcatcaatg gcggtgttgc cgttcatcaa tttaacatca atggaggtaa      60
   gagtcatgtt ttttcaacaa tataaaactt atacttcctc tgttctgttt taaatgaaac     120
   gtttgttttc tcacgcaacc caacccactt ttttaataat aaatattttt agttgtgtgc     180
   acgtaaaaaa tataaaaaag ttataatttg atagtatctt gtttgagatt gtgattatta     240
   agagagtcaa gtctcacaat attcgaaagt ctacgtaatc cacctcaaat tgacgaagaa     300
   aacaagcagg aaaggattaa gtaagttcgt ggaaccacta gaattgattt tcaaatatag     360
   ctctacctaa tatatggcct acttttaatt ttaaataagg agaaggtaat gtgattagaa     420
   acaaattggt cttaaattat tcattaagct taataatgta taaacataat caagtgctat     480
   cttcttttca gggccgtctt gaagattttg gggcccggtt ctattatgaa aattgggccc     540
   ctaaatttat agaaaataaa gatggaaggt tagagttcta aagatagaaa gttgaaaatc     600
   taaatataaa tcattgacaa atttattaag ggtgagaaac aagggtgttt tcttcaaata     660
   tgaagcaaaa ttttcaaaat aatacttcct ccgtttctaa ataagtgcaa catttgcata     720
   atgtttacta ttcacagttt aaactttaat tagctttggt gatttacatt ttaggaaaaa     780
   acatagtcat gtgggatctt gttagattcg tctgaatgtg aattttttta atatcaactt     840
   tttataattt ttacttattg ataattgaag atattaatgg ttaaaataat gcattggcaa     900
   acgtgaaaac aagaagtgtt gcacttattt agaaacggag gaagtatatt ttgggccttt     960
   ataattttgg agaccctgcg ctgttgggct ccttgcacac cttcatctac ccctctgctt    1020
   cttttgatac aatttttcag cgacatgatt gtcgattgat gcatatatta ttgtatactc    1080
   gatccatatt gtttaagatg aattgtttgt ctttgatggt ctccaatgca tattttgtat    1140
   acttaggaat tctaattatg tactattagt agacattgag atgaatacat aattgccata    1200
   atgaagtatg attattttag ttatatactt tctccattcc aaatatataa atgtaacact    1260
   tgtgtacttt atgcgtacta atgcataaca acgtgcactc tcatgtgttt aattatatac    1320
   tttttgagag aagtgttaca ttggggacca tgggactgtg tataatttga ccgcaaaatc    1380
   gaagtgtcgc atttgattga aaatggagag agtagtatat agatggaaca cagcagagac 1440
   tgctggtcat ctttggccaa caaacccaaa attgatatta atcccttatt caggtcattt 1500
   catctttttg acacaaaatg gatgttgtag gcactgcgct atctgctgcc caatctctgt 1560
   ttgcagccct gcaaagttct gagctcaaag agatcctctc gatctttggc tacaaatccc 1620
   gacttgatga cctccaacgc actgtctcta ccatcaacgc tgtattccgt gatgctgaga 1680
   ccaaacagga gctcactcat gaagcacagc attggctcga ggaactcaag gatgctgtct 1740
   ttgaagcaga tgatctgttc gacgagtttg tcactcttgc cgagcagaag caacttgtag 1800
   aggctggtgg cagtctttcc aaaaagatgc gccaattctt ttctgattcc aacccccttg 1860
   gcattgctta taggatgtca cgaggggtta agaagatcaa gaagaagttg gatgctatcg 1920
   cttacaatca tcaatttagc tttaagattg atcttgagcc tatgaaagag agaaggctag 1980
   agactggttc tgtcgtgaac gcaggtgata tcattggaag agaggacgac ttggagaaga 2040
   ttgtaggttt gttgcttgat tctaacatcc aacgtgatgt gtctttcctt actattgtgg 2100
   gaatgggagg gttgggtaaa actgctcttg cccaactcgt gtacaatgat ccaagggtca 2160
   gaactgcttt tccattgaga tgttggaatt gtgtgtctga tcaagatcaa aagcaactag 2220
   atgtgaaaga aattttgggt aagattctgg ctacagctac tggtaagaat catgagggtt 2280
   caaccatgga tcaggtgcaa acccaactac gagaacaact atgtggcaag agatacttgc 2340
   ttgttttgga tgatgtatgg aatgagaatc ctaatcaatt gcgtgatctg gtagaattct 2400
   tcatgggagg tcgaagcaga aattggattg tggtaactac gcgttcgcac gagacagcga 2460
   gaattataag agatggtcca ttgcacaagc tccaaggttt gtctgaggaa aactcttggc 2520
   gtttatttgt caggtggacc ttcggatcag tgcaagcaaa attccctaat gactttatca 2580
   tgattgcacg agatatagtt gacaaatgtg ctcgaaaccc tctggctata agagtggtag 2640
   gaagtctttt gtgtggtcaa gacaagagta agtggctttc atttcatgag atcgatttag 2700
   gcaacattag aaagagccat aatgatatca tgccaatact gaacctaagt taccatcatc 2760
   ttgaacctcc aattaagaga tgctttagtt attgtgcagt gtttccaaag gatttcctta 2820
   tagggaagca gacgctgata aacctctgga tggcacaagg ttatattgtt ccgttagaca 2880
   aagatcaaag catagatgat gctagtgagg aatacatatc aattttgttg cggagatgtt 2940
   ttttcgaaaa tgtcggagca gaaaaagatg gtgttattaa gatccatgat ctcatgcatg 3000
   atattgctca aaatgtcatg gggaaggagc tttgtacgac taaaaacatt agtggcagct 3060
   tggataaaag tgttcgccat ctatctcttg ccagaactag ttttgcaaga tactctttca 3120
   atgcaactca tattcgctcc tatttctgtg ctggctactg gtgtcaggat gctgagataa 3180
   accagttttc agttgaggca ttagtaccaa actgtttgta cctaagggca atggacctcg 3240
   cttggtcgaa gataaaaagt ttaccagact cgattggtgg attgttgcat ttgaggtact 3300
   tagatctttc gtataacgaa gatctggaag tacttccaaa ctcaattgct aaactatata 3360
   atctacaaac cttacaattg aagggttgca agagattgga agggttacca aaacatttga 3420
   gcaggctggt taagcttcaa actttggata tacatggttg caacaatgta acttatatgc    3480
   ccaaaggcat gggtaagttg acttgccttc acactctcag taagtttata gtgggtggag    3540
   aagggagttg ttcaagttgg aagcaatgtt ttgatgggtt ggaagatcta aaggctctca    3600
   ataacctaaa gggtcatctg gaaatccaaa tcaggtggcc caaaaatact acagatgctg    3660
   tcaaagaaga tgttacgagg gaaggattat acctgaatca taaggaacat ctcaatcaca    3720
   ttgtggttga ttttagatgt gaggagggtg gtggaagaat ggatgatgag gaagcaagaa    3780
   gattgatgga agagttgcgg ccacatcctt atcttgaaaa tttggctgtg aaagcatatt    3840
   atggtgtgaa aatgcctggt tgggcaaccc ttctcccaaa tcttacagag ctttttcttt    3900
   ctgattgtgg ggaactggag aaccttccat gcctgggaaa cttggatcat ctaaaagtcc    3960
   tccgactttc gcatttggca aaattggagt acattgaaga agatagctca tcagctaatt    4020
   tcaggtgtag gcctggacca gaaagtgcag gactatcatt atacttcccc tcccttgaac    4080
   gccttgagtt gaagcgtttg tgtaagttaa aaggatggag gagaggggaa gggttaggag    4140
   atgatcacca gccttttaat gaaagcagca gcaatacaca agtccaatta caattatgtc    4200
   ttcctcaatt gaagtcattg agaatagaaa gatgcccatt gctgacattt atgccgctgt    4260
   gtcccaagac agaaaaactg catttagttg tatttaatga acgactccgg atagtgcatg    4320
   ctaagagaga tgagaatttc tatgctccat tacattcatc atcatctgat cctgaaaacc    4380
   cgaggaacac tattcccatt cccatgttta gagaggtata cataaacaat gtggcgtggc    4440
   taaattcgct gcctatggag gcttttaggt gtctcactca tatgacaata aaaaacgacg    4500
   aggtagagag tttgggagaa gttggagagg tgtttcggag ctgctcatct tctttgcgat    4560
   ccttgaatat cacaggttgc tccaacttaa gaagtgtttc tggagggctg gagcatctca    4620
   ctgctttgga gatgttagaa atatacgaca cccataagct gagtctatca gaagacccag    4680
   aaggtgttgt gccatggaaa tcccttcatc actccctcag ctacttgcaa ctgatgaatc    4740
   tcccacagct ggtcaacctg cctgattcga tgcagttctt ggctgccctc cgaactcttt    4800
   caatagtgca ttgcactaaa ctgcaatcag tgccagattg gatgcccaga ctcacttctc    4860
   tcaggaagct tatggtttca ttctgttccg cacatctgga gagaagatgc caaaatccaa    4920
   ctggggtgga ctggcctaac attcaacaca tcccctccat tgatgtcacc tctagccttc    4980
   ctaagttttt agtgttgccg tatgaatag                                      5009
<210> 2
   <211> 1163
   <212> PRT
   <213> Beta vulgaris
<400> 2
   Met Asp Val Val Gly Thr Ala Leu Ser Ala Ala Gln Ser Leu Phe Ala
   1               5                   10                  15
   Ala Leu Gln Ser Ser Glu Leu Lys Glu Ile Leu Ser Ile Phe Gly Tyr
   20 25 30
   Lys Ser Arg Leu Asp Asp Leu Gln Arg Thr Val Ser Thr Ile Asn Ala
   35 40 45
   Val Phe Arg Asp Ala Glu Thr Lys Gln Glu Leu Thr His Glu Ala Gln
   50 55 60
   His Trp Leu Glu Glu Leu Lys Asp Ala Val Phe Glu Ala Asp Asp Leu
   65 70 75 80
   Phe Asp Glu Phe Val Thr Leu Ala Glu Gln Lys Gln Leu Val Glu Ala
   85 90 95
   Gly Gly Ser Leu Ser Lys Lys Met Arg Gln Phe Phe Ser Asp Ser Asn
   100 105 110
   Pro Leu Gly Ile Ala Tyr Arg Met Ser Arg Gly Val Lys Lys Ile Lys
   115 120 125
   Lys Lys Leu Asp Ala Ile Ala Tyr Asn His Gln Phe Ser Phe Lys Ile
   130 135 140
   Asp Leu Glu Pro Met Lys Glu Arg Arg Leu Glu Thr Gly Ser Val Val
   145 150 155 160
   Asn Ala Gly Asp Ile Ile Gly Arg Glu Asp Asp Leu Glu Lys Ile Val
   165 170 175
   Gly Leu Leu Leu Asp Ser Asn Ile Gln Arg Asp Val Ser Phe Leu Thr
   180 185 190
   Ile Val Gly Met Gly Gly Leu Gly Lys Thr Ala Leu Ala Gln Leu Val
   195 200 205
   Tyr Asn Asp Pro Arg Val Arg Thr Ala Phe Pro Leu Arg Cys Trp Asn
   210 215 220
   Cys Val Ser Asp Gln Asp Gln Lys Gln Leu Asp Val Lys Glu Ile Leu
   225 230 235 240
   Gly Lys Ile Leu Ala Thr Ala Thr Gly Lys Asn His Glu Gly Ser Thr
   245 250 255
   Met Asp Gln Val Gln Thr Gln Leu Arg Glu Gln Leu Cys Gly Lys Arg
   260 265 270
   Tyr Leu Leu Val Leu Asp Asp Val Trp Asn Glu Asn Pro Asn Gln Leu
   275 280 285
   Arg Asp Leu Val Glu Phe Phe Met Gly Gly Arg Ser Arg Asn Trp Ile
       290                 295                 300
   Val Val Thr Thr Arg Ser His Glu Thr Ala Arg Ile Ile Arg Asp Gly
   305                 310                 315                 320
   Pro Leu His Lys Leu Gln Gly Leu Ser Glu Glu Asn Ser Trp Arg Leu
                   325                 330                 335
   Phe Val Arg Trp Thr Phe Gly Ser Val Gln Ala Lys Phe Pro Asn Asp
               340                 345                 350
   Phe Ile Met Ile Ala Arg Asp Ile Val Asp Lys Cys Ala Arg Asn Pro
           355                 360                 365
   Leu Ala Ile Arg Val Val Gly Ser Leu Leu Cys Gly Gln Asp Lys Ser
       370                 375                 380
   Lys Trp Leu Ser Phe His Glu Ile Asp Leu Gly Asn Ile Arg Lys Ser
   385                 390                 395                 400
   His Asn Asp Ile Met Pro Ile Leu Asn Leu Ser Tyr His His Leu Glu
                   405                 410                 415
   Pro Pro Ile Lys Arg Cys Phe Ser Tyr Cys Ala Val Phe Pro Lys Asp
               420                 425                 430
   Phe Leu Ile Gly Lys Gln Thr Leu Ile Asn Leu Trp Met Ala Gln Gly
           435                 440                 445
   Tyr Ile Val Pro Leu Asp Lys Asp Gln Ser Ile Asp Asp Ala Ser Glu
       450                 455                 460
   Glu Tyr Ile Ser Ile Leu Leu Arg Arg Cys Phe Phe Glu Asn Val Gly
   465                 470                 475                 480
   Ala Glu Lys Asp Gly Val Ile Lys Ile His Asp Leu Met His Asp Ile
                   485                 490                 495
   Ala Gln Asn Val Met Gly Lys Glu Leu Cys Thr Thr Lys Asn Ile Ser
               500                 505                 510
   Gly Ser Leu Asp Lys Ser Val Arg His Leu Ser Leu Ala Arg Thr Ser
           515                 520                 525
   Phe Ala Arg Tyr Ser Phe Asn Ala Thr His Ile Arg Ser Tyr Phe Cys
       530                 535                 540
   Ala Gly Tyr Trp Cys Gln Asp Ala Glu Ile Asn Gln Phe Ser Val Glu
   545                 550                 555                 560
   Ala Leu Val Pro Asn Cys Leu Tyr Leu Arg Ala Met Asp Leu Ala Trp
   565 570 575
   Ser Lys Ile Lys Ser Leu Pro Asp Ser Ile Gly Gly Leu Leu His Leu
   580 585 590
   Arg Tyr Leu Asp Leu Ser Tyr Asn Glu Asp Leu Glu Val Leu Pro Asn
   595 600 605
   Ser Ile Ala Lys Leu Tyr Asn Leu Gln Thr Leu Gln Leu Lys Gly Cys
   610 615 620
   Lys Arg Leu Glu Gly Leu Pro Lys His Leu Ser Arg Leu Val Lys Leu
   625 630 635 640
   Gln Thr Leu Asp Ile His Gly Cys Asn Asn Val Thr Tyr Met Pro Lys
   645 650 655
   Gly Met Gly Lys Leu Thr Cys Leu His Thr Leu Ser Lys Phe Ile Val
   660 665 670
   Gly Gly Glu Gly Ser Cys Ser Ser Trp Lys Gln Cys Phe Asp Gly Leu
   675 680 685
   Glu Asp Leu Lys Ala Leu Asn Asn Leu Lys Gly His Leu Glu Ile Gln
   690 695 700
   Ile Arg Trp Pro Lys Asn Thr Thr Asp Ala Val Lys Glu Asp Val Thr
   705 710 715 720
   Arg Glu Gly Leu Tyr Leu Asn His Lys Glu His Leu Asn His Ile Val
   725 730 735
   Val Asp Phe Arg Cys Glu Glu Gly Gly Gly Arg Met Asp Asp Glu Glu
   740 745 750
   Ala Arg Arg Leu Met Glu Glu Leu Arg Pro His Pro Tyr Leu Glu Asn
   755 760 765
   Leu Ala Val Lys Ala Tyr Tyr Gly Val Lys Met Pro Gly Trp Ala Thr
   770 775 780
   Leu Leu Pro Asn Leu Thr Glu Leu Phe Leu Ser Asp Cys Gly Glu Leu
   785 790 795 800
   Glu Asn Leu Pro Cys Leu Gly Asn Leu Asp His Leu Lys Val Leu Arg
   805 810 815
   Leu Ser His Leu Ala Lys Leu Glu Tyr Ile Glu Glu Asp Ser Ser Ser
               820                 825                 830
   Ala Asn Phe Arg Cys Arg Pro Gly Pro Glu Ser Ala Gly Leu Ser Leu
           835                 840                 845
   Tyr Phe Pro Ser Leu Glu Arg Leu Glu Leu Lys Arg Leu Cys Lys Leu
       850                 855                 860
   Lys Gly Trp Arg Arg Gly Glu Gly Leu Gly Asp Asp His Gln Pro Phe
   865                 870                 875                 880
   Asn Glu Ser Ser Ser Asn Thr Gln Val Gln Leu Gln Leu Cys Leu Pro
                   885                 890                 895
   Gln Leu Lys Ser Leu Arg Ile Glu Arg Cys Pro Leu Leu Thr Phe Met
               900                 905                 910
   Pro Leu Cys Pro Lys Thr Glu Lys Leu His Leu Val Val Phe Asn Glu
           915                 920                 925
   Arg Leu Arg Ile Val His Ala Lys Arg Asp Glu Asn Phe Tyr Ala Pro
       930                 935                 940
   Leu His Ser Ser Ser Ser Asp Pro Glu Asn Pro Arg Asn Thr Ile Pro
   945                 950                 955                 960
   Ile Pro Met Phe Arg Glu Val Tyr Ile Asn Asn Val Ala Trp Leu Asn
                   965                 970                 975
   Ser Leu Pro Met Glu Ala Phe Arg Cys Leu Thr His Met Thr Ile Lys
               980                 985                 990
   Asn Asp Glu Val Glu Ser Leu Gly  Glu Val Gly Glu Val  Phe Arg Ser
           995                 1000                 1005
   Cys Ser  Ser Ser Leu Arg Ser  Leu Asn Ile Thr Gly  Cys Ser Asn
       1010                 1015                 1020
   Leu Arg  Ser Val Ser Gly Gly  Leu Glu His Leu Thr  Ala Leu Glu
       1025                 1030                 1035
   Met Leu  Glu Ile Tyr Asp Thr  His Lys Leu Ser Leu  Ser Glu Asp
       1040                 1045                 1050
   Pro Glu  Gly Val Val Pro Trp  Lys Ser Leu His His  Ser Leu Ser
       1055                 1060                 1065
   Tyr Leu  Gln Leu Met Asn Leu  Pro Gln Leu Val Asn  Leu Pro Asp
       1070                 1075                 1080
   Ser Met Gln Phe Leu Ala Ala Leu Arg Thr Leu Ser Ile Val His
   1085 1090 1095
   Cys Thr Lys Leu Gln Ser Val Pro Asp Trp Met Pro Arg Leu Thr
   1100 1105 1110
   Ser Leu Arg Lys Leu Met Val Ser Phe Cys Ser Ala His Leu Glu
   1115 1120 1125
   Arg Arg Cys Gln Asn Pro Thr Gly Val Asp Trp Pro Asn Ile Gln
   1130 1135 1140
   His Ile Pro Ser Ile Asp Val Thr Ser Ser Leu Pro Lys Phe Leu
   1145 1150 1155
   Val Leu Pro Tyr Glu
   1160
<210> 3
   <211> 1177
   <212> PRT
   <213> Beta vulgaris
<400> 3
   Met Glu Arg Val Val Tyr Arg Trp Asn Thr Ala Glu Thr Ala Gly His
   1 5 10 15
   Leu Trp Pro Thr Asn Pro Lys Leu Ile Leu Ile Pro Tyr Ser Ala Leu
   20 25 30
   Gln Ser Ser Glu Leu Lys Glu Ile Leu Ser Ile Phe Gly Tyr Lys Ser
   35 40 45
   Arg Leu Asp Asp Leu Gln Arg Thr Val Ser Thr Ile Asn Ala Val Phe
   50 55 60
   Arg Asp Ala Glu Thr Lys Gln Glu Leu Thr His Glu Ala Gln His Trp
   65 70 75 80
   Leu Glu Glu Leu Lys Asp Ala Val Phe Glu Ala Asp Asp Leu Phe Asp
   85 90 95
   Glu Phe Val Thr Leu Ala Glu Gln Lys Gln Leu Val Glu Ala Gly Gly
   100 105 110
   Ser Leu Ser Lys Lys Met Arg Gln Phe Phe Ser Asp Ser Asn Pro Leu
   115 120 125
   Gly Ile Ala Tyr Arg Met Ser Arg Gly Val Lys Lys Ile Lys Lys Lys
   130 135 140
   Leu Asp Ala Ile Ala Tyr Asn His Gln Phe Ser Phe Lys Ile Asp Leu
   145                 150                 155                 160
   Glu Pro Met Lys Glu Arg Arg Leu Glu Thr Gly Ser Val Val Asn Ala
                   165                 170                 175
   Gly Asp Ile Ile Gly Arg Glu Asp Asp Leu Glu Lys Ile Val Gly Leu
               180                 185                 190
   Leu Leu Asp Ser Asn Ile Gln Arg Asp Val Ser Phe Leu Thr Ile Val
           195                 200                 205
   Gly Met Gly Gly Leu Gly Lys Thr Ala Leu Ala Gln Leu Val Tyr Asn
       210                 215                 220
   Asp Pro Arg Val Arg Thr Ala Phe Pro Leu Arg Cys Trp Asn Cys Val
   225                 230                 235                 240
   Ser Asp Gln Asp Gln Lys Gln Leu Asp Val Lys Glu Ile Leu Gly Lys
                   245                 250                 255
   Ile Leu Ala Thr Ala Thr Gly Lys Asn His Glu Gly Ser Thr Met Asp
               260                 265                 270
   Gln Val Gln Thr Gln Leu Arg Glu Gln Leu Cys Gly Lys Arg Tyr Leu
           275                 280                 285
   Leu Val Leu Asp Asp Val Trp Asn Glu Asn Pro Asn Gln Leu Arg Asp
       290                 295                 300
   Leu Val Glu Phe Phe Met Gly Gly Arg Ser Arg Asn Trp Ile Val Val
   305                 310                 315                 320
   Thr Thr Arg Ser His Glu Thr Ala Arg Ile Ile Arg Asp Gly Pro Leu
                   325                 330                 335
   His Lys Leu Gln Gly Leu Ser Glu Glu Asn Ser Trp Arg Leu Phe Val
               340                 345                 350
   Arg Trp Thr Phe Gly Ser Val Gln Ala Lys Phe Pro Asn Asp Phe Ile
           355                 360                 365
   Met Ile Ala Arg Asp Ile Val Asp Lys Cys Ala Arg Asn Pro Leu Ala
       370                 375                 380
   Ile Arg Val Val Gly Ser Leu Leu Cys Gly Gln Asp Lys Ser Lys Trp
   385                 390                 395                 400
   Leu Ser Phe His Glu Ile Asp Leu Gly Asn Ile Arg Lys Ser His Asn
                   405                 410                 415
   Asp Ile Met Pro Ile Leu Asn Leu Ser Tyr His His Leu Glu Pro Pro
   420 425 430
   Ile Lys Arg Cys Phe Ser Tyr Cys Ala Val Phe Pro Lys Asp Phe Leu
   435 440 445
   Ile Gly Lys Gln Thr Leu Ile Asn Leu Trp Met Ala Gln Gly Tyr Ile
   450 455 460
   Val Pro Leu Asp Lys Asp Gln Ser Ile Asp Asp Ala Ser Glu Glu Tyr
   465 470 475 480
   Ile Ser Ile Leu Leu Arg Arg Cys Phe Phe Glu Asn Val Gly Ala Glu
   485 490 495
   Lys Asp Gly Val Ile Lys Ile His Asp Leu Met His Asp Ile Ala Gln
   500 505 510
   Asn Val Met Gly Lys Glu Leu Cys Thr Thr Lys Asn Ile Ser Gly Ser
   515 520 525
   Leu Asp Lys Ser Val Arg His Leu Ser Leu Ala Arg Thr Ser Phe Ala
   530 535 540
   Arg Tyr Ser Phe Asn Ala Thr His Ile Arg Ser Tyr Phe Cys Ala Gly
   545 550 555 560
   Tyr Trp Cys Gln Asp Ala Glu Ile Asn Gln Phe Ser Val Glu Ala Leu
   565 570 575
   Val Pro Asn Cys Leu Tyr Leu Arg Ala Met Asp Leu Ala Trp Ser Lys
   580 585 590
   Ile Lys Ser Leu Pro Asp Ser Ile Gly Gly Leu Leu His Leu Arg Tyr
   595 600 605
   Leu Asp Leu Ser Tyr Asn Glu Asp Leu Glu Val Leu Pro Asn Ser Ile
   610 615 620
   Ala Lys Leu Tyr Asn Leu Gln Thr Leu Gln Leu Lys Gly Cys Lys Arg
   625 630 635 640
   Leu Glu Gly Leu Pro Lys His Leu Ser Arg Leu Val Lys Leu Gln Thr
   645 650 655
   Leu Asp Ile His Gly Cys Asn Asn Val Thr Tyr Met Pro Lys Gly Met
   660 665 670
   Gly Lys Leu Thr Cys Leu His Thr Leu Ser Lys Phe Ile Val Gly Gly
   675 680 685
   Glu Gly Ser Cys Ser Ser Trp Lys Gln Cys Phe Asp Gly Leu Glu Asp
       690                 695                 700
   Leu Lys Ala Leu Asn Asn Leu Lys Gly His Leu Glu Ile Gln Ile Arg
   705                 710                 715                 720
   Trp Pro Lys Asn Thr Thr Asp Ala Val Lys Glu Asp Val Thr Arg Glu
                   725                 730                 735
   Gly Leu Tyr Leu Asn His Lys Glu His Leu Asn His Ile Val Val Asp
               740                 745                 750
   Phe Arg Cys Glu Glu Gly Gly Gly Arg Met Asp Asp Glu Glu Ala Arg
           755                 760                 765
   Arg Leu Met Glu Glu Leu Arg Pro His Pro Tyr Leu Glu Asn Leu Ala
       770                 775                 780
   Val Lys Ala Tyr Tyr Gly Val Lys Met Pro Gly Trp Ala Thr Leu Leu
   785                 790                 795                 800
   Pro Asn Leu Thr Glu Leu Phe Leu Ser Asp Cys Gly Glu Leu Glu Asn
                   805                 810                 815
   Leu Pro Cys Leu Gly Asn Leu Asp His Leu Lys Val Leu Arg Leu Ser
               820                 825                 830
   His Leu Ala Lys Leu Glu Tyr Ile Glu Glu Asp Ser Ser Ser Ala Asn
           835                 840                 845
   Phe Arg Cys Arg Pro Gly Pro Glu Ser Ala Gly Leu Ser Leu Tyr Phe
       850                 855                 860
   Pro Ser Leu Glu Arg Leu Glu Leu Lys Arg Leu Cys Lys Leu Lys Gly
   865                 870                 875                 880
   Trp Arg Arg Gly Glu Gly Leu Gly Asp Asp His Gln Pro Phe Asn Glu
                   885                 890                 895
   Ser Ser Ser Asn Thr Gln Val Gln Leu Gln Leu Cys Leu Pro Gln Leu
               900                 905                 910
   Lys Ser Leu Arg Ile Glu Arg Cys Pro Leu Leu Thr Phe Met Pro Leu
           915                 920                 925
   Cys Pro Lys Thr Glu Lys Leu His Leu Val Val Phe Asn Glu Arg Leu
       930                 935                 940
   Arg Ile Val His Ala Lys Arg Asp Glu Asn Phe Tyr Ala Pro Leu His
   945 950 955 960
   Ser Ser Ser Ser Asp Pro Glu Asn Pro Arg Asn Thr Ile Pro Ile Pro
   965 970 975
   Met Phe Arg Glu Val Tyr Ile Asn Asn Val Ala Trp Leu Asn Ser Leu
   980 985 990
   Pro Met Glu Ala Phe Arg Cys Leu Thr His Met Thr Ile Lys Asn Asp
   995 1000 1005
   Glu Val Glu Ser Leu Gly Glu Val Gly Glu Val Phe Arg Ser Cys
   1010 1015 1020
   Ser Ser Ser Leu Arg Ser Leu Asn Ile Thr Gly Cys Ser Asn Leu
   1025 1030 1035
   Arg Ser Val Ser Gly Gly Leu Glu His Leu Thr Ala Leu Glu Met
   1040 1045 1050
   Leu Glu Ile Tyr Asp Thr His Lys Leu Ser Leu Ser Glu Asp Pro
   1055 1060 1065
   Glu Gly Val Val Pro Trp Lys Ser Leu His His Ser Leu Ser Tyr
   1070 1075 1080
   Leu Gln Leu Met Asn Leu Pro Gln Leu Val Asn Leu Pro Asp Ser
   1085 1090 1095
   Met Gln Phe Leu Ala Ala Leu Arg Thr Leu Ser Ile Val His Cys
   1100 1105 1110
   Thr Lys Leu Gln Ser Val Pro Asp Trp Met Pro Arg Leu Thr Ser
   1115 1120 1125
   Leu Arg Lys Leu Met Val Ser Phe Cys Ser Ala His Leu Glu Arg
   1130 1135 1140
   Arg Cys Gln Asn Pro Thr Gly Val Asp Trp Pro Asn Ile Gln His
   1145 1150 1155
   Ile Pro Ser Ile Asp Val Thr Ser Ser Leu Pro Lys Phe Leu Val
   1160 1165 1170
   Leu Pro Tyr Glu
   1175
<210> 4
   <211> 987
   <212> DNA
   <213> Beta vulgaris
<400> 4
   ttcctcttta gatttccata gatttgaaca aattggggtg attttcatag attattcata 60
   attctctctc cataattctt ctctctcttt ctccatacat ttttcattcg caatactcag 120
   gaactgagta ttgggaattg tccccagttg tcaggttgat gtgcagtaac tttaagagaa 180
   gactttcctc tcatgcaaca tgtccctgat ctgttgcttg atggtcgtca tctcactatt 240
   ctctaataag ctcgatttgt atgaaacaga tgatactata ttccgtttcg tgcaatgtgc 300
   acgaaacgga atatagtatt ttgtgcaagg tgcacgaaac ggattcgatt gtttcgtgca 360
   cattgcacga aacggaatca actgtttcat gtagtctaca cgaaacggaa tcaattgttt 420
   cgtgtagtct acacgaaaca gactaatcat gcattacgaa tcataattac gaaaaaaaat 480
   taacaacttg aatcacaatg acgaaaaaaa attcagaaat tatatcagat tgaaattcga 540
   ttgggtcaaa attatggtcc attaaatatc aaattaaaat ttgtagatct tcaatgaagt 600
   tttttatatc taaccgttag agaggaggag agaatatttt tagagagaga aagggttttt 660
   tagaaagaat gtgataataa gggttttttg ggtttttttt aggctgcgtt agtaaagtga 720
   ggctgcattt agcaaccttt tttttttggt aaatttcatt tcctcgatga acaaggaaac 780
   gaaacggcga gatggcggcg ttggtggaat ttcccggcga aacgcagctt cctttcgatt 840
   catagttgcc ataaatttgc attttaccca gatttcaaat aatttttact aattcgctca 900
   aattgctcat gaaattgttt atttccgcaa attttttgat taacccctcc agaatttgat 960
   tcgcaaatat ggcgaagcta ttgagta 987
<210> 5
   <211> 12364
   <212> DNA
   <213> Beta vulgaris
<400> 5
   ttctgttccg cacatctgga gagaagatgc caaaatccaa ctggggtgga ctggcctaac      60
   attcaacaca tcccctccat tgatgtcacc tctagccttc ctaagttttt agtgttgccg     120
   tatgaatagg tatatacttc tttggttttg gttcgtgctt ccatttagct caaattggaa     180
   atgagcgtat ggcgtcagat ggtgaccaat ctgcagttat tgcgctacgt gtatgttctg     240
   gtttatattg atggcaatgt tcaatagttc attataatcc caatcaaatt tctttgtcca     300
   ttgtttataa tcccaaatcc aatttctttg gaagattgtg ctgaggagag cttgatgaag     360
   gaacttgttt aaggtttttt ctcttataga ttatctttct accattgttt ataatcccaa     420
   ctgcatcttg gtctgagaag gaattcaata ttttttctag ttttacttga ggttaaggct     480
   gtttataatc acaggctttg ccaatagtat aatttttata aagtactact gtacagatta     540
   tgtgaatctt caaaggtttg agagaatcgt cctaaattca tgtaaacttg gagttaagga     600
   gcaggaaatg gagttacttc aagtgttaat gcaatcagct caaaaaatta ctaatacaga     660
   acttattcgt gtcacaactc agaagccttt ataaattata aaagtagtaa agatttcgtt     720
   tcgaaagtat tattcatatt agagtacaat gaataatttg ctttggcaaa gccatctgaa 780
   ggtccttaag aaatgtttag tgcaggtgat ttaacttgct gtgattatgt caccaggaga 840
   acttgcttat caaagaattc agtagcaagt tggtcgatac tcgacagaac atccttatca 900
   aacatgttag tgcagaggtc agagcatgat accctttcat tagtattata ttttacatta 960
   ttaagaaata tatttacatc tgaaattata tatgctgata caataatctt atgcattttg 1020
   agttatacat taaacacggt gaaaaacatt atttcgagca tctgcatttg tgcttgtggt 1080
   tgtgtaatgg taaattggca attgcttgac ctttgtttaa ggctgggtaa gctttcaaaa 1140
   agttgaattg tttagcagta gatcagtaac tatccaaaca aagaccaagc tatacgccaa 1200
   ctgtttcttc acgttcactg tacaagtgtt aaagtatctt caatgggaat accgaaatct 1260
   aaagtcaatt atccaattaa tagtagtcaa aagtcaaaca tctctgtatg aatatgtaga 1320
   ttgaagagtt tctgtatgca ttcaattcaa tgcaacaagt tgtattcgtt cacaccttat 1380
   tacttggtca aaagttgact aattttacac aaggaacttt agcatcaatc atcatcctac 1440
   tatctacgag ttgaaagaaa cttgtacaaa aacttgtttt aaccctgata cagttcagtt 1500
   aagcctgttt gttgatcagt ctgcaatttg aatcactcgt ccactcgcat gacttagtgt 1560
   gcgatcttgt gtctagtttt ctcgagaccc cgcctcctga ccgtgcccag cccaccccaa 1620
   ccattcccag ctctactagc tagtatcagc atacatggga agagccagga ctgtaagcat 1680
   gtcgtttacc gagtcacagc tgctgtcagc agcaggagat ggagatgagg aattcctaat 1740
   acaagctctt gctactcaac ctattgatta cttcctaacg cggagcaaaa acaaaaatgg 1800
   tgaagaacat tgcaatatta tccacattgc agtgttaaat gaacaagcaa agttcctcaa 1860
   tcgagcattg agtatattac ccatctcaac tctgcatctc cttctctgtc agcaagattt 1920
   ttcctacttt agctacaacc ctcttcactg tgcatcttta cgaggtaact ttgctattgt 1980
   caagctcctc gtcgagtttt acgaatcatc atcatcttct tcttcgtcat tggttgatcc 2040
   aagctgtaag ccatggttag ccaaggatgt gaacgggaag acgcctttac aggtggcttt 2100
   ggataggggt agaggtgaat gtgcattaaa aataatggga ttagatgaag aattgctttg 2160
   taatatggtt gataataaag gtaacagccc gctatttcaa gctgtacaaa gaggtagtga 2220
   acaaattgct atgaagatct tggcatcagg gcattcttat agtactggtg gcgagtatga 2280
   gttgactccc cttcatgttc taccaaattg ctcaggtgtg tagtattgat ttgtttttca 2340
   atttgttaaa atttcttacg ctttctgtcc cttaattttt ctcacatgtg ggtttgacac 2400
   agacatttag tgttaagagc aacttcaatg gtcagctatg cactcttcta acttagcttt 2460
   ccacctcaac tacattcaag taacattagt ttcaggctac aacgtccttt tgggtgcatt 2520
   tcttcacttg aacttatatc aaaaccgagc ttattactct tgattggacc taatcaacca 2580
   tgatacgtgt tttcgggaag ttaacagttt cctaatttag ttttcttctg tacaatttca 2640
   agataactaa taaagtatta gcaatcttaa ctataaaaaa agaagaaaac actagcctaa 2700
   tatcatctga tctgcagagg aagtttgcga acttctactt gacaagcatc cagaaatgat 2760
   aaaagcagtc gacaaaaatg gacttacaat cctacacaaa tgggcaatga tgggtaaact 2820
   atggccattt caatttcttt taaagcaaga aaaaagttct aggttgagga aggacttcat 2880
   caacctttta tgtgcaactg agaagtcgac aggcaacaat cctttacaca cagcagctta 2940
   ttaccacaat gaagaaactg cgcaggttgt gcagcttctt gtagaagctt atatagatgc 3000
   taaggaacaa ggagtggagc ttcagccgag cccttggaca tgtgagaata tagaaggaga 3060
   tacacctttg atggtatcct taatcaacaa acatgaaaaa ttggcactgt atttcatgtc 3120
   tgtggatatg gagaattcag ttgtatatgc aaccaagagt gtactatatt gtgctgtact 3180
   gcgtggatgt gatgaagttg cagaagaaac agtggcttca gttgatcctg cctgcttcag 3240
   cttcatgcag cttaaagacg atggtgggcg aaatgtcttg catgttgcat caaattgcac 3300
   aggtgaggtg agagtacctt attgcttgta tatctttctt cttatttgaa aaatcttgga 3360
   ggaactaagc acaagcaagg ccaaggcttc gtcgtgctgt ggtgggctca aagaaggcct 3420
   atgcacagcc tggcccacat gggtgcaggc ggcctcttta agaaattgag gaggccgaaa 3480
   cacggctggc tttttgggct tgtgctcatt ttcaaaattg atgcccatta cagcccacgg 3540
   agcactgctg tgatcttgtg tcgtgagtca tgacaggcca gtaacgggct tgtgctgggc 3600
   aagtaatatg gcataactat ccttatcatt atttataacc ggaacatctc atatatgcaa 3660
   acctttaatt ctgactttga tcagtttatt atagaacatg gaaatcgaac atatctcagc 3720
   agcatattca agctattgta aacttcttta aataattaag gtggtagacg tatatgcctt 3780
   gtattatttc tggtcaaaat gagtatcagg gaaatctaat aaaacaattt aatctttacc 3840
   tatatagcaa gaaaacagtt gggattttga aaacgggaaa atcttgagtc ccatccattt 3900
   atttgcttca ttaccacgga actggggaaa tttcacatac ttgaagtttc ggtgcttcat 3960
   agattctaac attgaagtgt ttatacagag agaacaggca ccttgttggt ggaaaagcta 4020
   gcttggttga tcaacgagcc agatgatgat ggaaagagac cccttgatat agcttcagaa 4080
   gttggtaacg catggcttat aaaattactg ctgacaaaag acccttcctc aaacacaagt 4140
   gcgccatttg cttggattga agcatgtaaa aaaggctact tatcagcaat acatgctttc 4200
   atagaccatt cccctgattt tagaacattt tgtctccaaa gaaaagactc tcctttacat 4260
   cacatacaac tgagaagtta caaagaatac caagaatttc ttgctattcc gttgattcaa 4320
   gagatgaaaa atatgctcga tttcagtggt tcaacgccct tgcatcgagc attagaacgt 4380
   aaggatatcc tccttgctga agcactgctc tctggcgatg gggttcatag aagcatcaaa 4440
   gataaaaatg gtaaaactgc taccgacctg ctagtaaagc tgtgcgacca agagtatgaa 4500
   tgggtatgtg tctccagtct ccacctaatt cttccaatct agaaaactat gattgcatta 4560
   ggaaatactt ctatgtcagt tgtcactatc atcccttgct tgctataagt ctacattgtc 4620
   ttggcaaaaa cataaaatga gcataaatat aaaggttaat tttttctagg gaaattgtac 4680
   tgcgtatacc ctaaactaga atttgccaaa taaccttaga gattcaaaat atatccataa 4740
   ctattaactt tggctaattt gtaatggttc tctcttatac aatttattga ttcaaaccta 4800
   tttctgctga ttaaacttga tcattggtaa tattgaagaa aaattctcat ggccttgcaa 4860
   gacagtgaag tttccatgaa tctcggaaaa atgacagcca caggataaaa taagactgct 4920
   ttagacagca tagtagctct tatgttttac tatatacaag aatatgtaaa agccttgatc 4980
   tatgaaatga attgttctat atatattatt atggtgatat gcaagctcct tctttgaatt 5040
   caatttcaaa caaaatgcag gatactatgt gcaaacgtac acaaattagt ccgtggctaa 5100
   cgacaaacta tatcggaact tcccttgcta ataaggcctt tagatacaca ggcagtacaa 5160
   gacttggtac aacaccatca gcaggagaaa tgcgtagcac actttcagtc gtagcagccc 5220
   ttctagcaac ccttacattt gcagctgggt ttacacttcc tggaggcctt aacgaagata 5280
   ctggcgaagc catcttagca aagaaggttt catttctagt gtttatacta gcagacacat 5340
   acgcgatgtg ttgctccatg ttggtgctgt tctgcctcat atggtctatg gttagcgaca 5400
   gagataagtc acttctactg attgatcgaa gtgttgtgat actcgtccaa tcactttatg 5460
   gaacgttaat agcatttatg gctggagttt acactgctat atcacacaag tctttgtggg 5520
   cagctattat agtcattgtt atgtgctctt tcgttgcgat ttcagctaac agagctattc 5580
   tggataaagt gcttgataag ttgatccctt cggctgatag taagagaaga aattaaaccc 5640
   agacactgga tggatgtctg gatgatgtag gctctcctat aatctttcac tatcttatga 5700
   ttttggatat tactgtctgc aaatgttaaa ctcacacatt gctattatag ttctttgtta 5760
   tgcaagtatg gattcaactc tggactttgg tcagtctggt aactagttgc agcccatgac 5820
   cccaactttt agttattctt atactacctc tgttttgttt ataatacatt ggtacaaatc 5880
   ttattcatgt tactagatga acatgctaat gactagtttt ataatcggct tgttagatgg 5940
   agctctgagt acacatttga aaaatatatc taattaaaaa tattaaaccg actttaaact 6000
   tgtacgatac atgagattat aggaaataaa gaatataagt tttctaatta ttatacggtt 6060
   ttaggaaaat ttagcaatta gtttagttat tatattaatt tgatacttag attttcaatt 6120
   agattatact agagtatata aaatttccta attatatgta ttcggttttc aatcagaaaa 6180
   taatgattta gttttattat aaaattaaat tacttttttt agacggtgct tttctctcca 6240
   aagtttcctc ctttattcac acatgctaaa catggaagaa tatatgtagc attattgttt 6300
   tcacacatca ttttctaaag gttgtatgat tcttattcca aagcaaatta ccttcgataa 6360
   tgttgttact cgatatctaa taaaaaactt ttccatagat gatatcaaga ccttaatgat 6420
   tttaataatt atccaaataa tcgccagagc aactagtact ttttaaacaa taatatattt 6480
   tttttgacaa tggggtaaac aataatattt cttacataaa cattttcata ttcttagggg 6540
   gaaaaaccat taagaaaaat gcatgtatct attggatctc tatacaagtt tattttgata 6600
   gttcgagccc taaattttgt cagcaagtca tatgtaagat ttgtgtataa actataaagt 6660
   gaactattgt ttatttatta gctatgaatt aggtttcaca aaatattata taaagttgaa 6720
   tgatgattaa cggaaactat actgatatta tcatttgaga ttttctcttc atgtaaaaga 6780
   ccatttatct tatcccttat ctctactagt ctactttaag ttcttgtgtt atgtttaatt 6840
   tttgtcatgt atttacctaa atgctagttt tacattcaca actccttttc ttcactagag 6900
   ctatttaaca tttcaaaata cgctataatt ttatattagc aaatataaac gtaatgatcg 6960
   ggattcctta tattttttca caaattatta gaataggcgg tctaattttt acataaatta 7020
   gatgaactta gaagtgaatt tttcaaacaa acccattcca tttcactcta acccaacact 7080
   atcttagtca tcccttatct tttgcttcct ttgttttctt gattctcgaa ctacaacaga 7140
   caattttaag aaataactcg gtatttttat cgaacggatt aaactagtca ctaaatggat 7200
   aaacaagtca ctgaatgggt tagtgaatgt cattcacgaa atagattaaa ttggtcacga 7260
   aatagagtca ctatatttaa aaaggtggca tgttctctgc tgaatattag acttgcaccg 7320
   tgcctaattt taaaagtagg cgatatctta caagacaact gtcatttttc cacttcccta 7380
   ataatgagta atcatgttca tgtatcatac tccttgaaca tgacatatat atttttctag 7440
   aatgaaaaat cacctaacac aaaaagggga accaattaga aagagagaaa gaaaagtaac 7500
   acaaacaaca atcaaaacat gaaaacaact agcaaaattt attaagtact aattaataca 7560
   tctagttacc taaaatgcac tctaattact ttaaaaagtt caaactccaa caatagtgca 7620
   aattagcata aacacttgtt acagcaagtt gtgcaaactc agacacacag accacagaag 7680
   gcggagtccc cacaccagag gggcaactct aatttctcca acgtctcctt tttctttctc 7740
   ttcttctcct ttcatcttct ttgctttctc cctccagaat ctttctctct cttccatttc 7800
   caggtttctc tctcctcttc tctgcttctt attttttgaa agatgcaaac ttttactgaa 7860
   atttatgttt tgaatagtgt tacttattgt tatgctttaa attctgagtt gggtcacttt 7920
   catttttgtt tgaatttaat gggtttttgc tgaattatgc tctttttact ccagtgaacg 7980
   gtttttcagt ttctgggtgt taactgtatt tagttaatta agattggttt gaattcaaaa 8040
   aaaacttagg gtttactatt ttccatgctt aatctttatt ttttaatgtc tgaatatgta 8100
   aaaatgtaaa aattctatgt tgaaaaactg agtaaaatag tatcaaatca aagttttgaa 8160
   gctttgaatt actgatatgt tgtagtttgg tacttggttt gatctgtgag attattcata 8220
   agatgctatc tttatttcct gttttctttt tagtgcaaat attctgaata aaatatgcat 8280
   tagtttactt ttatatagaa tataagtatt tgggattcta agttatggga cactcaattt 8340
   tatatgcaga tccagctgtt ccagactaga ctaacttggt agcttgagct tcacttgttg 8400
   tgcttggatc tgttaagctt ggaagttttg ctgatttagc gccatatgtt ctagatgtat 8460
   tgtattagtc aagtaaagtt ttgatatcga aatttggacc tttagtggca taagagtggt 8520
   ttattcttca tttagaattt tgaccttgag cttagttttg gaattgagtg gttgagaaac 8580
   ttcaaacact ttggcttttc agtttattat acccgggttt ttattgagga aggtagtgag 8640
   aaagctccag gaaaatttga ctcttgtgtc tacagaaaag tcacttagtc ttctcctata 8700
   attttgctgt aatcctggtt ctggacctct aggctctgga atggcagttg gcaaaaacag 8760
   cagtaacgct ggatcattaa ctcggccatg tcattgtttc aaggtggcaa acttgaagga 8820
   aactattttg gatgctagcg agacatccga gttaaaagat cgttatgttt tgggagatca 8880
   actaggttgg gggcagtttg gtgtgatccg ggcatgtgct gataagttta ctggagaact 8940
   actggcgtgc aagtccattg ccaaagatag acttgtaaca caagatgatg ttcgaagtgt 9000
   gaagctcgaa atcgagatta tgagcaagtt gtccggtcat cctcatgttg tcgatctcaa 9060
   agctgtttat gaggaagaag attatgtcca cttggtgatg gagctttgtg ccggtgggga 9120
   gttgttccac cgattagaga aacaaggaag gtattgcgag tctcaagcca aagtcatctt 9180
   caggcatcta atgcaagtag tcttgtattg tcatgataat ggtgttgttc atagagattt 9240
   gaagcctgaa aatgttcttt tggcaaccaa gtcttcttct tcgccaatta aattagccga 9300
   ttttggtctt gctacatata tcaaaccagg tagaacactc ttcttcatct agtttgtgat 9360
   tttagctgtg ttactcggtc tctttcaatt cacctcaata gctgtccatg ttgaattttg 9420
   gataatttga tcaagtcagt ctggccccta aacatgttcc tgcgcccaac actaacagtg 9480
   tcttaagcct ttggttactg gtcaagcagg ggagagtttg catgggacag tggggagtcc 9540
   tttctatata gctcctgaag ttctgtcagg aggttacacc caggctgctg atgtatggag 9600
   tgctggcgta attctctaca ttctcttgag ttctatgcca ccattttggg ggaagacaaa 9660
   gtcaaggata tttgatgcag ttcgagcagc tgatctgcgc ttcccttctg aactttggga 9720
   tcggatatca gaacctgcca aggagctgat caggaaaatg ctttgtgtag atcctttgaa 9780
   gcgcttgaca gctgagcaag ttttaggtat atttttaatt tttgcctcct ttgctgaatt 9840
   cagatgacag ttatatgaca cagtatacat ttgtagaacc caagtgtctg aatagccaat 9900
   caccaatgtc tgacaagttt tttttggctt tcgattacaa aatcatttat tacatatttg 9960
   cattaactgt gttatttttg acacattaca tgaaatcttc attgcttatt tgtgattctt 10020
   tgtgaattgc tgtacattgg aagctgctcc cttttaacat ttgagatgtc cgtaagtggt 10080
   gagtgtaact catctgtccc cacggagaca aagcttttgg atgattttag gacaaatgca 10140
   tttaacgttc tcagcttatc tgacaatgtt atccatggtg tccgtggaca catgtaccaa 10200
   cactaaacat ctagtatttc ttgttggttc tttagttttg ctggatatac ttaagggcta 10260
   aggctttatt tttgttcctg cagctcactc atggatggaa gaggttactg tagctacgga 10320
   agaatcacat gaacatgatt tggcctgctc tgaacattta aaaacccgag atagctcatt 10380
   ttcagcgtca tgtatatcca gggatcacga tataagcttt ggcactggat ctgcggtaaa 10440
   ttgtgaacct caatctccaa catttacgtg cagatcttca ttctcggcat ttatggcgga 10500
   accatccacc cctaccctta tatctgctgg attttctttc cgtagcagcg gtgatttcac 10560
   tgctcttgag tttgtttctc caattccttc cttgcctagc ttcacatttt tcagccctag 10620
   gtcagtagat gagcatggaa accaaaataa ggttttttca agcaacaggg agaacactga 10680
   cgaaattcat acaggtaact cacttttcta tttgtaataa tttgttattg tcactgattc 10740
   tctaatgtca tttttgcatc tctaggtctt gtaacatttt aaacgtcata taaacatcca 10800
   tgacatttta tgtattttgc tcaactttca agtatttttg tatgactttt aaactttcaa 10860
   gttttatctg cgtataactt ttcatagcct aaccgtttct ctgttgtata agtatatgcg 10920
   aaaaatcatt catcacatga attgtttgaa tctaatgttg gattgtgcag cctaaccaac 10980
   tcgagtttct taaaaaaaat tgttctgtaa ttcaattatg tcctccatat cttatctttc 11040
   tttactttta aagcactatt gagaaacaga agaaaacccc agtgtgacga ctgtcagatg 11100
   tggtttgatt gagattagac tattttatca gtgttgtgtt attgagattc cagtgctgtt 11160
   aatatcatta gttccatcgt aagcttttaa agttatcaat tcataaaact agatctaatg 11220
   cccatcgtat ttttagaagg ggccgacttg gagaagcgat ttgaatcacc tcattcatcg 11280
   ctgtgctcag gaattgatgc tagggacctg aaagagaagt cagcagactc taagaggagt 11340
   ggaggaacag gggtgaggat ttttgggatc cataacaaga gaaataggac gattgggctt 11400
   ggcgaattca accagcttga tattgtggtc actgaatctg tcatccgatg ggcgtcatgc 11460
   acccacttac ctaccgcctc atcactcagg tcttctcttg tatgctgata atcatgggcc 11520
   gaccacaatg tatcaaatta acatcataaa tcatgatata aagttgggca acacgcaaac 11580
   gtgtgaattc tactgctctg ctacaagatt gaagatataa tgggtttgag tcgcgtgtac 11640
   tgttggtgat gatcatccca tattagagag ctaaatgtta gtaactacat tgtaatggat 11700
   ctgcagaata agcagattct tcttcaaaag gtgtaaagca tggattttga aagcaatggt 11760
   tttctcaacc tttttgatca tgatttagta gatatatata gggggctttt tgttcttatt 11820
   agatgtgatt gttaagcctt cttcatgaac aaacataagc actggtgact tgtgggatgg 11880
   tacatagaaa gagttccgtt tcatcgattt tgattttgtc cagatcctcc ttttctttcg 11940
   aaaaaaggga aggagagtat ccgaaagatt acccattttg tattttcggt tgtctttaaa 12000
   agattgatcc attacctttc ggacaatttg attcctataa tactcctatt tctctacttc 12060
   atttcctgcc atatactact atactaaaga caaaattact taatacgaac ttaaaattaa 12120
   gaggagaaac aaggtaaaat ttggtgagga tgatggagga atcatatgaa agcttacgca 12180
   cattatatat ttgggagtat gatgtacact agttaatgaa gatcagacac ttttctatat 12240
   cttttgattc gtatgttctt taataaatga agatggtata tttaaaattt cgtgtgtatt 12300
   tgaaaatatc atgcacatta gacatccaag tctaagatta attcacaact atctattttt 12360
   tttt 12364
<210> 6
   <211> 4659
   <212> DNA
   <213> Beta vulgaris
<220>
   <221> misc_feature
   <222> (461)..(485)
   <223> n is a, c, g, or t
<220>
   <221> misc_feature
   <222> (621)..(696)
   <223> n is a, c, g, or t
<220>
   <221> misc_feature
   <222> (704)..(707)
   <223> n is a, c, g, or t
<220>
   <221> misc_feature
   <222> (1115)..(1123)
   <223> n is a, c, g, or t
<220>
   <221> misc_feature
   <222> (1169)..(1170)
   <223> n is a, c, g, or t
<220>
   <221> misc_feature
   <222> (1879)..(1880)
   <223> n is a, c, g, or t
<220>
   <221> misc_feature
   <222> (2194)..(2194)
   <223> n is a, c, g, or t
<400> 6
   caaatcttct ggcatcaatg gcggtgttgc cgttcatcaw tttaacatca atggaggtaa    60
   gagtcatgtt ttttcaacaa tataaaactt atatgatttt tctgtttttc cccgtatctt   120
   gtttgagatt gtgattatta agagagtcaa gtctcacaat attcgaaagt ctacgtaatc   180
   cacctcaaat tgacgaagaa aacaagcagg aaaggattaa gtaagttcgt ggaaccayta   240
   gaattaattt tcaaatatag ctctacctaa tatatggcct acttttaatt ttaaataaga   300
   agaaggtaat gtgattagaa acaaattggt cttaaattat tcattaagct taataatgca   360
   taaactttat caagtgctat cttcttttca gggccgtctt gaagattttk ggkcccrgtt   420
   ctattatgaa aattgrgccc ctaaatttat asaaaataaa nnnnnnnnnn nnnnnnnnnn   480
   nnnnngatgg aaggttagag yyctaaagat agaaagttga aaatctaaat ataaatcatt   540
   gacaaattta ttaagggtga gaaacaaggg tgttttcttc aaatatgaag caaaattttc   600
   aaaataatat awtttkggsc nnnnnnnnnn nnnnnnnnnn nnnnnnnnnn nnnnnnnnnn   660
   nnnnnnnnnn nnnnnnnnnn nnnnnnnnnn nnnnnncttc tttnnnntga tacaattttt   720
   sagcgacatg attgtcgatt gatgcttata ttattgtata ctcgatccat attgtttaag   780
   atgaattgtt tgtctttgat ggtctccaat gcatattttg tatwmttagg aattytaatt   840
   atgtactatt agtasayaty gagaygaata caraatygcc ataatgaagt atgattattt   900
   tarttatata ctttctccgt tccaaatata taartgtaac acttgtgtac tttatgcgta   960
   ctaatgcata ayaacgtgca ctctccygtg tttaattata tactttttga gagaagtgwt  1020
   acattgggga ccatgggact gtgtataatt tgaccgcaaa atygaagtgt ygcatttgat  1080
   tgaaaaygga garrgtagta tatagrtgga acacnnnnnn nnntgctgrt catctttggc 1140
   caacaaaccm aaaattgata ttaatccynn twwtymrgkw yttttcatct ttttgacaca 1200
   aaatggatgt tgtaggcwct gcgctatctg ctgcccaatc tctgtttgca gccctgcaaa 1260
   gttctgagct caaagagatc ctctcgatct ttggctacaa atcccaactt gatgacctcc 1320
   aacgcaytgt mtctaccatc aaygctgtat tccgtgatgc tgagaccaaa caggagctca 1380
   ctcatgaagc acarcattgg ctcgaggaac tcaaggatgc tgtctttgaa gcagatgatc 1440
   tgttcgacga gtttgtcact cttgccgagc agaagcaact tgtagaggct ggtggcagtc 1500
   tttccaaaaa gatgcgccaa ttcttttctg attccaaccc ccttggcaty gcttatarga 1560
   tgtcacragg ggttaagaag atcaagaaga agttggatgy tatygcttac aatcatcaat 1620
   ttagctttaa gattgatctt gagcctataa aagagagaag gctcgagact ggttctgtcg 1680
   tgaacgcagg tgatatcatt ggaagagagg atgacttgga gaagatcgta ggtttgttkc 1740
   ttgattctaa catccagcgt gatgtgtctt tccttackat wgtgggaatg ggagggttgg 1800
   gtaaaactgc tcttgcccaa ctcgtgtaca atgatccaag ggtcagaact gcttttccat 1860
   tgagatgttg gaattgtsnn tctgatcaag atcaaaakma actagatgtg aaagaaattt 1920
   tgggtaagat tctgtctaca gctactggta agaatcayra gggttcaacc atggatcakg 1980
   tgcaaaccya actacrrgaa caactatgtg gcaagagata cttgcttgtt ttggatgatg 2040
   tatggaatga gaatcctaat caattgcgtr wyytkgkwra attcttcatg ggaggtcaaa 2100
   ggggaaattg gattstggta actacgcgtt cgcaygagac arcgagaatt ataagagatg 2160
   gtccattgca caagctscaa ggtttgtctg arrnaaaact yttggcgttt atytgtaagg 2220
   tggaccttcg gatcagtgca accaaaattc cctaatgact ttgtcatgat tgcacgagat 2280
   atagtygaca aatgtgctcg aaaccctytg gctataagag tggtaggaag tcttttgtgt 2340
   ggtcaagaca agagtaagtg gctttcattt catgagatmt gtttagccaa cattagaaag 2400
   agycataatg atatcatgcy aatactgaac ctaagttacc atcatcttga acctccaatc 2460
   akgagatgct ttagttattg tgcartgttt ccaaaggatt tccttatagg gaagaagacg 2520
   ttgataaacc tttggatggc acaaggttat attgttccat tagacaaaga tcaaagcata 2580
   gatgaygcta gtgaggaata catatcaatt ttgytgcaga gatgtttttt cgaaaacatc 2640
   ggaacagaaa aagatkatgt tattaagata catgatctca tgcatgatat tgctcaaaat 2700
   gtcatgggga aggagctttg tacgacaaaa aacattagtg gcagcttgga taaaaatgtt 2760
   cgccatctat ctcttgccag aactagtttt gcaagatact ctttyaatgc aactcatatt 2820
   cgctccyatt tctrtgctgg ctactggtgt caggawkctg agataamcca gttytcagtt 2880
   gaggcattag taccaaaytg tttgtgccta agggcattgk acctsgcttg gtcgaagata 2940
   aaaagtktac cagactcrat tggtggattg ttgcatttga ggtacttaga tctttcrtat 3000
   aasgaagaty tggaagtact tccgaactca attgcyaaac tatataatct rcaaacctta 3060
   caattgaagg gttgcaagag attggaaggg ttaycaaaac atttgagcag gctggttaag 3120
   cttcaaactt trgatatata tggttgcaay aatgtaactt atatgcccaa aggcatgggt 3180
   aagatgactt gccttcacac tctcagtaag tttatagtgg gtggagaagg garttgttca 3240
   agttggaagm aayggtttga tgggcwggaa gatctaaagg ctctcaacaa cctaaagggt 3300
   catctggraa tccaaatcag gtggcccgaa aatactacag atgctgtcaa ggaagatgtt 3360
   aagagggaag gattatacyt gaatcataag gaacatctca atcacattgt ggttgatttc 3420
   agatgtgagg agggtggtgg aagaatggat gatgaggaag caagaagatt gatggaagag 3480
   ytgcggccac atccttatct tgaaaatttg gctgtgaaag cataytatgg tgygaaaayg 3540
   cctgrttggg yaacccttct yccaaatctt acagagcttt wtctttytga ttgtggggaa 3600
   yyggagwrcc ttccatgcmt gggaaacttg gwtydtctra amgtyctccg rctttcgcat 3660
   ttggcraaat tggagtayat tgragaagat agcwcatcag ctmwtttcag ktktaggcct 3720
   ggaccrgaaa gtgcaggact atcattatac ttcccctccc ttgaackcct tgagttgaag 3780
   crtttgyrya agttaaaagg atggaggaga rgggaagggt taggagatga tcaccagcct 3840
   tttaatgaaa gcagcagcaa taagtcattg agaatagaaa gatgcccatt gctgacattt 3900
   atgccgctgt gtcccaagac agaaaaacdg catttagttg tatttaatga aygactccgg 3960
   atagtgcata ctaaaggaga tgagaatttc tatgctccat tacattcatc atcatctgat 4020
   cctgaaaacc cgaggagcac tattcccatt cccatgttaa gagaggtata cataaacaat 4080
   gtggcatggc taaattcgct gcctatggag gcttttaggt gtctcactca tatgacaata 4140
   aaaaacgaca aggtagagag tttgggagaa gttggggagg tgtttcggag ctrctcatct 4200
   tctttgcgat ccttgaatat cacaggttgc tccaacttaa gaagtgtttc tggagggctg 4260
   gagcatctca ctrctttgga gatkttagaa atatacgaca cccataagct gagtctwtca 4320
   gaagacccag aaggtgttgt gccatggaaa tcccttcatc actccctcag ctacttgmaa 4380
   ttgatgaatc tcccwcagct ggtcaacctg cctgattcga tgcagttctt ggyctccctc 4440
   caaacccttt caatggtgca ttgcagtaaa ctggaatcag tgccagattg gatgcccmga 4500
   ctcacttcyc tcaggaagct tatggtttca ttctgttccg cacatctgga gagaagatgy 4560
   caaaatccaa ctggggtgga ctggcctaac attcaacaca tcccctscat tgatgtcacc 4620
   tctagccgtc ctaagttttt agtgttgccg tatgaatag 4659
<210> 7
   <211> 434
   <212> DNA
   <213> Beta vulgaris
<400> 7
   gaactcaagg atgctgtctt tgaagcagat gatctgttcg acgagtttgt cactcttgcc    60
   gagcagaagc aacttgtaga ggctggtggc agtctttcca aaaagatgcg ccaattcttt   120
   tctgattcca acccccttgg cattgcttat aggatgtcac gaggggttaa gaagatcaag   180
   aagaagttgg atgctatcgc ttacaatcat caatttagct ttaagattga tcttgagcct   240
   atgaaagaga gaaggctaga gactggttct gtcgtgaacg caggtgatat cattggaaga   300
   gaggacgact tggagaagat tgtaggtttg ttgcttgatt ctaacatcca acgtgatgtg   360
   tctttcctta ctattgtggg aatgggaggg ttgggtaaaa ctgctcttgc ccaactcgtg   420
   tacaatgatc caag                                                     434